# EUROPEAN PATENT APPLICATION

(11) **EP 4 620 951 A1**
(43) Date of publication of application: **24.09.2025**
(21) Application number: 23890842.0
(22) Date of filing: 16.11.2023
(51) Int. Cl.: C07D 211/60, C07D 295/205, C07D 401/06, C07D 243/08, C07C 273/18, C07C 275/42, A61P 29/00, A61P 9/00, A61P 25/00, A61P 3/10, A61P 13/12, A61P 11/00, A61K 31/445, A61K 31/495

(54) **MEMANTINE UREA DERIVATIVE, PREPARATION METHOD THEREFOR, AND USE THEREOF IN PREPARATION OF MEDICAMENT FOR TREATING SOLUBLE EPOXIDE ENZYME-MEDIATED DISEASES**

(30) Priority: 18.11.2022 CN 202211458374
(71) Applicant: Shenyang Pharmaceutical University, Shenyang, Liaoning 110000 (CN); Wang, Gaohua, Beijing 100070 (CN)
(72) Inventor: CHEN, Guoliang, Shenyang, Liaoning 110000 (CN); WANG, Gaohua, Beijing 100070 (CN); CHEN, Yuanguang, Shenyang, Liaoning 110000 (CN); LIU, Zhongbo, Shenyang, Liaoning 110000 (CN); CAO, Ruolin, Shenyang, Liaoning 110000 (CN); XU, Huashen, Shenyang, Liaoning 110000 (CN); CHEN, Lu, Shenyang, Liaoning 110000 (CN)
(74) Representative: Lorenz & Kollegen
(86) International application number: PCT/CN2023/131933
(87) International publication number: WO 2024/104411

(57) **Abstract**

The present application provides a memantine urea derivative, a preparation method therefor, and use thereof, which relate to the technical field of pharmaceutics. The memantine urea derivative provided by the present application has a typical urea structure as a primary pharmacophore of sEH. Molecular docking shows that the memantine moiety acts as a hydrophobic fragment to generate hydrophobic interactions with a receptor. Notably, when R₁ and R₂ are all methyl groups (i.e., 3,5-dimethyl substitution), the van der Waals force can be enhanced. Therefore, the memantine urea derivative provided by the present application has high inhibitory activity against human sEH (HsEH) and murine sEH (MsEH), and can be used as an sEH inhibitor for preparing a medicament for treating a soluble epoxide enzyme-mediated disease.

## Description

The present application claims priority to the Chinese Patent Application CN202211458374.9 filed to the China National Intellectual Property Administration (CNIPA) on November 18, 2022 and entitled "MEMANTYL UREA DERIVATIVE, PREPARATION METHOD THEREOF, AND USE THEREOF IN PREPARATION OF DRUG FOR TREATING SOLUBLE EPOXIDE HYDROLASE-MEDIATED DISEASE", which is incorporated herein by reference in its entirety.

### TECHNICAL FIELD

The present disclosure relates to the technical field of medicine, and in particular to a memantyl urea derivative, a preparation method thereof, and use thereof in preparation of a drug for treating a soluble epoxide hydrolase (sEH)-mediated disease.

### BACKGROUND

In mammals, arachidonic acid is a polyunsaturated fatty acid metabolized by cyclooxygenase (COX), lipoxygenase (LOX), and cytochrome P450 (CYPs). COX and LOX pathways mainly lead to the production of proinflammatory lipid mediators such as prostaglandin and leukotriene and are drug-targetable (Toxicol. 2014, 114, 8 3-91.). In contrast, CYP450 enzymes act on long-chain polyunsaturated fatty acids to form EpFA by epoxidation of double bonds (Biochimica Et Biophysica Acta, 2011, 1814(1): 210-222), which is highly selective. According to the position of the epoxidized double bond, the EpFA is divided into epoxyeicosatrienoic acids (EETs), epoxyeicosatetraenoic acids (EEQs), and epoxyeicosapentaenoic acids (EDPs) (Journal of Lipid Research, 2010, 51(12): 3481-3490.). All types of EpFA can be hydrolyzed by sEH into diols, in which diols from hydrolyzing by EETs have the lowest activity (Neuron, 2007, 55(3): 353-364.).

Soluble epoxide hydrolase (sEH) is a member of the α/β hydrolase family. Studies have found that in mammals, sEH is a homodimer composed of two 60 kD subunits in an antiparallel manner, and each of subunits has C-terminal hydrolase and N-terminal phosphatase properties. The C-terminal domain has typical α/β hydrolase properties and hydrolyzes epoxides by adding water to the three-membered ethylene oxide ring. The N-terminal domain has phosphatase activity and can hydrolyze lipid phosphates, but its specific biological function in mammals still remains unclear. sEH as a group of enzymes with similar functions is divided into 8 different subtypes, including mammalian, plant, and microsomal peroxidases (Biochimie, 2013, 95(1): 91-95.), among which the mammalian subtype is the most important subtype. sEH in mammals is widely distributed throughout the body and is most active in the liver, kidney, intestine, and vascular system.

Pain is a complex signal transduction process that originates from the damage of harmful substances and the release of inflammatory mediators, such as cytokines, ions, bradykinins, prostaglandins, and leukotrienes, which directly act on pain receptors and drive action potentials to produce a sense of pain (Neuron, 2007, 55(3): 353-364.). Generally, pain is a signal to avoid further injury. Although many methods are currently available to relieve pain, most of these methods have dose-dependent or use-limiting side effects. Therefore, novel therapies are required to treat pain. Inflammatory pain is caused by inflammation of either biological or chemical origin. Inhibiting sEH activity to relieve inflammatory pain stems from researches on the anti-inflammatory effects of sEH inhibitors. In a study of a lipopolysaccharide-induced sepsis model, it was found that inhibition of sEH can not only alter the levels of EET and diol metabolites, but also alter the levels of several other metabolites in the COX and LOX metabolic pathways of the arachidonic acid cascade (Proc Natl Acad Sci USA, 2005, 102(28): 9772-9777). Notably, inhibition of sEH activity with small molecules reduces the level of prostaglandin 2 (PGE2), an inflammatory mediator and pain-inducing substance. This finding is groundbreaking since it demonstrates that stabilizing endogenous bioactive lipids is a new strategy to limit inflammation. Since previous studies have found that sEH inhibitors have the effect of inhibiting cyclooxygenase 2 (COX-2), the ability of sEH inhibitors to act synergistically with COX-2 selective inhibitors nonsteroidal anti-inflammatory drugs (NSAIDs) is first studied in an inflammatory pain model (Proc Natl Acad Sci USA, 2006, 103(37): 13646-13651.). The results show that sEH inhibitors and NSAIDs together reduce the levels of PGE2 and COX-2 expression in mice and increase the thermal withdrawal latency. Notably, these improvements have occurred without significant changes in the prostacyclin-to-thromboxane-ratio. The adverse side effects of COX-2 selective inhibitors NSAIDs inducing thrombosis are suspected to be caused by changes in the homeostatic balance of COX metabolites (N Engl J Med, 2004, 351: 1709-1711.). In subsequent studies, sEH inhibitors are tested alone to determine whether they could counteract hyperalgesia. Inceoglu et al. (Life Sciences, 2006, 79(24): 2311-2319.) have found that local administration of two different sEH inhibitors can effectively increase the thermal withdrawal latency and pain threshold in the lipopolysaccharide (LPS)-induced inflammatory pain model of rats. This study has also revealed that EpFA metabolites act against hyperalgesia by increasing thermal withdrawal latency to LPS pain. It can be inferred that inhibiting sEH activity may be an effective method for treating inflammatory pain.

In 2011, *PAIN,* the official academic journal of International Association for the Study of Pain (IASP), published a new definition of neuropathic pain (NPP): pains directly caused by damage or disease of the somatic sensory nervous system, which can be secondary to a variety of diseases or injuries, such as stroke and diabetes. NPP is one of the most difficult diseases to treat and imposes a huge burden on society and individuals. Accordingly, novel methods are urgently needed to treat the NPP. Initial studies on the effects of sEH inhibitors in neuropathy are aimed to compare the relationship between COX levels and inflammatory pain in pain models. Inceoglu et al. (Proceedings of the National Academy of Sciences of the United States of America, 2008, 105(48): 18901-18906.) have found that sEH inhibition could block diabetic neuropathy in a chronic pain model, and is thus proposed as a negative control experimental model. This is exciting because most NSAIDs that block COX have little effect on NPP (European Journal of Pharmacology, 2013, 700(1-3): 93-101.). Inceoglu et al. (Proc Natl Acad Sci USA, 2012, 109(28): 11390-11395.) have explored the effects of sEH inhibitors on diabetic neuropathy in preclinical models and revealed that sEH inhibitors improve mechanical pain thresholds in a dose-dependent manner and that sEH inhibitors are superior to standard treatment with gabapentin. This anti-hyperalgesia is independent of changes in glucose tolerance, insulin resistance, and glucose-stimulated insulin secretion. Wagner et al. (Behavioural Brain Research, 2017, 326: 69-76.) have further demonstrated the role of sEH inhibitors in the congenital Akita mouse type I diabetes model. In a study using Akita mice as a model, it was found that sEH inhibitors are effective against diabetic neuropathy in mice, and sEH activity is correlated with the severity of this disease. Guedes A et al. (Equine Veterinary Journal, 2017, 49(3): 345-351.) have found that sEH inhibitors are more effective than previous standard treatments in treating severe equine laminitis, and that sEH inhibitors continue to be successful as a treatment for this disease. It can be inferred that sEH inhibitors may be one of the effective strategies for treating NPP.

Given the importance of sEH inhibitors and EpFA in the occurrence and development of inflammation and pain, as well as their protective effects on multiple organs such as the heart, kidney, and brain, inhibiting sEH activity could improve and stabilize the content of EpFA *in vivo,* such as EETs, thereby exerting analgesic, anti-inflammatory, and protective effects on multiple organs. As a result, it is urgent and necessary to develop novel and efficient sEH inhibitors for the treatment of pain.

### SUMMARY

An object of the present disclosure is to provide a memantyl urea derivative, a preparation method thereof, and use thereof in preparation of a drug for treating an sEH-mediated disease. In the present disclosure, the memantyl urea derivative shows a high inhibitory activity and low side effects against human sEH (HsEH) and mouse sEH (MsEH), and can be used as an sEH inhibitor in preparation of a drug for treating sEH-mediated diseases.

To achieve the above object, the present disclosure provides the following technical solutions.

The present disclosure provides a memantyl urea derivative having a structure selected from the group consisting of Formula A and Formula B: 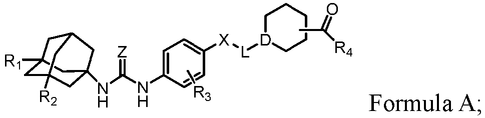 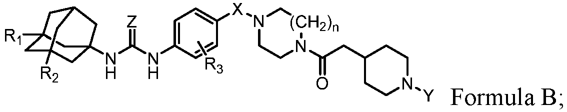 where
R₁ and R₂ each are independently selected from the group consisting of -H, -OH, -NH₂, -SH, -CN, a halogen group, alkyl, alkoxy, aryl, and heteroaryl;
R₃ is selected from the group consisting of -H, -OH, -NH₂, -SH, -CN, a halogen group, alkyl, and alkoxy;
R₄ is selected from the group consisting of -OH, -NH₂, hydroxylamino, alkyl-substituted amino, alkoxy-substituted amino, alcohol group-substituted amino, phenyl-substituted amino, naphthyl-substituted amino, and 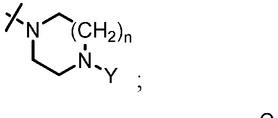
Y is selected from the group consisting of -H, 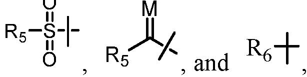 wherein
R₅ is selected from the group consisting of alkyl and a heterocyclic group, the alkyl being selected from the group consisting of chain alkyl and cycloalkyl; and
R₆ is selected from the group consisting of alkyl-substituted amino and alcohol group-substituted alkyl;
X is selected from the group consisting of -CH₂- and 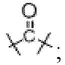
L is absent or -NH-;
D is selected from the group consisting of 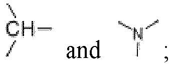
n is 1 or 2; and
Z and M each are independently selected from the group consisting of =O and =S.

In some embodiments, R₁ and R₂ each are independently selected from the group consisting of -H, -OH, -NH₂, -SH, -CN, -F, -Cl, -Br, methyl, ethyl, propyl, butyl, pentyl, isobutyl, isopropyl, isopentyl, tert-butyl, methoxy, ethoxy, propoxy, isopropoxy, butoxy, cyclopentyloxy, cyclohexyloxy, phenoxy, and benzyloxy.

In some embodiments, R₃ is selected from the group consisting of -H, -OH, -NH₂, -SH, -CN, -F, -Cl, -Br, unsubstituted or substituted C1-C6 alkyl, and unsubstituted or substituted C1-C6 alkoxy; and a substituent on the substituted C1-C6 alkyl and the substituted C1-C6 alkoxy is independently selected from the group consisting of -F, -Cl, -Br, -OH, -NH₂, -NHCH₃, -N(CH₃)₂, and C1-C6 alkyl.

In some embodiments, the alkyl-substituted amino for R₄ is unsubstituted or substituted C1-C6 alkyl-substituted amino; the alkoxy-substituted amino for R₄ is unsubstituted or substituted C1-C6 alkoxy-substituted amino; the phenyl-substituted amino for R₄ is unsubstituted or substituted phenyl-substituted amino; the naphthyl-substituted amino for R₄ is unsubstituted or substituted naphthyl-substituted amino; the alcohol group-substituted amino for R₄ is unsubstituted or substituted C1-C6 alcohol group-substituted amino; and a substituent on substituted C1-C6 alkoxy, substituted phenyl, substituted naphthyl, and substituted C1-C6 alcohol group is independently selected from the group consisting of -F, -Cl, -Br, -OH, -NH₂, -NHCH₃, -N(CH₃)₂, and C1-C6 alkyl.

In some embodiments, the chain alkyl for R₅ is unsubstituted or substituted C1-C6 saturated or unsaturated chain alkyl, and a substituent on the substituted C1-C6 saturated or unsaturated chain alkyl is selected from the group consisting of -OH, -NH₂, and C1-C6 alkyl;
the cycloalkyl for R₅ is unsubstituted or substituted C3-C6 cycloalkyl, and a substituent on the substituted C3-C6 cycloalkyl is selected from the group consisting of -F, -Cl, -Br, -OH, -NH₂, -NHCH₃, -N(CH₃)₂, and C1-C6 alkyl; and
the heterocyclic group for R₅ is an unsubstituted or substituted C3-C6 saturated or unsaturated heterocyclic group, and a substituent on the substituted C3-C6 saturated or unsaturated heterocyclic group is independently selected from the group consisting of -F, -Cl, -Br, -OH, -NH₂, -NHCH₃, -N(CH₃)₂, and C1-C6 alkyl.

In some embodiments, the alkyl-substituted amino for R₆ is unsubstituted or substituted C1-C6 alkyl-substituted amino, and the alcohol group-substituted alkyl is unsubstituted or substituted C1-C6 alcohol group-substituted alkyl.

In some embodiments, the memantyl urea derivative includes (S)-1-(4-(3-((1r,3R,5 S,7S)-3,5-dimethyladamantan-1-yl)ureido)-3-fluorobenzyl)piperidine-3-carboxamide, 1-((1r,3R, 5S,7S)-3,5-dimethyladamantan-1-yl)-3-(2-fluoro-4-(((S)-3-(4-propionyl-1,4-)diaza-1-carbonyl)pi peridin-1-yl)methyl)phenyl)urea, 1-((1r,3R,5S,7S)-3,5-dimethyladamantan-1-yl)-3-(2-fluoro-4-(((3 S)-3-(4-(2-methylbutanoyl)-1,4-diaza-1-carbonyl)piperidin-1-yl)methyl)phenyl)urea, (S)-1-(4-(3-((1r,3R,5S,7S)-3,5-dimethyladamantan-1-yl)ureido)-3-fluorobenzyl)-N,N-dimethylpiperidi ne-3-carboxamide, (S)-1-(4-(3-((1r,3R,5S,7S)-3,5-dimethyladamantan-1-yl)ureido)-3-fluoroben zyl)-N,N-diethylpiperidine-3-carboxamide, (S)-1-(4-(3-((1r,3R,5S,7S)-3,5-dimethyladamantan-1-yl)ureido)-3-fluorobenzyl)-N,N-bis(2-hydroxyethyl)piperidine-3-carboxamide, 1-(4-(3-((1r,7r) -3,5-dimethyladamantan-1-yl)ureido)-3-fluorobenzyl)-N,N-bis(2-hydroxyethyl)piperidine-4-carb oxamide, (S)-N-(1,3-dihydroxypropan-2-yl)-1-(4-(3-((1r,3R,5S,7S)-3,5-dimethyladamantan-1-yl) ureido)-3-fluorobenzyl)piperidine-3-carboxamide, (3S)-1-(4-(3-((1r,3R,5S,7S)-3,5-dimethylada mantan-1-yl)ureido)-3-fluorobenzyl)-N-(2-hydroxypropyl)piperidine-3-carboxamide, (S)-1-(4-(3-((1r,3R,5S,7S)-3,5-dimethyladamantan-1-yl)ureido)-3-fluorobenzyl)-N-(2-hydroxyethyl)piper idine-3-carboxamide, (S)-N-(1,3-dihydroxy-2-(hydroxymethyl)propan-2-yl)-1-(4-(3-((1r,3R,5S, 7S)-3, 5-dimethyladamantan-1-yl)ureido)-3-fluorobenzyl)piperidine-3-carboxamide, 1-((1r,3R, 5S,7S)-3,5-dimethyladamantan-1-yl)-3-(2-fluoro-4-(((S)-3-(4-(2-hydroxyethyl)piperazine-1-carb onyl)piperidin-1-yl)methyl)phenyl)urea, N-(1,3-dihydroxy-2-(hydroxymethyl)propan-2-yl)-1-(4 -(3-((1r,7r)-3,5-dimethyladamantan-1-yl)ureido)-3-fluorobenzyl)piperidine-4-carboxamide, 1-(4 -(3-((1r,7r)-3,5-dimethyladamantan-1-yl)ureido)-3-fluorobenzyl)piperidine-4-carboxamide, N-(1, 3-dihydroxypropan-2-yl)-1-(4-(3-((1r,7r)-3,5-dimethyladamantan-1-yl)ureido)-3-fluorobenzyl)pi peridine-4-carboxamide, 1-(4-(3-((1r,7r)-3,5-dimethyladamantan-1-yl)ureido)-3-fluorobenzyl)-N-(2-hydroxypropyl)piperidine-4-carboxamide, 1-(4-(((S)-3-(4-acryloyl-1,4-diaza-1-carbonyl)pi peridin-1-yl)methyl)-2-fluorophenyl)-3-((1r,3R,5S,7S)-3,5-dimethyladamantan-1-yl)urea, 1-(4-((S)-3-(4-(cyclopropanecarbonyl)-1,4-diaza-1-carbonyl)piperidin-1-yl)methyl)-2-fluorophenyl)-3 -((1r,3R,5S,7S)-3,5-dimethyladamantan-1-yl)urea, 1-((1r,3R,5S,7S)-3,5-dimethyladamantan-1-y 1)-3-(2-fluoro-4-(((3S)-3-(1-(2-methylbutyryl)azacyclohexane-4-carbonyl)piperidin-1-yl)methyl) phenyl)urea, (S)-1-(4-(3-((1r,3R,5S,7S)-3,5-dimethyladamantan-1-yl)ureido)-3-fluorobenzyl)pip eridine-3-carboxylic acid, 1-((1r,3R,5S,7r)-3,5-dimethyladamantan-1-yl)-3-(2-fluoro-4-(4-(2-(1 -(2-methylbutyryl)piperidin-4-yl)acetyl)-1,4-diaza-1-carbonyl)phenyl)urea, 1-((1r,3R,5S,7r)-3,5 -dimethyladamantan-1-yl)-3-(2-fluoro-4-(4-(2-(1-propionylpiperidin-4-yl)acetyl)-1,4-diaza-1-car bonyl)phenyl)urea, 1-(4-(4-(2-(1-acetylpiperidin-4-yl)acetyl)-1,4-diaza-1-carbonyl)-2-fluorophe nyl)-3-((1r,3R,5S,7r)-3,5-dimethyladamantan-1-yl)urea, (S)-1-(4-(3-((1r,3R,5S,7S)-3,5-dimethyl adamantan-1-yl)ureido)-3-fluorobenzoyl)-N,N-diethylpiperidine-3-carboxamide, 1-(4-(3-((1r,3R, 5S,7r)-3,5-dimethyladamantan-1-yl)ureido)-3-fluorobenzoyl)-N,N-diethylpiperidine-4-carboxam ide, 1-((1r,3R,5S,7r)-3,5-dimethyladamantan-1-yl)-3-(2-fluoro-4-(4-(4-(2-hydroxyethyl)piperazi ne-1-carbonyl)piperidine-1-carbonyl)phenyl)urea, (3S)-1-(4-(3-((1r,3R,5S,7S)-3,5-dimethylada mantan-1-yl)ureido)-3-fluorobenzoyl)-N-(2-hydroxypropyl)piperidine-3-carboxamide, 1-(4-(3-((1r,3R,5S,7r)-3,5-dimethyladamantan-1-yl)ureido)-3-fluorobenzoyl)-N-(2-hydroxyethyl)piperid ine-4-carboxamide, 1-(4-(3-((1r,3R,5S,7r)-3,5-dimethyladamantan-1-yl)ureido)-3-fluorobenzoyl) -N-(2-hydroxypropyl)piperidine-4-carboxamide, N-(1,3-dihydroxypropan-2-yl)-1-(4-(3-((1r,3R, 5S,7r)-3,5-dimethyladamantan-1-yl)ureido)-3-fluorobenzoyl)piperidine-4-carboxamide, (S)-1-(4 -(3-((1r,3R,5S,7S)-3,5-dimethyladamantan-1-yl)ureido)-3-fluorobenzoyl)-N-(2-hydroxyethyl)pip eridine-3-carboxamide, 1-((1r,3R,5S,7S)-3,5-dimethyladamantan-1-yl)-3-(2-fluoro-4-((S)-3-(4-(2-hydroxyethyl)piperazine-1-carbonyl)piperidine-1-carbonyl)phenyl)urea, 1-((1r,3R,5S,7r)-3, 5-dimethyladamantan-1-yl)-3-(4-(4-(2-(1-(1-(dimethylamino)propan-2-yl)piperidin-4-yl)acetyl)-1,4-diaza-1-carbonyl)-2-fluorophenyl)urea, 1-((1r,3R,5S,7r)-3,5-dimethyladamantan-1-yl)-3-(2-fluoro-4-((4-(2-(1-propionylpiperidin-4-yl)acetyl)-1,4-diaza-1-yl)methyl)phenyl)urea, 1-((1r,3R, 5S,7r)-3,5-dimethyladamantan-1-yl)-3-(2-fluoro-4-((4-(2-(1-(2-methylbutyryl)piperidin-4-yl)ace tyl)-1,4-diaza-1-yl)methyl)phenyl)urea, 1-(4-((4-(2-(1-acetylpiperidin-4-yl)acetyl)-1,4-diaza-1-y l)methyl)-2-fluorophenyl)-3-((1r,3R,5S,7r)-3,5-dimethyladamantan-1-yl)urea, methyl 4-(2-(4-(4-(3-((1r,3R,5S,7r)-3,5-dimethyladamantan-1-yl)ureido)-3-fluorobenzyl)-1,4-diaza-1-yl)-2-oxoe thyl)piperidine-1-carboxylate, 1-((1r,3R,5S,7r)-3,5-dimethyladamantan-1-yl)-3-(2-fluoro-4-((4-(2-(1-(2-hydroxyethyl)piperidin-4-yl)acetyl)-1,4-diaza-1-yl)methyl)phenyl)urea, 1-(4-((4-(2-(1-(2,3-dihydroxypropyl)piperidin-4-yl)acetyl)-1,4-diaza-1-yl)methyl)-2-fluorophenyl)-3-((1r,3R,5 S,7r)-3,5-dimethyladamantan-1-yl)urea, 1-((1r,3R,5S,7r)-3,5-dimethyladamantan-1-yl)-3-(4-((4-(2-(1-(2-(dimethylamino)ethyl)piperidin-4-yl)acetyl)-1,4-diaza-1-yl)methyl)-2-fluorophenyl)urea, 1-(4-((4-(2-(1-(2-(diethylamino)ethyl)piperidin-4-yl)acetyl)-1,4-diaza-1-yl)methyl)-2-fluorophe nyl)-3-((1r,3R,5S,7r)-3,5-dimethyladamantan-1-yl)urea, 1-(4-((S)-3-(4-acetyl-1,4-diaza-1-carbo nyl)piperidine-1-carbonyl)-2-fluorophenyl)-3-((1r,3R,5S,7S)-3,5-dimethyladamantan-1-yl)urea, 1-((1r,3R,5S,7S)-3,5-dimethyladamantan-1-yl)-3-(2-fluoro-4-((S)-3-(4-propionyl-1,4-diaza-1-car bonyl)piperidine-1-carbonyl)phenyl)urea, 1-((1r,3R,5S,7S)-3,5-dimethyladamantan-1-yl)-3-(2-fl uoro-4-((3S)-3-(4-(2-methylbutanoyl)-1,4-diaza-1-carbonyl)piperidine-1-carbonyl)phenyl)urea, 4-(3-((1r,3R,5S,7r)-3,5-dimethyladamantan-1-yl)ureido)-3-fluoro-N-((1r,4R)-4-((2-hydroxyethyl) carbamoyl)cyclohexyl)benzamide, 4-(3-((1r,3R,5S,7r)-3,5-dimethyladamantan-1-yl)ureido)-3-fl uoro-N-((1r,4R)-4-((2-hydroxypropyl)carbamoyl)cyclohexyl)benzamide, N-((1r,4R)-4-(bis(2-hy droxyethyl)carbamoyl)cyclohexyl)-4-(3-((1lr,3R,5S,7r)-3,5-dimethyladamantan-1-yl)ureido)-3-fl uorobenzamide, N-((1r,4R)-4-carbamoylcyclohexyl)-4-(3-((1r,3R,5S,7r)-3,5-dimethyladamanta n-1-yl)ureido)-3-fluorobenzamide, N-((1r,4R)-4-((1,3-dihydroxypropan-2-yl)carbamoyl)cyclohe xyl)-4-(3-((1r,3R,5S,7r)-3,5-dimethyladamantan-1-yl)ureido)-3-fluorobenzamide, N-((1r,4R)-4-((1,3-dihydroxy-2-(hydroxymethyl)propan-2-yl)carbamoyl)cyclohexyl)-4-(3-((1r,3R,5S,7r)-3,5-dimethyladamantan-1-yl)ureido)-3-fluorobenzamide, tert-butyl 4-(2-((1R, 4r)-4-(4-(3-((1r,3R,5 S,7r)-3,5-dimethyladamantan-1-yl)ureido)-3-fluorobenzamido)cyclohexane-1-carboxamido)ethy l)piperazine-1-carboxylate, or 4-(3-((1r,3R,5S,7r)-3,5-dimethyladamantan-1-yl)ureido)-3-fluoro -N-((1r,4R)-4-((2-hydroxyethyl)carbamoyl)cyclohexyl)benzamide.

The present disclosure provides a method for preparing the above-mentioned memantyl urea derivative, where
(1) for preparation of the memantyl urea derivative having the structure shown in Formula A:
   (1-1) under the condition that the memantyl urea derivative has the structure shown in Formula A where X is 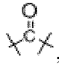 L is absent, D is 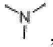 and R₄ is not 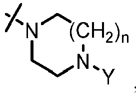 the method includes the following steps:
      subjecting a compound 1 and a compound a to a first acylation to obtain a compound b;
      subjecting the compound b to a first reduction to obtain a compound c;
      subjecting a compound 2 and a compound 4 to a second acylation to obtain an intermediate compound 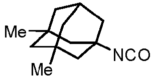 or 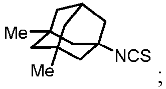
      subjecting the intermediate compound and the compound c to a first nucleophilic substitution to obtain a compound d;
      subjecting the compound d to a first hydrolysis to obtain the memantyl urea derivative having the structure shown in Formula A where R₄ is -OH; and
      mixing the memantyl urea derivative having the structure shown in Formula A where R₄ is -OH with a compound 3, and subjecting a resulting mixture to a third acylation in the presence of 2-(7-azabenzotriazol-1-yl)-*N*,*N*,*N'*,*N'*-tetramethyluronium hexafluorophosphate (HATU) and *N,N*-diisopropylethylamine (DIEA) to obtain the memantyl urea derivative having the structure shown in Formula A; where
      structural formulas of the compound 1 and the compound 2 are 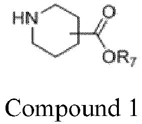 and 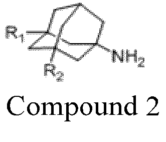 respectively;
      the compound 3 is selected from the group consisting of NH₃, hydroxylamine, alkylamine, alkoxyamine, alcoholamine, phenylamine, and naphthylamine;
      the compound 4 is selected from the group consisting of bis(trichloromethyl)carbonate (BTC) and thiophosgene;
      structural formulas of the compound a, the compound b, the compound c, the compound d, and the compound e are as follows: 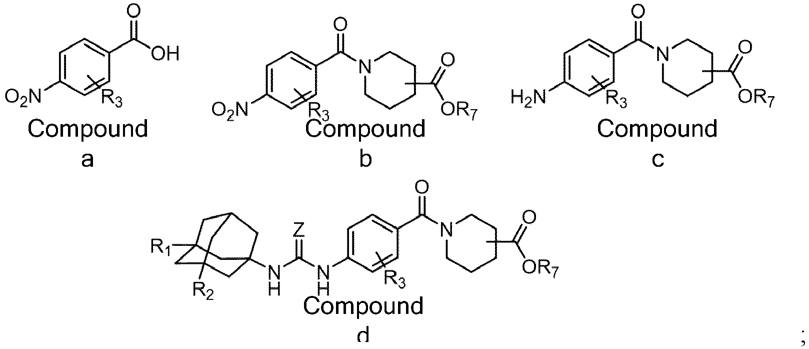
      in the structural formulas of the compound 1, the compound b, the compound c, and the compound d, R₇ is C₁-C₆ alkyl;
   (1-2) under the condition that the memantyl urea derivative has the structure shown in Formula A where X is -CH₂-, L is absent, D is 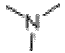 and R₄ is not 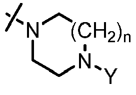 the method includes the following steps:
      subjecting the compound a to a second reduction to obtain a compound e;
      subjecting the compound e and a bromination reagent to a second nucleophilic substitution to obtain a compound f;
      subjecting the compound f and the compound 1 to a third nucleophilic substitution to obtain a compound g;
      subjecting the compound g to a third reduction to obtain a compound h;
      subjecting the intermediate compound and the compound h to a fourth nucleophilic substitution to obtain a compound i;
      subjecting the compound i to a second hydrolysis to obtain the memantyl urea derivative having the structure shown in Formula A where R₄ is -OH; and
      mixing the memantyl urea derivative having the structure shown in Formula A where R₄ is -OH with the compound 3, and subjecting the obtained mixture to a fourth acylation in the presence of HATU and DIEA to obtain the memantyl urea derivative having the structure shown in Formula A; where
      structural formulas of the compound e, the compound f, the compound g, the compound h, and the compound i are as follows: 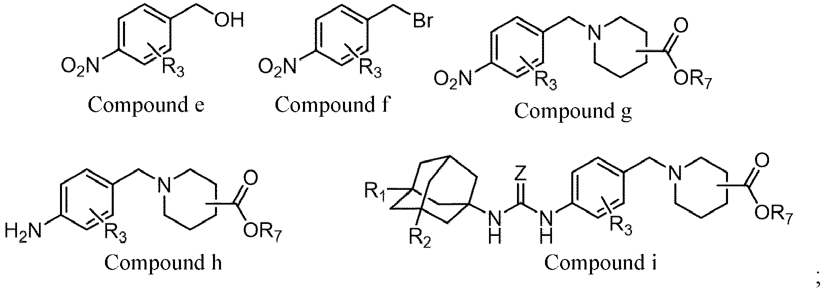 and
      in structural formulas of the compound g, the compound h, and the compound i, R₇ is C₁-C₆ alkyl;
   (1-3) under the condition that the memantyl urea derivative has the structure shown in Formula A where X is 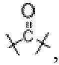 L is -NH-, D is 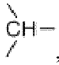 and R₄ is not 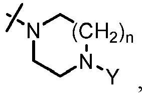 the method includes the following steps:
      subjecting a compound z and the compound a to a fifth acylation to obtain a compound j;
      subjecting the intermediate compound and the compound j to a fifth nucleophilic substitution to obtain a compound y;
      subjecting the compound y to a third hydrolysis to obtain the memantyl urea derivative having the structure shown in Formula A where R₄ is -OH; and
      mixing the memantyl urea derivative having the structure shown in Formula A where R₄ is -OH with the compound 3, and subjecting a mixture thereof to a sixth acylation in the presence of HATU and DIEA to obtain the memantyl urea derivative having the structure shown in Formula A; where
      the compound z has a structural formula shown as 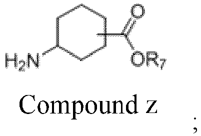
      the compound j has a structural formula shown as 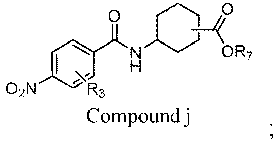
      the compound y has a structural formula shown as 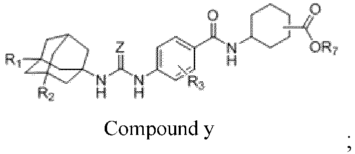 and
      in structural formulas of the compound z, the compound j, and the compound y, R₇ is C₁-C₆ alkyl;
   (1-4) under the condition that the memantyl urea derivative has the structure shown in Formula A where X is -CH₂-, L is -NH-, D is 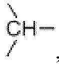 and R₄ is not 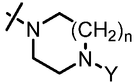 the method includes the following steps:
      subjecting the compound f and the compound z to a sixth nucleophilic substitution to obtain a compound k;
      subjecting the compound k to a fourth reduction to obtain a compound 5;
      subjecting the intermediate compound and the compound 5 to a seventh nucleophilic substitution to obtain a compound 6;
      subjecting the compound 6 to a fourth hydrolysis to obtain the memantyl urea derivative having the structure shown in Formula A where R₄ is -OH; and
      mixing the memantyl urea derivative having the structure shown in Formula A where R₄ is -OH with the compound 3, and subjecting the acquired mixture to a seventh acylation in the presence of HATU and DIEA to obtain the memantyl urea derivative having the structure shown in Formula A; where
      structural formulas of the compound k, the compound 5, and the compound 6 are as follows: 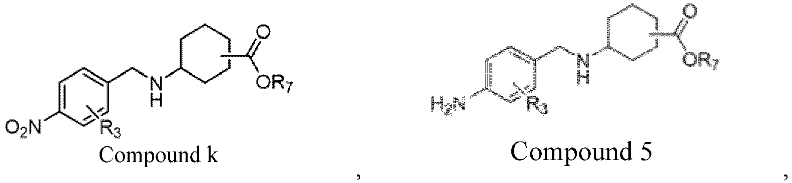 and 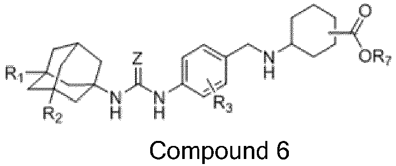 and
      in structural formulas of the compound k, the compound 5, and the compound 6, R₇ is C₁-C₆ alkyl;
   (1-5) under the condition that the memantyl urea derivative has the structure shown in Formula A where X is 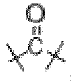 L is absent, D is 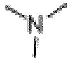 and R₄ is 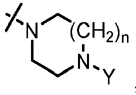 the method includes the following steps:
      subjecting a compound 7 and a compound L to an eighth acylation to obtain a compound m;
      subjecting the compound m to a first deprotection in an acidic environment to obtain a compound n, the compound n being the memantyl urea derivative having the structure shown in Formula A where Y is -H;
      mixing the compound n with a compound 8, and subjecting the resulted mixture to a ninth acylation in the presence of HATU and DIEA to obtain the memantyl urea derivative having the structure shown in Formula A where Y is 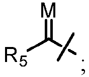
      subjecting the compound n and a compound 9 to a tenth acylation to obtain the memantyl urea derivative having the structure shown in Formula A where Y is 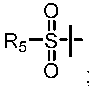 and
      subjecting the compound n and a compound 10 to an eighth nucleophilic substitution to obtain the memantyl urea derivative having the structure shown in Formula A where Y is 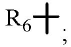 where
      the compound 7 has a structural formula shown as 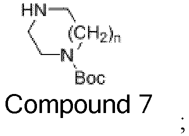
      the compound 8 has a structural formula shown as 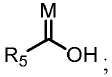
      the compound 9 has a structural formula shown as 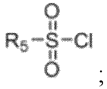
      the compound 10 has a structural formula shown as R₆-Cl; and
      structural formulas of the compound L, the compound m, and the compound n are as follows: 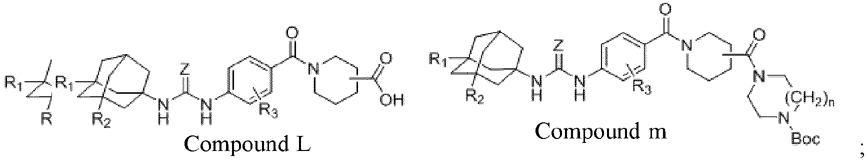 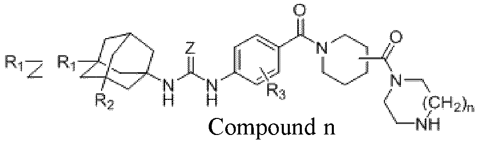 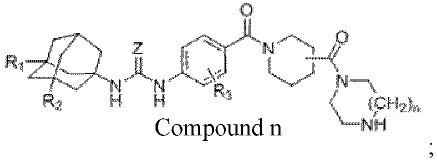
   (1-6) under the condition that the memantyl urea derivative has the structure shown in Formula A where X is -CH₂-, L is absent, D is 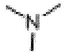 and R₄ is 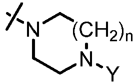 the method includes the following steps:
      subjecting a compound 7 and a compound o to an eleventh acylation to obtain a compound 11;
      subjecting the compound 11 to a second deprotection in an acidic environment to obtain a compound 12, the compound 12 being the memantyl urea derivative having the structure shown in Formula A where Y is -H;
      mixing the compound 12 with the compound 8 and conducting a twelfth acylation in the presence of HATU and DIEA to obtain the memantyl urea derivative having the structure shown in Formula A where Y is 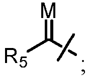
      subjecting the compound 12 and the compound 9 to a thirteenth acylation to obtain the memantyl urea derivative having the structure shown in Formula A where Y is 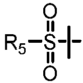 and
      subjecting the compound 12 and the compound 10 to a ninth nucleophilic substitution to obtain the memantyl urea derivative having the structure shown in Formula A where Y is 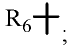 where
      the compound o is 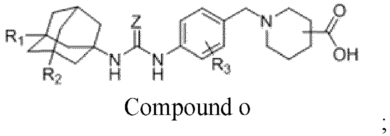
      the compound 11 has a structural formula shown as 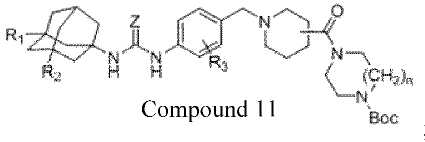 and
      the compound 12 has a structural formula shown as 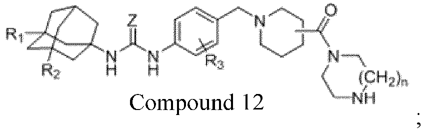
(2) for preparation of the memantyl urea derivative having the structure shown in Formula B:
   (2-1) under the condition that the memantyl urea derivative has the structure shown in Formula B where X is 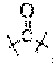 the method includes the following steps:
      subjecting the compound 7 and the compound a to a fourteenth acylation to obtain a compound p;
      subjecting the compound p to a fifth reduction to obtain a compound q;
      subjecting the intermediate compound and the compound q to a tenth nucleophilic substitution to obtain a compound r;
      subjecting the compound r to a third deprotection in an acidic environment to obtain a compound s;
      subjecting the compound 13 and the compound s to a fifteenth acylation to obtain a compound t;
      subjecting the compound t to a fourth deprotection in an acidic environment to obtain a compound u, the compound u being the memantyl urea derivative having the structure shown in Formula B where Y is -H;
      mixing the compound u with the compound 8 and conducting a sixteenth acylation in the presence of HATU and DIEA to obtain the memantyl urea derivative having the structure shown in Formula B where Y is 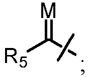
      subjecting the compound u and the compound 9 to a seventeenth acylation to obtain the memantyl urea derivative having the structure shown in Formula B where Y is 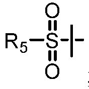 and
      subjecting the compound u and the compound 10 to an eleventh nucleophilic substitution to obtain the memantyl urea derivative having the structure shown in Formula B where Y is 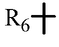 where
      the compound 13 has a structural formula shown as 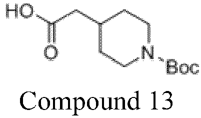 and
      structural formulas of the compound p, the compound q, the compound r, the compound s, the compound t, and the compound u are as follows: 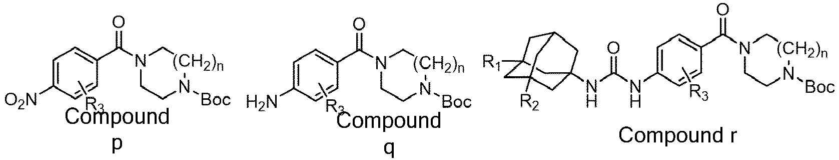 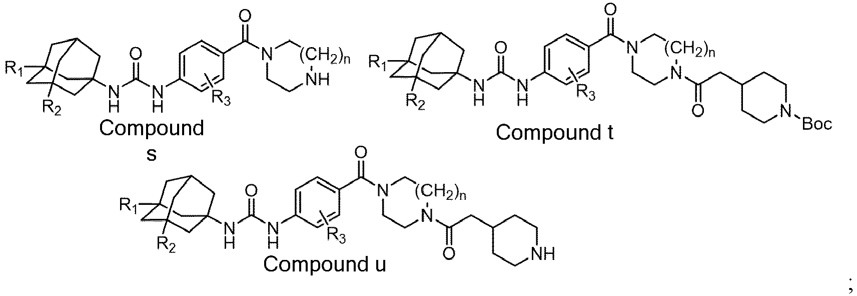
   (2-2) under the condition that the memantyl urea derivative has the structure shown in Formula B where X is -CH₂-, the method includes the following steps:
      subjecting the compound g and the compound 7 to a twelfth nucleophilic substitution to obtain a compound v;
      subjecting the compound v to a sixth reduction to obtain a compound w;
      subjecting the intermediate compound and the compound w to a thirteenth nucleophilic substitution to obtain a compound x, the compound x being the memantyl urea derivative having the structure shown in Formula B where Y is -H;
      mixing the compound x with the compound 8 and conducting an eighteenth acylation in the presence of HATU and DIEA to obtain the memantyl urea derivative having the structure shown in Formula B where Y is 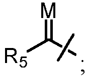
      subjecting the compound x and the compound 9 to a nineteenth acylation to obtain the memantyl urea derivative having the structure shown in Formula B where Y is 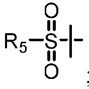 and
      subjecting the compound x and the compound 10 to a fourteenth nucleophilic substitution to obtain the memantyl urea derivative having the structure shown in Formula B where Y is 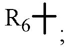 where
      structural formulas of the compound v, the compound w, and the compound x are as follows:

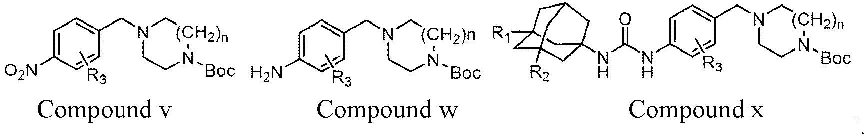

The present disclosure provides use of the above-mentioned memantyl urea derivative or the memantyl urea derivative prepared by the above-mentioned method in preparation of a drug for treating a soluble epoxide hydrolase (sEH)-mediated disease.

In some embodiments, the sEH-mediated disease includes an inflammatory disease, a pain, a cardiovascular disease, a neurodegenerative disease, diabetes, a diabetic complication, chronic nephritis, renal failure, a chronic obstructive pulmonary disease, or pulmonary hypertension.

The present disclosure provides a memantyl urea derivative. In the present disclosure, the memantyl urea derivative has a typical urea structure as a primary pharmacophore of sEH; and a memantine part, as a hydrophobic fragment, generates hydrophobic forces with a receptor. Molecular docking shows that a memantine part, as a hydrophobic fragment, generates hydrophobic forces with a receptor. Especially when R₁ and R₂ both are methyl (i.e., 3,5-dimethyl substituted), the Van der Waals force can be enhanced. Therefore, the memantyl urea derivative shows a high inhibitory activity against HsEH and MsEH, and can be used as an sEH inhibitor in preparation of a drug for treating sEH-mediated diseases.

### BRIEF DESCRIPTION OF THE DRAWINGS

FIG. 1 shows a reaction route of the memantyl urea derivative having a structure shown in Formula A in the present disclosure;
FIG. 2 shows a reaction route of the memantyl urea derivative having a structure shown in Formula A in the present disclosure;
FIG. 3 shows a reaction route of the memantyl urea derivative having a structure shown in Formula A in the present disclosure;
FIG. 4 shows a reaction route of the memantyl urea derivative having a structure shown in Formula A in the present disclosure;
FIG. 5 shows a reaction route of the memantyl urea derivative having a structure shown in Formula A in the present disclosure;
FIG. 6 shows a reaction route of the memantyl urea derivative having a structure shown in Formula A in the present disclosure;
FIG. 7 shows a reaction route of the memantyl urea derivative having a structure shown in Formula B in the present disclosure; and
FIG. 8 shows a reaction route of the memantyl urea derivative having a structure shown in Formula B in the present disclosure.

### DETAILED DESCRIPTION OF THE EMBODIMENTS

The present disclosure provides a memantyl urea derivative having a structure shown in Formula A and Formula B: 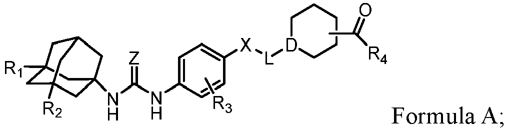 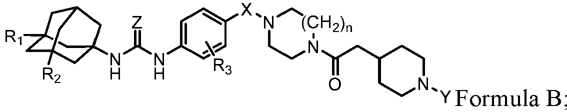 where
R₁ and R₂ are independently selected from the group consisting of -H, -OH, -NH₂, -SH, -CN, a halogen group, alkyl, alkoxy, aryl, and heteroaryl;
R₃ is selected from the group consisting of -H, -OH, -NH₂, -SH, -CN, a halogen group, alkyl, and alkoxy;
R₄ is selected from the group consisting of -OH, -NH₂, hydroxylamino, alkyl-substituted amino, alkoxy-substituted amino, alcohol group-substituted amino, phenyl-substituted amino, naphthyl-substituted amino, and 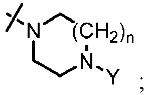
Y is selected from the group consisting of -H, 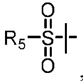 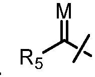 and 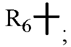
R₅ is selected from the group consisting of alkyl and a heterocyclic group, and the alkyl is selected from the group consisting of chain alkyl and cycloalkyl;
R₆ is selected from the group consisting of alkyl-substituted amino and alcohol group-substituted alkyl;
X is selected from the group consisting of -CH₂- and 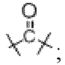
L is absent or -NH-;
D is selected from the group consisting of 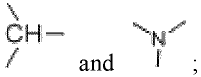
n is 1 or 2; and
Z and M are independently selected from the group consisting of =O and =S.

In some embodiments, R₁ and R₂ are independently selected from the group consisting of -H, -OH, -NH₂, -SH, -CN, -F, -Cl, -Br, methyl, ethyl, propyl, butyl, pentyl, isobutyl, isopropyl, isopentyl, tert-butyl, methoxy, ethoxy, propoxy, isopropoxy, butoxy, cyclopentyloxy, cyclohexyloxy, phenoxy, and benzyloxy. Further, in some embodiments, R₁ and R₂ both are methyl.

In some embodiments of the present disclosure, R₃ is selected from the group consisting of -H, -OH, -NH₂, -SH, -CN, -F, -Cl, -Br, unsubstituted or substituted C1-C6 alkyl, and unsubstituted or substituted C1-C6 alkoxy; and a substituent on the substituted C1-C6 alkyl and the substituted C1-C6 alkoxy is independently selected from the group consisting of -F, -Cl, -Br, -OH, -NH₂, -NHCH₃, -N(CH₃)₂, and C1-C6 alkyl. Further, in some embodiments, R₃ is -F.

In some embodiments of the present disclosure, the alkyl-substituted amino for R₄ is unsubstituted or substituted C1-C6 alkyl-substituted amino; the alkoxy-substituted amino for R₄ is unsubstituted or substituted C1-C6 alkoxy-substituted amino; the phenyl-substituted amino for R₄ is unsubstituted or substituted phenyl-substituted amino; the naphthyl-substituted amino for R₄ is unsubstituted or substituted naphthyl-substituted amino; the alcohol group-substituted amino for R₄ is unsubstituted or substituted C1-C6 alcohol group-substituted amino; and a substituent on substituted C1-C6 alkoxy, substituted phenyl, substituted naphthyl, and substituted C1-C6 alcohol group is independently selected from the group consisting of -F, -Cl, -Br, -OH, -NH₂, -NHCH₃, -N(CH₃)₂, and C1-C6 alkyl. Further, in some embodiment, R₄ is selected from the group consisting of -OH, -NH₂, -N(CH₃)₂, -N(CH₂CH₃)₂, -N(CH₂CH₂OH)₂, -NHCH(CH2OH)₂, -NHC(CH₂OH)₃, -NHCH₂CH₂OH, -NHCH₂CHCH₃OH, -N(CH₃)₂,

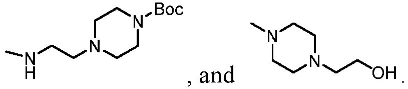

In some embodiments of the present disclosure, the chain alkyl for R₅ is unsubstituted or substituted C1-C6 saturated or unsaturated chain alkyl, and a substituent on the substituted C1-C6 saturated or unsaturated chain alkyl is selected from the group consisting of -OH, -NH₂, and C1-C6 alkyl; the cycloalkyl for R₅ is unsubstituted or substituted C3-C6 cycloalkyl, and a substituent on the substituted C3-C6 cycloalkyl is selected from the group consisting of -F, -Cl, -Br, -OH, -NH₂, -NHCH₃, -N(CH₃)₂, and C1-C6 alkyl; and the heterocyclic group for R₅ is an unsubstituted or substituted C3-C6 saturated or unsaturated heterocyclic group, and a substituent on the substituted C3-C6 saturated or unsaturated heterocyclic group is selected from the group consisting of -F, -Cl, -Br, -OH, -NH₂, -NHCH₃, -N(CH₃)₂, and C1-C6 alkyl. Further, in some embodiments, R₅ is selected from the group consisting of -CH₃, -CH₂CH₃, -CHCH₃CH₂CH₃, -OCH₃,

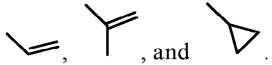

In some embodiemnts of the present disclosure, the alkyl-substituted amino for R₆ is unsubstituted or substituted C1-C6 alkyl-substituted amino, and the alcohol group-substituted alkyl is unsubstituted or substituted C1-C6 alcohol group-substituted alkyl. Further, in some embodiments, R₆ is selected from the group consisting of -CHCH₃CH₂N(CH₃)₂, -CH₂CH₂N(CH₂CH₃)₂, -CH₂CH₂N(CH₃)₂, -CH₂CH₂OH, and -CH₂CHOHCH₂OH.

In some embodiments of the present disclosure, n is 2.

In some embodiments of the present disclosure, the memantyl urea derivative includes but is not limited to (S)-1-(4-(3-((1r,3R,5S,7S)-3,5-dimethyladamantan-1-yl)ureido)-3-fluorobenzyl)piperidine-3-car boxamide (**CL-101**), 1-((1r,3R,5S,7S)-3,5-dimethyladamantan-1-yl)-3-(2-fluoro-4-(((S)-3-(4-propionyl-1,4-)diaza-1-c arbonyl)piperidin-1-yl)methyl)phenyl)urea (**CL-102**), 1-((1r,3R,5S,7S)-3,5-dimethyladamantan-1-yl)-3-(2-fluoro-4-(((3S)-3-(4-(2-methylbutanoyl)-1,4 -diaza-1-carbonyl)piperidin-1-yl)methyl)phenyl)urea (**CL-103**), (S)-1-(4-(3-((1r,3R,5S,7S)-3,5-dimethyladamantan-1-yl)ureido)-3-fluorobenzyl)-N,N-dimethylpi peridine-3-carboxamide (**CL-105**), (S)-1-(4-(3-((1r,3R,5S,7S)-3,5-dimethyladamantan-1-yl)ureido)-3-fluorobenzyl)-N,N-diethylpip eridine-3-carboxamide (**CL-106**), (S)-1-(4-(3-((1 r,3R, 5 S, 7S)-3, 5-dimethyladamantan-1-yl)ureido)-3-fluorobenzyl)-N,N-bis(2-hydr oxyethyl)piperidine-3-carboxamide (**CL-107**), 1-(4-(3-((1r,7r)-3,5-dimethyladamantan-1-yl)ureido)-3-fluorobenzyl)-N,N-bis(2-hydroxyethyl)pi peridine-4-carboxamide (**CL-109**), (S)-N-(1,3-dihydroxypropan-2-yl)-1-(4-(3-((1r,3R,5S,7S)-3,5-dimethyladamantan-1-yl)ureido)-3 -fluorobenzyl)piperidine-3-carboxamide (**CL-112**), (3S)-1-(4-(3-((1r,3R,5S,7S)-3,5-dimethyladamantan-1-yl)ureido)-3-fluorobenzyl)-N-(2-hydroxy propyl)piperidine-3-carboxamide (**CL-113**), (S)-1-(4-(3-((1r,3R,5S,7S)-3,5-dimethyladamantan-1-yl)ureido)-3-fluorobenzyl)-N-(2-hydroxyet hyl)piperidine-3-carboxamide (**CL-114**), (S)-N-(1,3-dihydroxy-2-(hydroxymethyl)propan-2-yl)-1-(4-(3-((1r,3R,5S,7S)-3,5-dimethyladam antan-1-yl)ureido)-3-fluorobenzyl)piperidine-3-carboxamide (**CL-115**), 1-((1r,3R,5S,7S)-3,5-dimethyladamantan-1-yl)-3-(2-fluoro-4-(((S)-3-(4-(2-hydroxyethyl)piperazi ne-1-carbonyl)piperidin-1-yl)methyl)phenyl)urea (**CL-116**), N-(1,3-dihydroxy-2-(hydroxymethyl)propan-2-yl)-1-(4-(3-((1r,7r)-3,5-dimethyladamantan-1-yl) ureido)-3-fluorobenzyl)piperidine-4-carboxamide (**CL-117**), 1-(4-(3 -((1r,7r)-3,5-dimethyladamantan-1-yl)ureido)-3-fluorobenzyl)piperidine-4-carboxamide (**CL-118**), N-(1,3-dihydroxypropan-2-yl)-1-(4-(3-((1r,7r)-3,5-dimethyladamantan-1-yl)ureido)-3-fluorobenz yl)piperidine-4-carboxamide (**CL-119**), 1-(4-(3-((1r,7r)-3,5-dimethyladamantan-1-yl)ureido)-3-fluorobenzyl)-N-(2-hydroxypropyl)piperi dine-4-carboxamide (**CL-120**), 1-(4-(((S)-3-(4-acryloyl-1,4-diaza-1-carbonyl)piperidin-1-yl)methyl)-2-fluorophenyl)-3-((1r,3R,5 S,7S)-3,5-dimethyladamantan-1-yl)urea (**CL-201**), 1-(4-((S)-3-(4-(cyclopropanecarbonyl)-1,4-diaza-1-carbonyl)piperidin-1-yl)methyl)-2-fluorophe nyl)-3-((1r,3R,5S,7S)-3,5-dimethyladamantan-1-yl)urea (**CL-204**), 1-((1r,3R,5S,7S)-3,5-dimethyladamantan-1-yl)-3-(2-fluoro-4-(((3 S)-3-(1-(2-methylbutyryl)azacy clohexane-4-carbonyl)piperidin-1-yl)methyl)phenyl)urea (**CL-205**), (S)-1-(4-(3-((1r,3R,5S,7S)-3,5-dimethyladamantan-1-yl)ureido)-3-fluorobenzyl)piperidine-3-car boxylic acid (**CL-5**), 1-((1r,3R,5S,7r)-3,5-dimethyladamantan-1-yl)-3-(2-fluoro-4-(4-(2-(1-(2-methylbutyryl)piperidin -4-yl)acetyl)-1,4-diaza-1-carbonyl)phenyl)urea (**CC-103**), 1-((1r,3R,5S,7r)-3,5-dimethyladamantan-1-yl)-3-(2-fluoro-4-(4-(2-(1-propionylpiperidin-4-yl)ac etyl)-1,4-diaza-1-carbonyl)phenyl)urea (**CC-105**), 1-(4-(4-(2-(1-acetylpiperidin-4-yl)acetyl)-1,4-diaza-1-carbonyl)-2-fluorophenyl)-3-((1r,3R,5S,7r) -3,5-dimethyladamantan-1-yl)urea (**CC-106**), (S)-1-(4-(3-((1r,3R,5S,7S)-3,5-dimethyladamantan-1-yl)ureido)-3-fluorobenzoyl)-N,N-diethylpi peridine-3-carboxamide (**CC-110**), 1-(4-(3-((1r,3R,5S,7r)-3,5-dimethyladamantan-1-yl)ureido)-3-fluorobenzoyl)-N,N-diethylpiperid ine-4-carboxamide (**CC-111**), 1-((1r,3R,5S,7r)-3,5-dimethyladamantan-1-yl)-3-(2-fluoro-4-(4-(4-(2-hydroxyethyl)piperazine-1-carbonyl)piperidine-1-carbonyl)phenyl)urea (**CC-112**), (3S)-1-(4-(3-((1r,3R,5S,7S)-3,5-dimethyladamantan-1-yl)ureido)-3-fluorobenzoyl)-N-(2-hydrox ypropyl)piperidine-3-carboxamide (**CC-113**), 1-(4-(3-((1r,3R,5S,7r)-3,5-dimethyladamantan-1-yl)ureido)-3-fluorobenzoyl)-N-(2-hydroxyethyl )piperidine-4-carboxamide (**CC-114**), 1-(4-(3-((1r,3R,5S,7r)-3,5-dimethyladamantan-1-yl)ureido)-3-fluorobenzoyl)-N-(2-hydroxyprop yl)piperidine-4-carboxamide (**CC-115**), N-(1,3-dihydroxypropan-2-yl)-1-(4-(3-((1r,3R,5S,7r)-3,5-dimethyladamantan-1-yl)ureido)-3-flu orobenzoyl)piperidine-4-carboxamide (**CC-116**), (S)-1-(4-(3-((1r,3R,5S,7S)-3,5-dimethyladamantan-1-yl)ureido)-3-fluorobenzoyl)-N-(2-hydroxye thyl)piperidine-3-carboxamide (**CC-120**), 1-((1r,3R,5S,7S)-3,5-dimethyladamantan-1-yl)-3-(2-fluoro-4-((S)-3-(4-(2-hydroxyethyl)piperazi ne-1-carbonyl)piperidine-1-carbonyl)phenyl)urea (**CC-121**), 1-((1r,3R,5S,7r)-3,5-dimethyladamantan-1-yl)-3-(4-(4-(2-(1-(1-(dimethylamino)propan-2-yl)pip eridin-4-yl)acetyl)-1,4-diaza-1-carbonyl)-2-fluorophenyl)urea (**CC-124**), 1-((1r,3R,5S,7r)-3,5-dimethyladamantan-1-yl)-3-(2-fluoro-4-((4-(2-(1-propionylpiperidin-4-yl)ac etyl)-1,4-diaza-1-yl)methyl)phenyl)urea (**GL-1**), 1-((1r,3R,5S,7r)-3,5-dimethyladamantan-1-yl)-3-(2-fluoro-4-((4-(2-(1-(2-methylbutyryl)piperidi n-4-yl)acetyl)-1,4-diaza-1-yl)methyl)phenyl)urea (**GL-2**), 1-(4-((4-(2-(1-acetylpiperidin-4-yl)acetyl)-1,4-diaza-1-yl)methyl)-2-fluorophenyl)-3-((1r,3R,5S, 7r)-3,5-dimethyladamantan-1-yl)urea (**GL-3**), methyl 4-(2-(4-(4-(3-((1r,3R,5S,7r)-3,5-dimethyladamantan-1-yl)ureido)-3-fluorobenzyl)-1,4-diaza-1-yl) -2-oxoethyl)piperidine-1-carboxylate (**GL-4**), 1-((1r,3R,5S,7r)-3,5-dimethyladamantan-1-yl)-3-(2-fluoro-4-((4-(2-(1-(2-hydroxyethyl)piperidin -4-yl)acetyl)-1,4-diaza-1-yl)methyl)phenyl)urea (**GL-21**), 1-(4-((4-(2-(1-(2,3-dihydroxypropyl)piperidin-4-yl)acetyl)-1,4-diaza-1-yl)methyl)-2-fluoropheny 1)-3-((1r,3R,5S,7r)-3,5-dimethyladamantan-1-yl)urea (**GL-22**), 1-((1r,3R,5S,7r)-3,5-dimethyladamantan-1-yl)-3-(4-((4-(2-(1-(2-(dimethylamino)ethyl)piperidin-4-yl)acetyl)-1,4-diaza-1-yl)methyl)-2-fluorophenyl)urea (**GL-23**), 1-(4-((4-(2-(1-(2-(diethylamino)ethyl)piperidin-4-yl)acetyl)-1,4-diaza-1-yl)methyl)-2-fluorophen yl)-3-((1r,3R,5S,7r)-3,5-dimethyladamantan-1-yl)urea (**GL-24**), 1-(4-((S)-3-(4-acetyl-1,4-diaza-1-carbonyl)piperidine-1-carbonyl)-2-fluorophenyl)-3-((1r,3R,5S, 7S)-3,5-dimethyladamantan-1-yl)urea (**GL-101**), 1-((1r,3R,5S,7S)-3,5-dimethyladamantan-1-yl)-3-(2-fluoro-4-((S)-3-(4-propionyl-1,4-diaza-1-car bonyl)piperidine-1-carbonyl)phenyl)urea (**GL-102**), 1-((1r,3R,5S,7S)-3,5-dimethyladamantan-1-yl)-3-(2-fluoro-4-((3S)-3-(4-(2-methylbutanoyl)-1,4-diaza-1-carbonyl)piperidine-1-carbonyl)phenyl)urea (**GL-104**), 4-(3-((1r,3R,5S,7r)-3,5-dimethyladamantan-1-yl)ureido)-3-fluoro-N-((1r,4R)-4-((2-hydroxyethyl )carbamoyl)cyclohexyl)benzamide (**ZT-110**), 4-(3-((1r,3R,5S,7r)-3,5-dimethyladamantan-1-yl)ureido)-3-fluoro-N-((1r,4R)-4-((2-hydroxyprop yl)carbamoyl)cyclohexyl)benzamide (**ZT-111**), N-((1r,4R)-4-(bis(2-hydroxyethyl)carbamoyl)cyclohexyl)-4-(3-((1r,3R,5S,7r)-3,5-dimethyladam antan-1-yl)ureido)-3-fluorobenzamide (**ZT-112**), N-((1r,4R)-4-carbamoylcyclohexyl)-4-(3-((1r,3R,5S,7r)-3,5-dimethyladamantan-1-yl)ureido)-3-f luorobenzamide (**ZT-113**), N-((1r,4R)-4-((1,3-dihydroxypropan-2-yl)carbamoyl)cyclohexyl)-4-(3-((1r,3R,5S,7r)-3,5-dimeth yladamantan-1-yl)ureido)-3-fluorobenzamide (**ZT-114**), N-((1r,4R)-4-((1,3-dihydroxy-2-(hydroxymethyl)propan-2-yl)carbamoyl)cyclohexyl)-4-(3-((1r,3 R,5S,7r)-3,5-dimethyladamantan-1-yl)ureido)-3-fluorobenzamide (**ZT-115**), tert-butyl 4-(2-((1R,4r)-4-(4-(3-((1r,3R,5S,7r)-3,5-dimethyladamantan-1-yl)ureido)-3-fluorobenzamido)cy clohexane-1-carboxamido)ethyl)piperazine-1-carboxylate (**ZT-116**), 4-(3-((1r,3R,5S,7r)-3,5-dimethyladamantan-1-yl)ureido)-3-fluoro-N-((1r,4R)-4-((2-hydroxyethyl )carbamoyl)cyclohexyl)benzamide (**ZT-117**), where their structural formulas thereof are as follows:

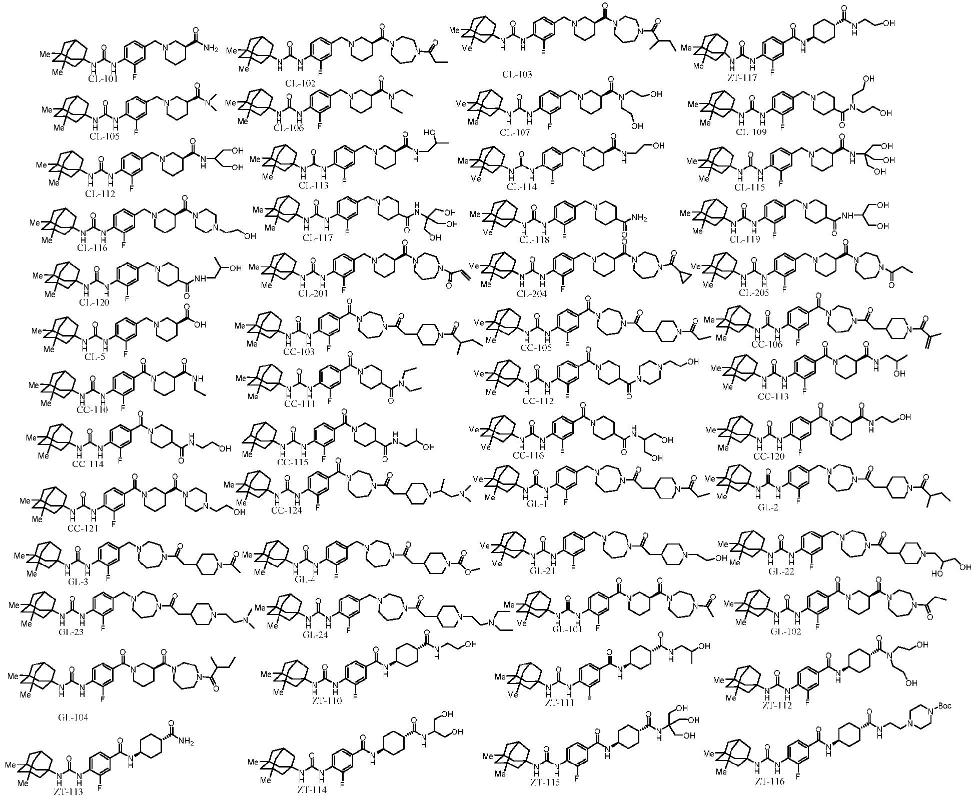

The present disclosure provides a method for preparing the memantyl urea derivative. In the present disclosure, the method is selected according to the specific structure of the memantyl urea derivatives, and specific descriptions are given below.

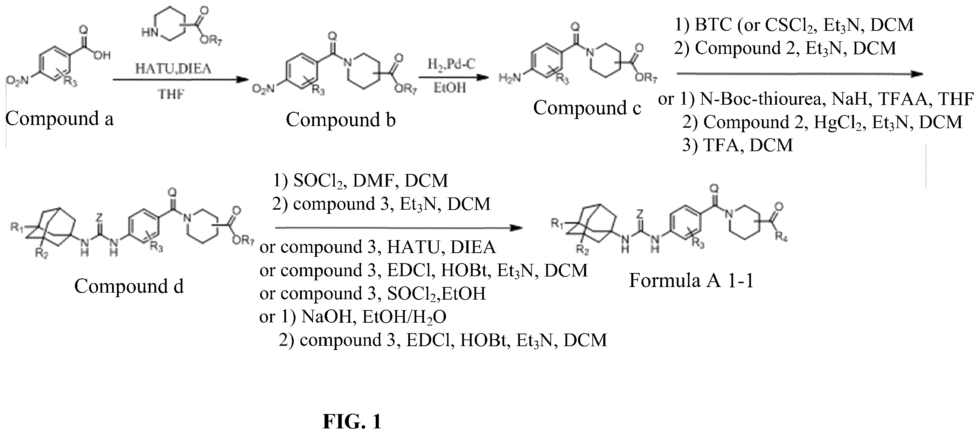

In a first case, the memantyl urea derivative having a structure shown in Formula A is prepared. The specific method as shown in FIG. 1 includes the following steps:
(1-1) under the condition that the memantyl urea derivative has the structure shown in Formula A where X is 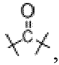 L is absent, D is 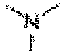 and R₄ is not 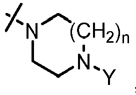 the method includes the following steps:
subjecting a compound 1 and a compound a to a first acylation to obtain a compound b;
subjecting the compound b to a first reduction to obtain a compound c;
subjecting a compound 2 and a compound 4 to a second acylation to obtain an intermediate compound 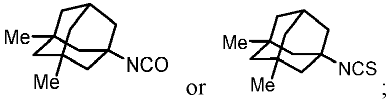
subjecting the intermediate compound and the compound c to a first nucleophilic substitution to obtain a compound d;
subjecting the compound d to a first hydrolysis to obtain the memantyl urea derivative having the structure shown in Formula A where R₄ is -OH; and
mixing the memantyl urea derivative having the structure shown in Formula A where R₄ is -OH with a compound 3, and subjecting a resulting mixture to a third acylation in the presence of 2-(7-azabenzotriazol-1-yl)-*N*,*N*,*N'*,*N'*-tetramethyluronium hexafluorophosphate (HATU) and *N,N*-diisopropylethylamine (DIEA) to obtain the memantyl urea derivative having the structure shown in Formula A; where
structural formulas of the compound 1 and the compound 2 are 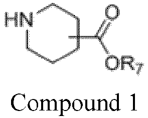 and 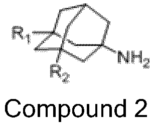 respectively;
   the compound 3 is selected from the group consisting of NH₃, hydroxylamine, alkylamine, alkoxyamine, alcoholamine, phenylamine, and naphthylamine;
   the compound 4 is selected from the group consisting of bis(trichloromethyl)carbonate (BTC) and thiophosgene;
   structural formulas of the compound a, the compound b, the compound c, the compound d, and the compound e are as follows: 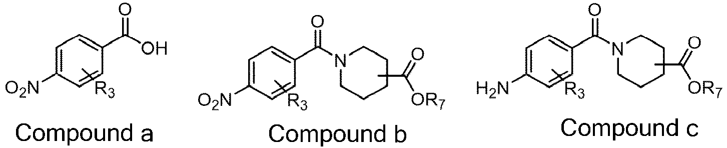 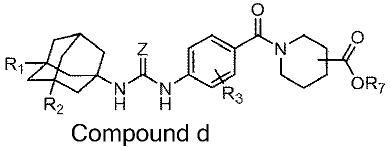 where
   in the structural formulas of the compound 1, the compound b, the compound c, and the compound d, R₇ is C₁-C₆ alkyl.

In the present disclosure, a compound 1 and a compound a are subjected to a first acylation to obtain a compound b. In some embodiments, a molar ratio of the compound 1, the compound a, an organic alkali, and a condensing agent is in a range of (1.0-1.5):1:(2-4):(1.2-2), preferably 1.2:1:3:1.5. In some embodiments, the organic alkali includes triethylamine (TEA), pyridine, or DIEA, preferably is the DIEA. In some embodiments, the condensing agent includes HATU, 1-(3-dimethylaminopropyl)-3-ethylcarbodiimide hydrochloride (EDCl), or *N,N*-dicyclohexylcarbodiimide (DCCI), preferably is the HATU. In some embodiments, an organic solvent used includes dichloromethane (DCM), chloroform, and THF, preferably is the THF. In some embodiments, the first acylation is conducted at a temperature of 10 °C to 30 °C, preferably 25 °C; the first acylation is conducted for preferably 30 min to 60 min, more preferably 30 min. In some embodiments, after the first acylation is completed, an obtained reaction solution is concentrated under reduced pressure to remove the solvent, water is added thereto, ethyl acetate is added for extraction, and an obtained organic phase is dried, filtered, and concentrated to obtain the compound b.

In the present disclosure, the compound b is subjected to first reduction to obtain a compound c. In some embodiments, a molar ratio of the compound b to the catalyst is in a range of 1:(0.1-0.2), preferably 1:0.1. In some embodiments, a reducing agent in the first reduction is hydrogen, and a catalyst used is preferably Pd-C; the first reduction is preferably conducted in the presence of anhydrous ethanol. In some embodiments, the first reduction is conducted at a temperature of 25 °C to 70 °C, preferably 60 °C; the first reduction is conducted for preferably 10 h to 15 h, more preferably 12 h. In some embodiments, after the first reduction is completed, a reaction solution is cooled to room temperature and then filtered, and a filtrate is concentrated under reduced pressure to obtain the compound c.

In the present disclosure, a compound 2 and a compound 4 are subjected to a second acylation to obtain an intermediate compound. In some embodiments, during the preparation of the intermediate compound, a molar ratio of the compound 2 to the compound 4 is in a range of 1:(0.3-0.5), preferably 1:0.5. In some embodiments, a reaction for preparing the intermediate compound is conducted in the presence of TEA and DCM. In some embodiments, the reaction for preparing the intermediate compound is conducted at a temperature of -78 °C to -30 °C, preferably -78 °C; the reaction for preparing the intermediate compound is conducted for preferably 30 min to 60 min, more preferably 30 min. In some embodiments, after the intermediate compound is prepared, an obtained reaction solution is concentrated under reduced pressure at 40 °C for later use.

In the present disclosure, the intermediate compound and the compound c are subjected to a first nucleophilic substitution to obtain a compound d. In some embodiments, during the first nucleophilic substitution, a molar ratio of the compound c to TEA is in a range of 1:(4-6), preferably 1:6. In some embodiments, the first nucleophilic substitution is conducted in the presence of TEA and DCM. In some embodiments, the first nucleophilic substitution is conducted at a temperature of -10 °C to -30 °C, preferably 25 °C; preferably the first nucleophilic substitution is conducted for 30 min to 60 min, more preferably 30 min. After the first nucleophilic substitution is completed, water is added to the reaction product, and ethyl acetate is added for extraction, followed by drying, filtering with suction, and concentrating to obtain the compound d.

In the present disclosure, the compound d is subjected to a first hydrolysis to obtain the memantyl urea derivative having a structure shown in Formula A where R₄ is -OH. The memantyl urea derivative having the structure shown in Formula A where R₄ is -OH is mixed with a compound 3, and a resulting mixture is subjected to a third acylation in the presence of HATU and DIEA to obtain the memantyl urea derivative having the structure shown in Formula A. In some embodiments, a molar ratio of the compound d to the compound 3 is in a range of 1:(1.5-2.0), preferably 1:2. In some embodiments, a molar ratio of the compound d, the HATU, and the DIEA is in a range of 1:(1.2-1.7):(2-3), preferably 1:1.2:2.4. In some embodiments, the third acylation is conducted at a temperature of 25 °C to 35 °C, preferably 30 °C; preferably the third acylation is conducted for 1 h to 3 h, preferably for 2 h. In some embodiments, the method further includes, after the third acylation is completed, an obtained reaction solution is poured into water, and a resulting mixture is extracted with DCM, and a resulting filtrate is then washed with 1 mol/L hydrochloric acid, 5 wt% sodium carbonate aqueous solution, water, and saturated aqueous NaCl solution, and dried over anhydrous sodium sulfate, followed by filtering with suction. An obtained filtrate is concentrated under reduced pressure, followed by column chromatography, to obtain the memantyl urea derivative having the structure shown in Formula A. In the examples, the compound may be CC-110, CC-111, CC-112, CC-113, CC-114, CC-115, CC-116, CC-120, or CC-121.

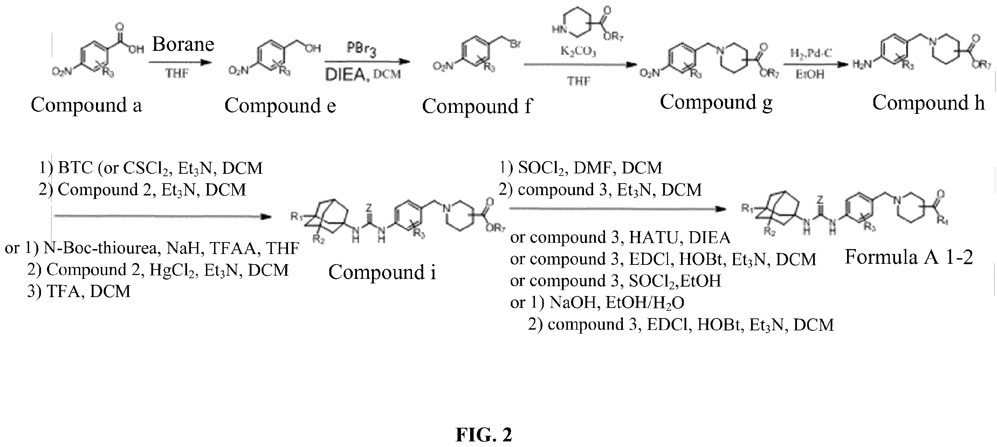

In a second case, a memantyl urea derivative having a structure shown in Formula A is prepared. The specific method as shown in FIG. 2 includes the following steps:
(1-2) under the condition that the memantyl urea derivative has the structure shown in Formula A where X is -CH₂-, L is absent, D is 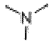 and R₄ is not 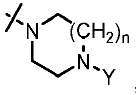 the method includes the following steps:
subjecting the compound a to a second reduction to obtain a compound e;
subjecting the compound e and a bromination reagent to a second nucleophilic substitution to obtain a compound f;
subjecting the compound f and the compound 1 to a third nucleophilic substitution to obtain a compound g;
subjecting the compound g to a third reduction to obtain a compound h;
subjecting the intermediate compound and the compound h to a fourth nucleophilic substitution to obtain a compound i;
subjecting the compound i to a second hydrolysis to obtain the memantyl urea derivative having the structure shown in Formula A where R₄ is -OH; and
mixing the memantyl urea derivative having the structure shown in Formula A where R₄ is -OH with the compound 3, and subjecting the obtained mixture to a fourth acylation in the presence of HATU and DIEA to obtain the memantyl urea derivative having the structure shown in Formula A; where
structural formulas of the compound e, the compound f, the compound g, the compound h, and the compound i are as follows: 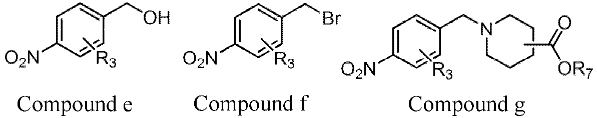 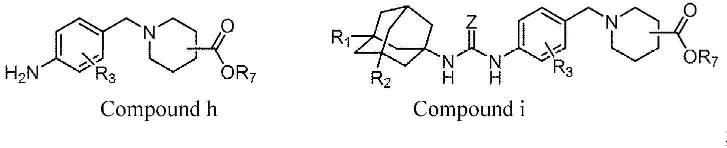 and
in structural formulas of the compound g, the compound h, and the compound i, R₇ is C₁-C₆ alkyl.

In the present disclosure, the compound a is subjected to a second reduction to obtain a compound e. In some embodiments, a molar ratio of the compound a to a reducing agent used is in a range of 1:(1.2-3), preferably 1:2. In some embodiments, the reducing agent includes borane, lithium aluminum tetrahydride, or diisobutylaluminum hydride, preferably the borane, and an organic solvent used preferably includes acetonitrile, acetone, and THF, preferably the THF. In some embodiments, the second reduction is conducted at a temperature of -10 °C to 10 °C, preferably 0 °C; preferably the second reduction is conducted for 8 to 12 h, preferably 10 h. In some embodiments, after the second reduction is completed, an obtained reaction solution is concentrated under reduced pressure to obtain the compound e.

In the present disclosure, after obtaining the compound e, the compound e and a bromination reagent are subjected to a second nucleophilic substitution to obtain a compound f. In some embodiments, a molar ratio of the compound e to the bromination reagent is in a range of 1:(1.2-2), preferably 1:1.5. In some embodiments, the bromination reagent includes hydrogen bromide, sulfoxide bromide, phosphorus tribromide, phosphorus pentabromide, or carbon tetrabromide, more preferably the phosphorus tribromide; an organic solvent used preferably includes acetonitrile, DCM, and THF, preferably the DCM; an organic alkali preferably includes TEA, pyridine, or DIEA, more preferably the DIEA; the second nucleophilic substitution is preferably conducted at a temperature of 25 °C to 50 °C, more preferably 30 °C; the second nucleophilic substitution is preferably conducted for 8 h to 15 h, more preferably 10 h. After the second nucleophilic substitution is completed, water is added to the reaction product, and ethyl acetate is added for extraction, followed by drying, filtering with suction, and concentrating to obtain the compound f.

In the present disclosure, the compound f and the compound 1 are subjected to a third nucleophilic substitution to obtain a compound g. In some embodiments, a molar ratio of the compound f to the compound 1 is in a range of 1:(1.2-2), preferably 1:1.5; a molar ratio of the compound f to an acid-binding agent used is preferably in a range of 1: (2-4), more preferably 1:2; the organic solvent used preferably includes acetonitrile, acetone, and THF, preferably the THF; the acid-binding agent preferably includes TEA, DIEA, 1,8-diazabicyclo [5.4.0] undec-7-ene, potassium carbonate, cesium carbonate, or sodium carbonate, more preferably the potassium carbonate; the third nucleophilic substitution is preferably conducted at a temperature of 50 °C to 80 °C, more preferably 60 °C; the third nucleophilic substitution is preferably conducted for preferably 4 h to 8 h, more preferably 6 h. After the third nucleophilic substitution is completed, water is added to the reaction product, and ethyl acetate is added for extraction, followed by drying, filtering with suction, and concentrating, to obtain the compound g.

In the present disclosure, the compound g is subjected to a third reduction to obtain a compound h. In some embodiments, the reducing agent used in the third reduction, the type of the third catalyst, the conditions of the third reduction, and the post-treatment are consistent with the reducing agent used in the first reduction, the type of the first catalyst, the first reduction, and the post-treatment in step (1-1), and will not be repeated here.

In the present disclosure, after obtaining the compound h, the intermediate compound and the compound h are subjected to a fourth nucleophilic substitution to obtain a compound i. In some embodiments, the reaction conditions and post-treatment used in the fourth nucleophilic substitution are consistent with the reaction conditions and post-treatment of the first nucleophilic substitution in step (1-1), and will not be repeated here.

In the present disclosure, after obtaining the compound i, the compound i is subjected to a second hydrolysis to obtain the memantyl urea derivative having the structure shown in Formula A where R₄ is -OH; and the memantyl urea derivative having the structure shown in Formula A where R₄ is -OH is mixed with the compound 3, and a resulting mixture is subjected to a fourth acylation in the presence of HATU and DIEA to obtain the memantyl urea derivative having the structure shown in Formula A. In some embodiments, the reaction conditions and post-treatment used in the fourth acylation are consistent with the reaction conditions and post-treatment of the third acylation in step (1-1), and will not be repeated here. In the examples, the compound may be CL-101, CL-105, CL-106, CL-107, CL-109, CL-112, CL-114, CL-115, CL-116, CL-117, CL-118, CL-119, or CL-120.

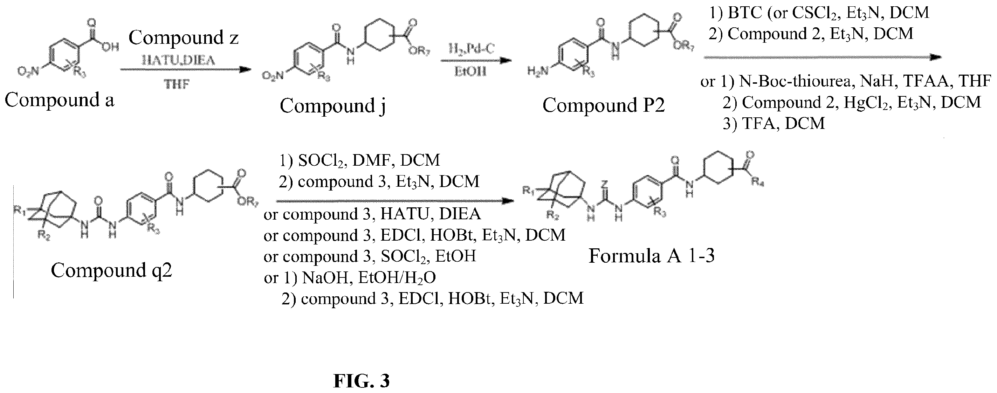

In a third case, a memantyl urea derivative having a structure shown in Formula A is prepared. The specific method as shown in FIG. 3 includes the following steps:
(1-3) under the condition that the memantyl urea derivative has the structure shown in Formula A where X is 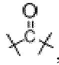 L is -NH-, D is 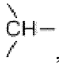 and R₄ is not 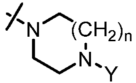 the method includes the following steps:
subjecting a compound z and the compound a to a fifth acylation to obtain a compound j;
subjecting the intermediate compound and the compound j to a fifth nucleophilic substitution to obtain a compound y;
subjecting the compound y to a third hydrolysis to obtain the memantyl urea derivative having the structure shown in Formula A where R₄ is -OH; and
mixing the memantyl urea derivative having the structure shown in Formula A where R₄ is -OH with the compound 3, and subjecting a mixture thereof to a sixth acylation in the presence of HATU and DIEA to obtain the memantyl urea derivative having the structure shown in Formula A; where
the compound z has a structural formula shown as 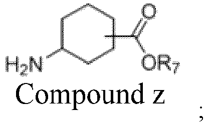
the compound j has a structural formula shown as 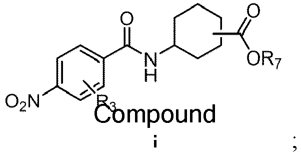
the compound y has a structural formula shown as 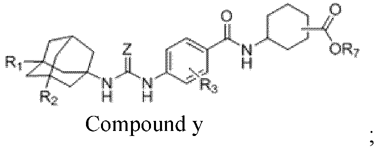 and in structural formulas of the compound z, the compound j, and the compound y, R₇ is C₁-C₆ alkyl.

In the present disclosure, a compound z and the compound a are subjected to a fifth acylation to obtain a compound j. In some embodiments, the reaction conditions and post-treatment used in the fifth acylation are consistent with the reaction conditions and post-treatment of the first acylation in step (1-1), and will not be repeated here.

In the present disclosure, the intermediate compound and the compound j are subjected to a fifth nucleophilic substitution to obtain a compound y. According to step (1-1), the memantyl urea derivative having the structure shown in Formula A is prepared. In the examples, the compound may be ZT-110, ZT-111, ZT-112, ZT-113, ZT-114, ZT-115, ZT-116, or ZT-117.

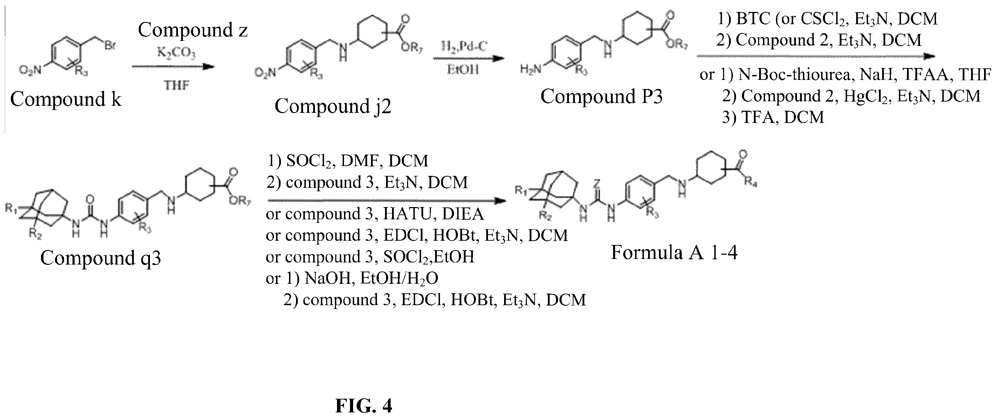

In a fourth case, a memantyl urea derivative having a structure shown in Formula A is prepared. The specific method as shown in FIG. 4 includes the following steps:
(1-4) under the condition that the memantyl urea derivative has the structure shown in Formula A where X is -CH₂-, L is -NH-, D is 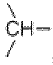 and R₄ is not 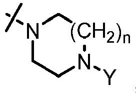 the method includes the following steps:
subjecting the compound f and the compound z to a sixth nucleophilic substitution to obtain a compound k;
subjecting the compound k to a fourth reduction to obtain a compound 5;
subjecting the intermediate compound and the compound 5 to a seventh nucleophilic substitution to obtain a compound 6;
subjecting the compound 6 to a fourth hydrolysis to obtain the memantyl urea derivative having the structure shown in Formula A where R₄ is -OH; and
mixing the memantyl urea derivative having the structure shown in Formula A where R₄ is -OH with the compound 3, and subjecting the acquired mixture to a seventh acylation in the presence of HATU and DIEA to obtain the memantyl urea derivative having the structure shown in Formula A; where
structural formulas of the compound k, the compound 5, and the compound 6 are as follows: 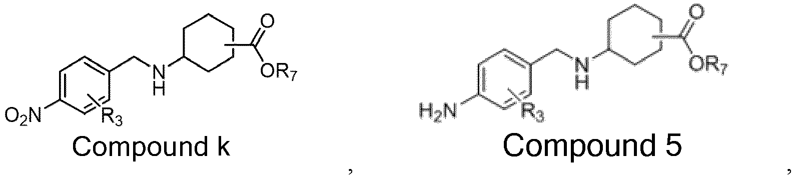 and 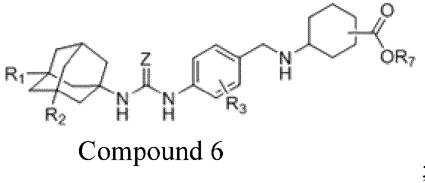 and
in structural formulas of the compound k, the compound 5, and the compound 6, R₇ is C₁-C₆ alkyl.

In the present disclosure, the compound f and the compound z are subjected to a sixth nucleophilic substitution to obtain a compound k. In some embodiments, the reaction conditions and post-treatment used in the sixth nucleophilic substitution are consistent with the reaction conditions and post-treatment of the third nucleophilic substitution in step (1-2), and will not be repeated here.

Based on the compound k, a memantyl urea derivative having a structure shown in Formula A is prepared according to step (1-2).

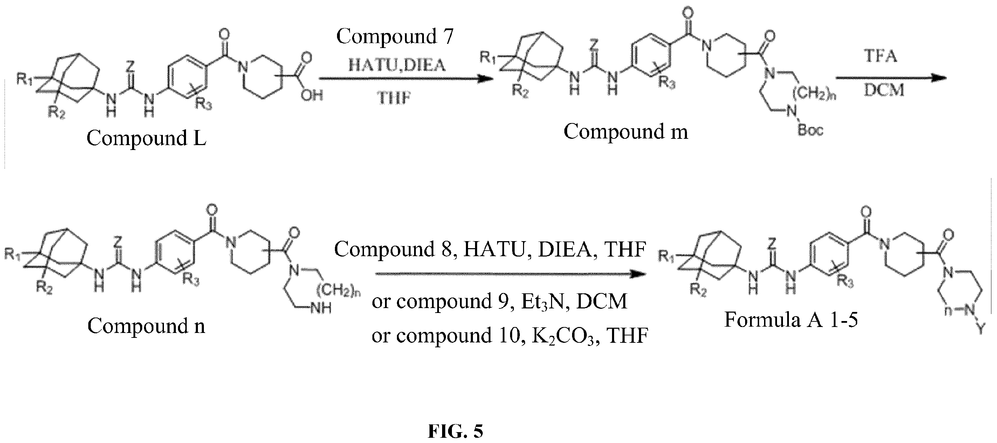

In a fifth case, a memantyl urea derivative having a structure shown in Formula A is prepared. The specific method as shown in FIG. 5 includes the following steps:
(1-5) under the condition that the memantyl urea derivative has the structure shown in Formula A where X is 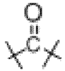 L is absent, D is 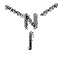 and R₄ is 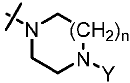 the method includes the following steps:
subjecting a compound 7 and a compound L to an eighth acylation to obtain a compound m;
subjecting the compound m to a first deprotection in an acidic environment to obtain a compound n, the compound n being the memantyl urea derivative having the structure shown in Formula A where Y is -H;
mixing the compound n with a compound 8, and subjecting a resulted mixture to a ninth acylation in the presence of HATU and DIEA to obtain the memantyl urea derivative having the structure shown in Formula A where Y is 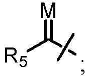
subjecting the compound n and a compound 9 to a tenth acylation to obtain the memantyl urea derivative having the structure shown in Formula A where Y is 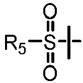 and
subjecting the compound n and a compound 10 to an eighth nucleophilic substitution to obtain the memantyl urea derivative having the structure shown in Formula A where Y is 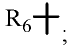 where
the compound 7 has a structural formula shown as 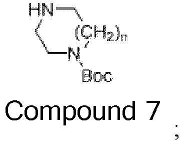
the compound 8 has a structural formula shown as 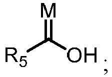
the compound 9 has a structural formula shown as 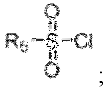
the compound 10 has a structural formula shown as R₆-Cl; and
structural formulas of the compound L, the compound m, and the compound n are as follows:

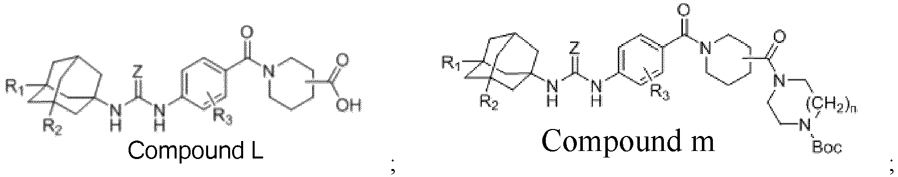

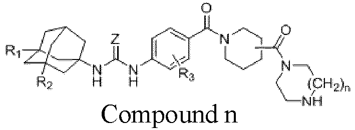

In the present disclosure, a compound 7 and a compound L are subjected to an eighth acylation to obtain a compound m. In some embodiments, the reaction conditions and post-treatment used in the eighth acylation are consistent with the reaction conditions and post-treatment of the first acylation in step (1-1), and will not be repeated here.

In the present disclosure, the compound m is subjected to a first deprotection to obtain a compound n. In some embodiments, a molar ratio of the compound m to the acidic reagent is in a range of 1:(3-10), preferably 1:5. In some embodiments, the acidic environment includes 4-6 mol/L HCl in methanol or ethyl acetate, trifluoroacetic acid (TFA), more preferably the TFA, and an organic solvent used preferably includes acetonitrile, DCM and THF, preferably the DCM. In some embodiments, the first deprotection is conducted at a temperature of -20 °C to 50 °C, more preferably 30 °C; the first deprotection is preferably conducted for 2 h to 8 h, more preferably 3 h. In some embodiments, after the first deprotection is completed, an obtained reaction solution is concentrated under reduced pressure to obtain the compound n.

In the present disclosure, the compound n is the memantyl urea derivative having the structure shown in Formula A where Y is -H; the compound n is mixed with the compound 8 and a resulting mixture is subjected to a ninth acylation in the presence of HATU and DIEA to obtain the memantyl urea derivative having the structure shown in Formula A where Y is 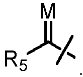 In some embodiments, the reaction conditions and post-treatment used in the ninth acylation are consistent with the reaction conditions and post-treatment of the first acylation in step (1-1), and will not be repeated here. In the examples, the compound may be GL-101, GL-102, or GL-104.

In the present disclosure, the compound n is mixed with a compound 9, and a resulting mixture is subjected to a tenth acylation to obtain the memantyl urea derivative having the structure shown in Formula A where Y is 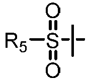 In some embodiments, a molar ratio of the compound n to the compound 9 is in a range of 1:(1.5-2.5), more preferably 1:2. In some embodiments, the tenth acylation is conducted in the presence of TEA and DCM. In some embodiments, the tenth acylation is conducted at room temperature; the tenth acylation is preferably conducted for 1.5 h to 2.5 h, more preferably 2 h. In some embodiments, after the tenth acylation is completed, an obtained reaction solution is poured into water, and a resulting mixture is extracted using the DCM, and a resulting organic layer is washed with 6 mol/L hydrochloric acid, 5 wt% aqueous sodium carbonate solution, water, and saturated aqueous NaCl solution, and dried over anhydrous sodium sulfate, followed by filtering with suction. The resulting filtrate is concentrated under reduced pressure, followed by column chromatography, to obtain the memantyl urea derivative having the structure shown in Formula A.

In the present disclosure, after obtaining the compound n, the compound n is mixed with a compound 10, and a resulting mixture is subjected to an eighth nucleophilic substitution to obtain the memantyl urea derivative having the structure shown in Formula A where Y is 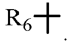 In some embodiments, the reaction conditions and post-treatment used in the eighth nucleophilic substitution are consistent with the reaction conditions and post-treatment of the third nucleophilic substitution in step (1-2), and will not be repeated here.

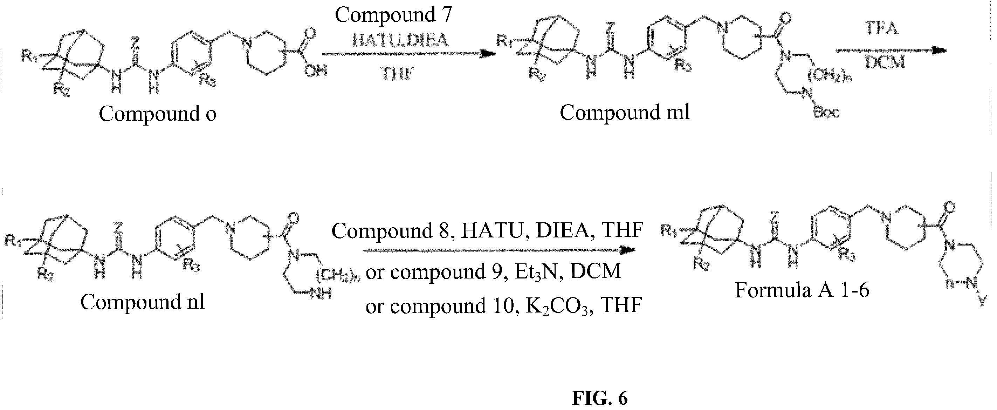

In a sixth case, a memantyl urea derivative having a structure shown in Formula A is prepared. The specific method as shown in FIG. 6 includes the following steps:
(1-6) under the condition that the memantyl urea derivative has the structure shown in Formula A where X is -CH₂-, L is absent, D is 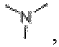 and R₄ is 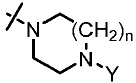 , the method includes the following steps:
subjecting a compound 7 and a compound o to an eleventh acylation to obtain a compound 11;
subjecting the compound 11 to a second deprotection in an acidic environment to obtain a compound 12, the compound 12 being the memantyl urea derivative having the structure shown in Formula A where Y is -H;
mixing the compound 12 with the compound 8 and conducting a twelfth acylation in the presence of HATU and DIEA to obtain the memantyl urea derivative having the structure shown in Formula A where Y is 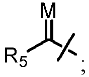
subjecting the compound 12 and the compound 9 to a thirteenth acylation to obtain the memantyl urea derivative having the structure shown in Formula A where Y is 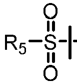 and
subjecting the compound 12 and the compound 10 to a ninth nucleophilic substitution to obtain the memantyl urea derivative having the structure shown in Formula A where Y is 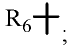 where
the compound o is 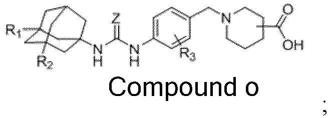
the compound 11 has a structural formula shown as 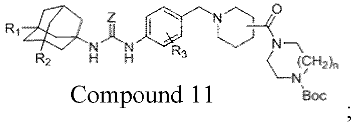 and
the compound 12 has a structural formula shown as 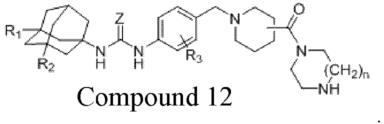
Based on the compound o 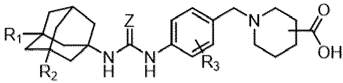 a memantyl urea derivative having a structure shown in Formula A is prepared according to step (1-5). In the examples, the compound may be CL-102, CL-103, CL-201, CL-204, or CL-205.

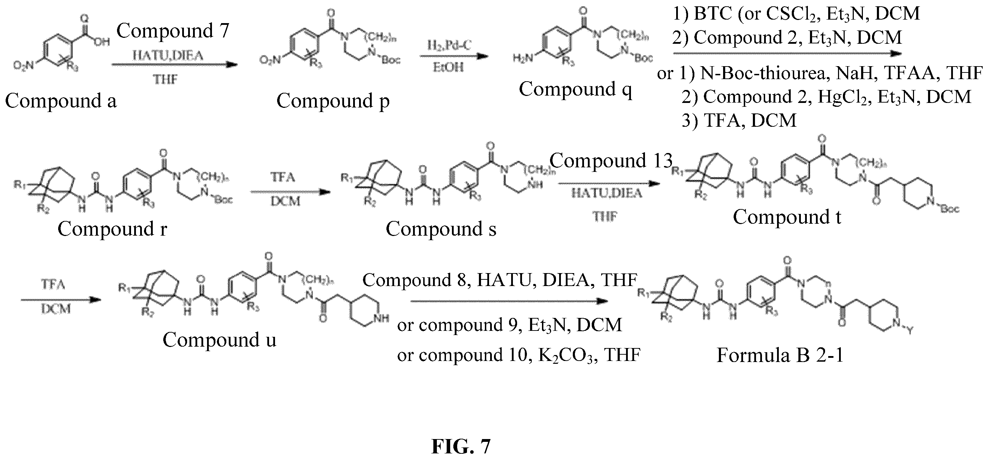

In a seventh case, a memantyl urea derivative having a structure shown in Formula B is prepared. The specific method as shown in FIG. 7 includes the following steps:
(2-1) under the condition that the memantyl urea derivative has the structure shown in Formula B where X is 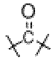 the method includes the following steps:
subjecting the compound 7 and the compound a to a fourteenth acylation to obtain a compound p;
subjecting the compound p to a fifth reduction to obtain a compound q;
subjecting the intermediate compound and the compound q to a tenth nucleophilic substitution to obtain a compound r;
subjecting the compound r to a third deprotection in an acidic environment to obtain a compound s;
subjecting the compound 13 and the compound s to a fifteenth acylation to obtain a compound t;
subjecting the compound t to a fourth deprotection in an acidic environment to obtain a compound u, the compound u being the memantyl urea derivative having the structure shown in Formula B where Y is -H;
mixing the compound u with the compound 8 and conducting a sixteenth acylation in the presence of HATU and DIEA to obtain the memantyl urea derivative having the structure shown in Formula B where Y is 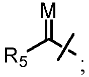
subjecting the compound u and the compound 9 to a seventeenth acylation to obtain the memantyl urea derivative having the structure shown in Formula B where Y is 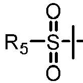 and
subjecting the compound u and the compound 10 to an eleventh nucleophilic substitution to obtain the memantyl urea derivative having the structure shown in Formula B where Y is 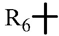 where
the compound 13 has a structural formula shown as 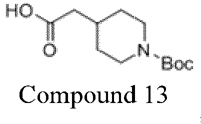 and
structural formulas of the compound p, the compound q, the compound r, the compound s, the compound t, and the compound u are as follows:

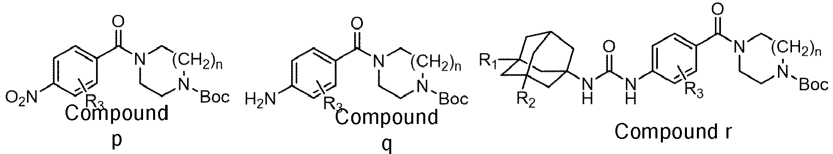

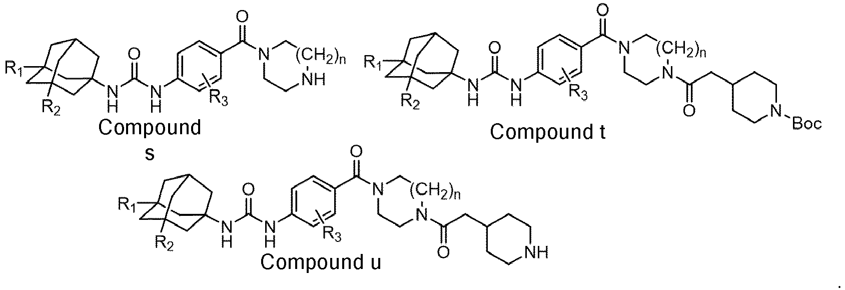

In the present disclosure, the compound 7 and the compound a are subjected to a fourteenth acylation to obtain a compound p. In some embodiments, the reaction conditions and post-treatment used in the fourteenth acylation are consistent with the reaction conditions and post-treatment of the first acylation in step (1-1), and will not be repeated here.

In the present disclosure, after obtaining the compound p, the compound p is subjected to a fifth reduction to obtain a compound q. In some embodiments, the reaction conditions and post-treatment used in the fifth reduction are consistent with the reaction conditions and post-treatment of the first reduction in step (1-1), and will not be repeated here.

In the present disclosure, after obtaining the compound q, the intermediate compound and the compound q are subjected to a tenth nucleophilic substitution to obtain a compound r; the reaction conditions and post-treatment used in the tenth nucleophilic substitution are preferably consistent with the reaction conditions and post-treatment of the first nucleophilic substitution in step (1-1), and will not be repeated here.

In the present disclosure, after obtaining the compound r, the compound r is subjected to a third deprotection in an acidic environment to obtain a compound s. In some embodiments, the reaction conditions and post-treatment used in the third deprotection are consistent with the reaction conditions and post-treatment of the first deprotection in step (1-5), and will not be repeated here.

In the present disclosure, the compound 13 and the compound s are subjected to a fifteenth acylation to obtain a compound t. In some embodiments, the reaction conditions and post-treatment used in the fifteenth acylation are consistent with the reaction conditions and post-treatment of the first acylation in step (1-1), and will not be repeated here.

In the present disclosure, after obtaining the compound t, the compound t is subjected to a fourth deprotection in an acidic environment to obtain a compound u. In some embodiments, the reaction conditions and post-treatment used in the fourth deprotection are consistent with the reaction conditions and post-treatment of the first deprotection in step (1-5), and will not be repeated here.

Based on the compound u, a memantyl urea derivative having a structure shown in Formula B is prepared according to step (1-5). In the examples, the compound may be CC-103, CC-105, CC-106, or CC-124.

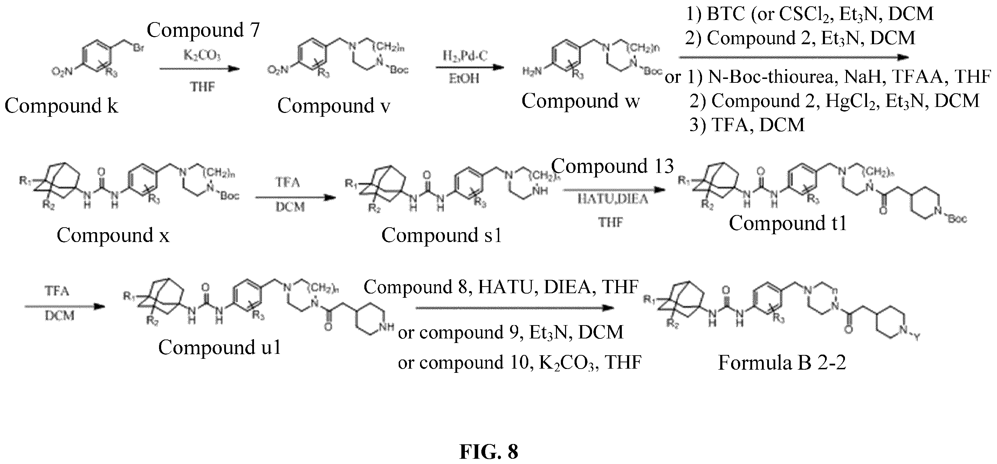

In an eighth case, a memantyl urea derivative having a structure shown in Formula B is prepared. The specific method as shown in FIG. 8 includes the following steps:
(2-2) under the condition that the memantyl urea derivative has the structure shown in Formula B where X is -CH₂-, the method includes the following steps:
subjecting the compound g and the compound 7 to a twelfth nucleophilic substitution to obtain a compound v;
subjecting the compound v to a sixth reduction to obtain a compound w;
subjecting the intermediate compound and the compound w to a thirteenth nucleophilic substitution to obtain a compound x, the compound x being the memantyl urea derivative having the structure shown in Formula B where Y is -H;
mixing the compound x with the compound 8 and conducting an eighteenth acylation in the presence of HATU and DIEA to obtain the memantyl urea derivative having the structure shown in Formula B where Y is 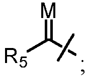
subjecting the compound x and the compound 9 to a nineteenth acylation to obtain the memantyl urea derivative having the structure shown in Formula B where Y is 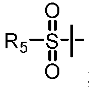 and
subjecting the compound x and the compound 10 to a fourteenth nucleophilic substitution to obtain the memantyl urea derivative having the structure shown in Formula B where Y is R₆+; where
structural formulas of the compound v, the compound w, and the compound x are as follows:

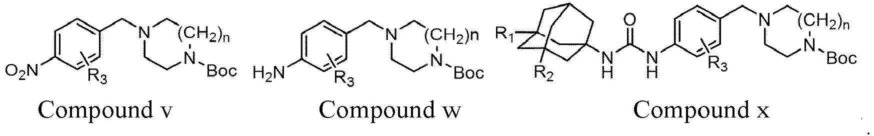

In the present disclosure, the compound g and the compound 7 are subjected to a twelfth nucleophilic substitution to obtain a compound v. In some embodiments, the reaction conditions and post-treatment used in the twelfth nucleophilic substitution are consistent with the reaction conditions and post-treatment of the third nucleophilic substitution in step (1-2), and will not be repeated here.

In the present disclosure, the compound v is subjected to a sixth reduction to obtain a compound w. In some embodiments, the reaction conditions and post-treatment used in the sixth reduction are consistent with the reaction conditions and post-treatment of the first reduction in step (1-1), and will not be repeated here.

In the present disclosure, after obtaining the compound w, the intermediate compound and the compound w are subjected to a thirteenth nucleophilic substitution to obtain a compound x; the reaction conditions and post-treatment used in the thirteenth nucleophilic substitution are consistent with the reaction conditions and post-treatment of the first nucleophilic substitution in step (1-1), and will not be repeated here.

Based on the compound x, a memantyl urea derivative having a structure shown in Formula B is prepared according to step (2-1). In the examples, the compound may be GL-1, GL-2, GL-3, GL-4, GL-21, GL-22, GL-23, or GL-24.

### Example 1 Synthesis of ethyl 1-(3-fluoro-4-nitrobenzoyl)piperidine-4-carboxylate

3-Fluoro-4-nitrobenzoic acid (3.0 g, 16.2 mmol) and dry THF (40 mL) were added into a single-necked flask. After the 3-fluoro-4-nitrobenzoic acid was dissolved, HATU (7.39 g, 19.5 mmol) and DIEA (5.03 g, 38.90 mmol) were added thereto, and the mixture was stirred for 60 min until the solution turned light yellow, and then ethyl 4-piperidine-carboxylate (2.9 g, 16.2 mmol) was added dropwise. After 15 min, the reaction was completed as monitored by TLC, and the reaction was stopped. The reaction system was concentrated under reduced pressure to remove THF, water was then added thereto (20 mL), and the resulting system was extracted with DCM (15 mL×2). The organic layers were combined and washed once with 1 N hydrochloric acid (20 mL), once with saturated sodium carbonate (20 mL), once with water (20 mL), and once with saturated aqueous NaCl solution (20 mL). The organic phase was concentrated under reduced pressure to obtain 5.7 g of a light yellow oil, which was loaded into a column with 5 times silica gel after being mixed with 1.2 times silica gel, and subjected to column chromatography with an eluent (EA: PE=1:1) to obtain a white solid, namely the ethyl 1-(3-fluoro-4-nitrobenzoyl)piperidine-4-carboxylate, with an output of 4.8 g and a yield of 92%.

### Example 2 Synthesis of ethyl 1-(4-amino-3-fluorobenzoyl)piperidine-4-carboxylate

The ethyl 1-(3-fluoro-4-nitrobenzoyl)piperidine-4-carboxylate (3.60 g, 11.6 mmol), 5% Pd-C (0.4 g), and anhydrous ethanol (50 mL) were added into a single-necked flask, and the atmosphere therein was replaced with argon three times, and replaced with hydrogen three times. The resulting system was heated to 60°C and stirred for reaction for 12 h. The reaction was completed as monitored by TLC. The reaction solution was then cooled to room temperature (25°C), and filtered, and a filtrate was concentrated under reduced pressure to obtain a yellow oily substance, namely the ethyl 1-(4-amino-3-fluorobenzoyl)piperidine-4-carboxylate, with an output of 4.0 g and a yield of 90%.

### Example 3 Synthesis of ethyl 1-(4-(3-((1r,3R,5S,7r)-3,5-dimethyladamantan-1-yl)ureido)-3-fluorobenzoyl)piperidine-4-carbox ylate

BTC (1.06 g, 3.8 mmol) and dry DCM (30 mL) were added into a three-necked flask, and a resulting system was cooled to below -78 °C with cold trap. A solution of the ethyl 1-(4-amino-3-fluorobenzoyl)piperidine-4-carboxylate (2.13 g, 7.6 mmol) and TEA (1.06 g, 3.980 mmol) in dry DCM (60 mL) was added dropwise thereto, and a resulting mixture was stirred at room temperature for 0.5 h, and the reaction was stopped. An obtained reaction solution was concentrated to dryness under reduced pressure, and the residue was dissolved with dry DCM (10 mL) to obtain an isocyanate solution for later use.

Memantine (1.36 g, 7.6 mmol), TEA (1.54 g, 15.2 mmol), and dry DCM (25 mL) were added into a three-necked flask, and the isocyanate solution was added dropwise thereto. A reaction was conducted at room temperature for 0.5 h, and the reaction was completed as monitored by TLC. A resulting reaction solution was poured into water (40 mL), and a resulting mixture was extracted with DCM (40 mL×3), followed by washing with 1 mol/L HCl (40 mL×2), washing with water (40 mL×2),and washing with saturated aqueous NaCl solution (40 mL) in sequence, and drying over anhydrous sodium sulfate, and filtering. A resulting filtrate was concentrated under reduced pressure to obtain 3.4 g of a light yellow oil, which was the ethyl 1-(4-(3-((1r,3R,5S,7r)-3,5-dimethyladamantan-1-yl)ureido)-3-fluorobenzoyl)piperidine-4-carbox ylate, with an output of 5.3 g and a yield of 79%.

### Example 4 Synthesis of 1-(4-(3-((1r,3R,5S,7r))-3,5-dimethyladamantan-1-yl)ureido)-3-fluorobenzoyl)piperidine-4-carboxylic acid

The ethyl 1-(4-(3-((1r,3R,5S,7r)-3,5-dimethyladamantan-1-yl)ureido)-3-fluorobenzoyl) piperidine-4-carboxylate (3.4 g, 7.0 mmol) was added into a single-necked flask, dissolve d in THF (20 mL), and sodium hydroxide (0.36 g, 9.00 mmol) and water (10 mL) were added thereto, and a resulting mixture was refluxed for reaction. After 12 h, TLC show ed that the reaction was completed. A resulting reaction solution was concentrated under reduced pressure to remove THF, and water (300 mL) was added into the residue, which was placed in a cold trap and the pH value was adjusted to 3 with 6 N hydrochloric a cid (10 mL). A light yellow solid was precipitated and filtration with suction was condu cted. A filter cake was rinsed with water (20 mL) and dried to obtain 2.96 g of a crude product (dried in an oven at 60 °C for 24 h). The crude product was purified by slurr ying with petroleum ether and diethyl ether to obtain a light yellow solid, namely the (1 R,4r)-4-(4-(3-((1r,3R,5S,7r)-3,5-dimethyladamantan-1-yl)ureido)-3-fluorobenzamido)cyclohexa ne-1-carboxylic acid, with an output of 2.1 g and a yield of 90%.

### Example 5 Synthesis of ethyl (S)-1-(3-fluoro-4-nitrobenzoyl)piperidine-3-carboxylate

3-Fluoro-4-nitrobenzoic acid (3.0 g, 16.2 mmol) and dry THF (40 mL) were added into a single-necked flask. After the 3-fluoro-4-nitrobenzoic acid was dissolved, HATU (7.39 g, 19.5 mmol) and DIEA (5.03 g, 38.90 mmol) were added thereto, and the resulting mixture was stirred for 60 min until the solution turned light yellow, and then ethyl (S)-piperidine-3-carboxylate (2.9 g, 16.2 mmol) was added dropwise thereto. After 15 min, the reaction was completed as monitored by TLC, and then stopped. The reaction system was concentrated under reduced pressure to remove THF, water (20 mL) was added thereto, and the resulting mixture was extracted with DCM (15 mL×2). The organic layers were combined and washed once with 1 N hydrochloric acid (20 mL), once with saturated sodium carbonate (20 mL), once with water (20 mL), and once with saturated aqueous NaCl solution (20 mL). The organic phase was concentrated under reduced pressure to obtain 5.7 g of a light yellow oil, which was loaded in a column with 5 times silica gel, after being mixed with 1.2 times silica gel, and subjected to column chromatography with an eluent (EA: PE=1:1) to obtain a white solid, namely the ethyl (S)-1-(3-fluoro-4-nitrobenzoyl)piperidine-3-carboxylate, with an output of 4.8 g and a yield of 92%.

### Example 6 Synthesis of ethyl (S)-1-(4-amino-3-fluorobenzoyl)piperidine-3-carboxylate

This example was operated according to the method in **Example 2,** except that the ethyl 1-(3-fluoro-4-nitrobenzoyl)piperidine-4-carboxylate was replaced with ethyl (S)-1-(4-amino-3-fluorobenzoyl)piperidine-3-carboxylate. A white solid finally obtained was the ethyl (S)-1-(4-amino-3-fluorobenzoyl)piperidine-3-carboxylate, with an output of 4.1 g and a yield of 90%.

### Example 7 Synthesis of ethyl (S)-1-(4-(3-((1r,3R,5S,7S)-3,5-dimethyladamantan-1-yl) ureido)-3-fluorobenzoyl)piperidine-3-carboxylate

This example was operated according to the method in **Example 3,** except that the ethyl 1-(4-amino-3-fluorobenzoyl)piperidine-4-carboxylate was replaced with ethyl (S)-1-(4-amino-3-fluorobenzoyl)piperidine-3-carboxylate. A white solid finally obtained was the ethyl (S)-1-(4-(3-((1r,3R,5S,7S)-3,5-dimethyladamantan-1-yl)ureido)-3-fluorobenzoyl) piperidine-3-carboxylate, with an output of 5.4 g and a yield of 80%.

### Example 8 Synthesis of (S)-1-(4-(3-((1r,3R,5S,7S)-3,5-dimethyladamantan-1-yl) ureido)-3-fluorobenzoyl)piperidine-3-carboxylic acid

This example was operated according to the method in **Example 4,** except that the ethyl 1-(4-(3 -((1r,3R,5S,7r)-3,5-dimethyladamantan-1-yl)ureido)-3-fluorobenzoyl)piperidine-4-carbox ylate was replaced with ethyl (S)-1-(4-(3-((1r,3R,5S,7S)-3,5-dimethyladamantan-1-yl)ureido)-3-fluorobenzoyl)piperidine-3-ca rboxylate. A white solid finally obtained was the (S)-1-(4-(3-((1r,3R,5S,7S)-3,5-dimethyladamantan-1-yl)ureido)-3-fluorobenzoyl)piperidine-3-ca rboxylic acid, with an output of 4.8 g and a yield of 90%.

### Example 9 Synthesis of (S)-1-(4-(3-((1r,3R,5S,7S)-3,5-dimethyladamantan-1-yl)ureido)-3-fluorobenzoyl)-N-(2-hydroxye thyl)piperidine-3-carboxamide (CC-120)

1-(4-(3-((1r,3R,5S,7r))-3,5-dimethyladamantan-1-yl)ureido)-3-fluorobenzoyl)piperidine-4-carboxylic acid (0.4 g, 0.85 mmol) and dry THF (10 mL) were added into a single-necked flask. After the 1-(4-(3-((1r,3R,5S,7r))-3,5-dimethyladamantan-1-yl)ureido)-3-fluorobenzoyl)piperidine-4-carbo xylic acid was dissolved, HATU (0.5 g, 1.28 mmol) was added thereto and a resulting mixture was stirred for 15 min. DIEA (0.33 g, 2.55 mmol) was added dropwise thereto, followed by stirring for 30 min, and a mixed solution of 2-aminoethanol-1-ol ((0.038 g, 0.85 mmol)) in dry THF (5 mL) was added dropwise thereto. After 15 min, TLC indicated that the reaction was completed and the reaction was stopped. The reaction system was concentrated under reduced pressure to remove THF, water (20 mL) was added thereto, and a resulting mixture was extracted with DCM (15 mL×2). The organic layers were combined and washed once with 1 N hydrochloric acid (20 mL), once with saturated sodium carbonate (20 mL), once with water (20 mL), and once with saturated aqueous NaCl solution (20 mL). The organic phase was concentrated under reduced pressure to obtain 0.6 g of a light yellow oil, which was loaded in a column with 5 times silica gel, after being mixed with 1.2 times silica gel, and subjected to column chromatography with an eluent (EA: PE=1:1) to obtain a white solid, namely the **CC-120,** with an output of 0.21 g and a yield of 23%, m.p. 128-129 °C. ¹H NMR (400 MHz, DMSO-*d*₆): δ(ppm) 8.30 (s, 1H), 8.20 (t, 1H, *J =* 8.4 Hz), 7.91 (s, 1H), 7.20 (dd, 1H, *J=* 1.6 Hz, 11.7 Hz), 7.08 (d, 1H, *J=* 8.4 Hz), 6.58 (s, 1H), 4.66 (s, 1H), 4.34 (s, 1H), 3.62 (s, 1H), 3.36 (s, 2H), 3.09 (s, 2H), 2.89 (s, 2H), 2.36-2.30 (m, 1H), 2.09 (s, 1H), 1.87-1.84 (m, 1H), 1.76 (s, 2H), 1.62-1.55 (m, 5H), 1,35-1.32 (m, 3H), 1.27-1.24 (m, 3H), 1.12 (s, 2H), 0.83 (s, 6H). ¹³C NMR (100 MHz, DMSO-d6): δ173.03, 168.33, 153.81, 152.15, 149.75, 123.84, 119.24, 114.44, 114.24, 60.25, 60.25, 52.14, 50.71, 47.99, 47.99, 42.76, 42.76, 41.81, 32.39, 32.39, 32.39, 30.52, 30.52,30.52, 30.04, 30.04, 28.19.

### Example 10 Synthesis of 1-((1r,3R,5S,7S)-3,5-dimethyladamantan-1-yl)-3-(2-fluoro-4-((S)-3-(4-(2-hydroxyethyl)piperazine-1-carbonyl)piperidine-1-carbonyl)phenyl)urea (CC-12 1)

This example was operated according to the method of **Example 9,** 2-(piperazin-1-yl)ethane-1-ol was used as a raw material (the amine in Example 9 was replaced with 2-(piperazin-1-yl)ethane-1-ol, and the acid was replaced with 1-(4-(3-((1r,3R,5S,7r)-3,5-dimethyladamantan-1-yl)ureido)-3-fluorobenzoyl)piperidine-3-carbox ylic acid) to obtain a white solid, namely **CC-121,** with an output of 0.21 g and a yield of 23%, m.p. 141-145 °C. ¹H NMR (400 MHz, DMSO-*d*₆): δ (ppm) 8.30 (s, 1H), 8.20 (t, 1H, *J =* 8.4 Hz), 7.24 (d, 1H, *J*=11.8 Hz), 7.11 (d, 1H, *J=* 8.5 Hz), 6.58 (s, 1H), 4.43 (s, 1H), 4.32 (s, 1H), 3.49 (s, 6H), 3.05 (s, 1H), 2.79 (s, 2H), 2.38 (s, 5H), 2.09 (s, 1H), 1.82-1.76 (m, 3H), 1.58 (s, 8H), 1.35-1.32 (m, 2H), 1.27-1.24 (m, 3H), 1.18-1.12 (m, 3H), 0.83 (s, 6H). ¹³C NMR (100 MHz, DMSO-*d*₆): δ 168.44, 153.81, 152.16, 149.76, 123.75, 119.34, 114.31, 114.11, 60.44, 58.79, 54.04, 53.29, 52.13, 52.13, 50.71, 47.98, 47.98, 45.19, 42.76, 42.76, 41.39, 38.29, 32.38, 32.38, 32.38, 32.38, 30.52, 30.52, 30.52, 30.04, 30.04, 27.98.

### Example 11 Synthesis of (S)-1-(4-(3-((1r,3R,5S,7S)-3,5-dimethyladamantan-1-yl)urei do)-3-fluorobenzoyl)-N,N-diethylpiperidine-3-carboxamide (CC-110)

This example was operated according to the method of **Example 9,** diethylamine (DEA) was used as a raw material (the amine in Example 9 was replaced with DEA, and the acid was replaced with 1-(4-(3-((1r,3R,5S,7r)-3,5-dimethyladamantan-1-yl)ureido)-3-fluorobenzoyl) piperidine-3-carboxylic acid) to obtain a white solid, namely **CC-110,** with an output of 0.25 g and a yield of 26%, m.p. 125-127 °C. ¹H NMR (400 MHz, CDCl₃): δ (ppm) 8.20 (t, *J =* 8.0 Hz, 1H), 7.57-7.54 (m, 1H), 7.07-6.99 (m, 2H), 5.63 (br, 1H), 4.63 (br, 1H), 3.81 (s, 1H), 3.48-3.35 (m, 4H), 3.18-2.99 (m, 2H), 2.73-2.56 (m, 1H), 2.13 (s, 1H), 1.91 (s, 2H), 1.84 (s, 3H), 1.63 (s, 4H), 1.38-1.35 (m, 2H), 1.29-1.24 (m, 4H), 1.18-1.10 (m, 7H), 0.83 (s, 6H). ¹³C-NMR (100 MHz, CDCl₃): δ (ppm) 172.26, 169.89, 154.13, 136.38, 130.30, 127.80, 123.48, 120.02, 113.97, 52.72, 50.62, 48.01, 42.72, 42.02, 40.61, 40.38, 32.37, 30.13, 14.95, 13.06.

### Example 12 Synthesis of (3S)-1-(4-(3-((1r,3R,5S,7S)-3,5-dimethyladamantan-1-yl)ur eido)-3-fluorobenzoyl)-N-(2-hydroxypropyl)piperidine-3-carboxamide (CC-113)

This example was operated according to the method of **Example 9,** 1-aminopropan-2-ol was used as a raw material (the amine in Example 9 was replaced with 1-aminopropan-2-ol, and the acid was replaced with 1-(4-(3-((1r,3R,5S,7r)-3,5-dimethyladamantan-1-yl)ureido)-3-fluorobenzoyl)piperidine-3-carboxylic acid) to obtain a white solid, namely **CC-113,** with an output of 0.27 g and a yield of 29%, m.p. 131-132 °C. ¹H NMR (400 MHz, CDCl₃): δ (ppm) 8.20 (s, 1H), 7.61 (s, 1H), 7.05-7.03 (m, 2H), 5.85 (br, 1H), 4.05 (br, 1H), 3.86 (br, 1H), 3.55 (br, 4H), 3.38-3.35 (m, 1H), 3.29-3.20 (m, 1H), 3.2-2.95 (m, 1H), 2.81 (br, 1H), 2.48 (br, 1H), 2.13 (br, 1H), 2.06-2.02 (m, 1H), 1.88-1.84 (m, 1H), 1.81 (s, 2H), 1.62 (s, 5H), 1.44-1.43 (m, 1H), 137-1.34 (m, 2H), 1.30-1.24 (m, 3H), 1.44-1.11 (m, 5H), 0.84 (m, 6H). ¹³C NMR (100 MHz, CDCl₃): δ (ppm) 173.03, 169.72, 154.14, 130.26, 130.17, 127.87, 123.27, 120.48, 113.85, 52.82, 50.04, 47.98, 42.72, 41.98, 40.56, 40.47, 38.49, 32.39, 30.17, 30.13, 28.82, 15.06, 13.10.

### Example 13 Synthesis of 1-((1r,3R,5S,7r)-3,5-dimethyladamantan-1-yl)-3-(2-fluoro-4-(4-(4-(2-hydroxyethyl)piperazine-1-carbonyl)piperidine-1-carbonyl)phenyl)urea (CC-112)

This example was operated according to the method of **Example 9,** 2-(piperazin-1-yl)ethane-1-ol was used as a raw material (the amine in Example 9 was replaced with 2-(piperazine-1-yl)ethane-1-ol, and the acid was replaced with (1R,4r)-4-(4-(3-((1r,3R,5S,7r)-3,5-dimethyladamantan-1-yl)ureido)-3-fluorobenzamido)cyclohex ane-1-carboxylic acid) to obtain a white solid, namely **CC-112,** with an output of 0.21 g and a yield of 25%, m.p. 128-129 °C. ¹H NMR (400 MHz, DMSO-*d*₆): δ (ppm) 8.29 (s, 1H), 8.19 (t, 1H, *J =* 8.24 Hz), 7.20 (d, 1H, *J*=11.72 Hz), 7.08 (d, 1H, *J=* 8.32 Hz), 6.58 (s, 1H), 4.44 (s, 1H), 3.50-3.49 (m, 4H), 3.43 (s, 2H), 2.92 (s, 2H), 2.40-2.37 (m, 4H), 2.33 (s, 2H), 2.09 (s, 1H), 1.76 (s, 2H), 1.58 (s, 6H), 1.49-1.46 (m, 3H), 1.35-1.32 (m, 2H), 1.27-1.24 (m, 3H), 1.12 (s, 2H), 1.05 (s, 2H), 0.83 (s, 6H). ¹³C NMR (100 MHz, DMSO-*d*₆): δ 172.43, 168.26, 153.82, 152.19, 149.78, 123.82, 119.28, 114.42, 114.21, 60.54, 58.93, 58.93, 54.20, 53.43, 52.13, 50.71, 47.99, 47.99, 46.15, 45.28, 42.76, 42.76, 41.60, 37.41, 32.39, 32.39, 32.39, 30.52, 30.52, 30.52, 30.03, 28.88.

### Example 14 Synthesis of 1-(4-(3-((1r,3R,5S,7r)-3,5-dimethyladamantan-1-yl)ureido)-3-fluorobenzoyl)-N-(2-hydroxyethyl )piperidine-4-carboxamide (CC-114)

This example was operated according to the method of **Example 9,** 2-aminoethanol-1-ol was used as a raw material (the amine in Example 9 was replaced with 2-aminoethanol-1-ol, and the acid was replaced with (1R,4r)-4-(4-(3-((1r,3R,5S,7r)-3,5-dimethyladamantan-1-yl)ureido)-3-fluorobenzamido)cyclohex ane-1-carboxylic acid) to obtain a white solid, namely **CC-114,** with an output of 0.24 g and a yield of 27%, m.p. 125-126 °C. ¹H NMR (400 MHz, DMSO-*d*₆): δ (ppm) 8.30 (s, 1H), 8.20 (t, 1H, *J =* 8.3 Hz), 7.82 (t, 1H, *J=* 5.2 Hz), 7.20 (d, 1H, *J=* 11.6 Hz), 7.09 (d, 1H, *J=* 8.4 Hz), 6.58 (s, 1H), 4.65 (t, 1H, *J=* 5.5 Hz), 4.33 (s, 1H), 3.72 (s, 1H), 3.40-3.37 (m, 1H), 3.10 (q, 2H, J= 5.9 Hz), 2.91 (s, 2H), 2.42-2.37 (m, 1H), 2.09 (s, 1H), 1.76 (s, 2H), 1.68 (s, 2H), 1.58 (s, 4H), 1.50-1.48 (m, 2H), 1.35-1.32 (m, 2H), 1.27-1.24 (m, 2H), 1.12 (s, 2H), 0.83 (s, 6H). ¹³C NMR (100 MHz, DMSO-*d*₆): δ 174.32, 168.27, 153.81, 149.77, 123.80, 119.30, 114.38, 114.17, 60.33, 60.33, 52.13, 52.13, 50.71, 47.99, 47.99, 42.76, 42.76, 42.19, 41.85, 32.38, 32.38, 32.38, 32.38, 30.52,30.52, 30.52, 30.03, 30.03, 29.04.

### Example 15 Synthesis of 1-(4-(3-((1r,3R,5S,7r)-3,5-dimethyladamantan-1-yl)ureido)-3-fluorobenzoyl)-N-(2-hydroxyprop yl)piperidine-4-carboxamide (CC-115)

This example was operated according to the method of **Example 9,** 1-aminopropan-2-ol was used as a raw material (the amine in Example 9 was replaced with 1-aminopropan-2-ol, and the acid was replaced with (1R,4r)-4-(4-(3-((1r,3R,5S,7r)-3,5-dimethyladamantan-1-yl)ureido)-3-fluorobenzamido)cyclohex ane-1-carboxylic acid) to obtain a white solid, namely **CC-115,** with an output of 0.23 g and a yield of 26%, m.p. 115-116 °C. ¹H NMR (400 MHz, DMSO-*d*₆): δ (ppm) 8.30 (s, 1H), 8.19 (t, 1H, *J* = 8.4 Hz), 7.63 (d, 1H, *J*=7.7 Hz), 7.20 (q, 1H, *J*=1.7, 11.7 Hz), 7.09 (d, 1H, *J*= 8.4 Hz), 6.58 (s, 1H), 4.52 (s, 1H), 3.43 (s, 1H), 2.89 (s, 2H), 2.36-2.30 (m, 1H), 2.09 (s, 1H), 1.78-1.76 (m, 2H), 1.72-1.71 (m, 2H), 1.58 (s, 4H), 1.49-1.46 (m, 2H), 1.35-1.32 (m, 2H), 1.27-1.32 (m, 4H), 1.17-1.15 (m, 4H), 1.21 (s, 3H), 0.83 (s, 6H). ¹³C NMR (100 MHz, DMSO-*d*₆): δ 173.42, 168.28, 153.81, 152.18, 149.77, 123.81, 119.30, 114.38, 114.17, 68.63, 52.13, 50.71, 47.99, 47.99, 47.42, 42.76, 42.76, 42.24, 42.24, 34.40, 34.40, 32.38,32.38, 32.38, 30.73, 30.52, 30.52, 30.52, 30.04, 29.00.

### Example 16 Synthesis of N-(1,3-dihydroxypropan-2-yl)-1-(4-(3-((1r,3R,5S,7r)-3,5-dimethyladamantan-1-yl)ureido)-3-flu orobenzoyl)piperidine-4-carboxamide (CC-116)

This example was operated according to the method of **Example 9,** 2,2'-azanediylbis(ethan-1-ol) was used as a raw material (the amine in Example 9 was replaced with 2,2'-azadiylbis(ethan-1-ol), and the acid was replaced with (1R,4r)-4-(4-(3-((1r,3R,5S,7r)-3,5-dimethyladamantan-1-yl)ureido)-3-fluorobenzamido)cyclohex ane-1-carboxylic acid) to obtain a white solid, namely **CC-116,** with an output of 0.23 g and a yield of 26%, m.p. 113-114 °C. ¹H NMR (400 MHz, DMSO-*d*₆): δ (ppm) 8.36 (s, 1H), 8.20 (t, 1H, *J* = 8.3 Hz), 7.63 (d, 1H, J=8.0 Hz), 7.21 (d, 1H, *J=* 11.7 Hz), 7.09 (d, 1H, *J*= 8.68 Hz), 6.67 (s, 1H), 4.62 (t, 2H, *J*=5.4 Hz), 3.71-3.66 (m, 1H), 3.40-3.39 (m, 2H), 3.08-3.06 (m, 4H), 2.10 (s, 1H), 1.76 (s, 1H), 1.68 (s, 1H), 1.59 (s, 3H), 1.50-1.48 (m, 2H), 1.41-1.35 (m, 1H), 1.32 (s, 1H), 1.28-1.24 (m, 3H), 1.21-1.18 (m, 5H), 1.12 (s, 2H), 0.83 (s, 6H). ¹³C NMR (100 MHz, DMSO-*d*₆): δ 174.24, 168.29, 153.86, 152.19, 149.79, 123.80, 119.35, 114.38, 114.17, 60.59, 60.59, 53.16, 52.13, 50.72, 47.99, 47.99, 45.87, 45.87, 42.77, 42.19, 32.38, 32.38, 32.38, 30.53, 30.53, 30.53, 30.03, 29.03, 8.94.

### Example 17 Synthesis of 1-(4-(3-((1r,3R,5S,7r)-3,5-dimethyladamantan-1-yl)ureido)-3-fluorobenzoyl)-N,N-diethylpiperid ine-4-carboxamide (CC-111)

This example was operated according to the method of **Example 9,** diethylamine (DEA) was used as a raw material (the amine in Example 9 was replaced with DEA, and the acid was replaced with (1R,4r)-4-(4-(3-((1r,3R,5S,7r)-3,5-dimethyladamantan-1-yl)ureido)-3-fluorobenzamido)cyclohexane-1-carboxylic acid) to obtain a white solid, namely **CC-111,** with an output of 0.33 g and a yield of 36%, m.p. 102-104 °C. ¹H NMR (400 MHz, CDCl₃) δ (ppm) 8.15 (t, *J =* 8.5 Hz, 1H), 7.33 (br, 1H), 7.07-7.05 (m, 2H), 5.65 (br, 1H), 4.61 (br, 1H), 3.93 (br, 1H), 3.41-3.33 (m, 4H), 3.03-2.95 (m, 2H), 2.75-2.69 (m, 1H), 2.14-2.13 (m, 1H), 1.82 (s, 2H), 1.72 (br, 3H), 1.64 (s, 5H), 1.39-1.36 (m, 2H), 1.29-1.23 (m, 6H), 1.16-1.09 (m, 5H), 0.82 (s, 6H). ¹³C NMR (100 MHz, CDCl₃): δ (ppm) 173.03, 169.72, 154.14, 130.26, 130.17, 127.87, 123.27, 120.48, 113.85, 52.82, 50.04, 47.98, 42.72, 41.98, 40.56, 40.47, 38.49, 32.39, 30.17, 30.13, 28.82, 15.06, 13.10.

### Example 18 Synthesis of 3-fluoro-4-nitrobenzyl alcohol

3-Fluoro-4-nitrobenzoic acid (20 g, 0.11 mol) and THF (200 mL) were added into a three-necked flask, and a resulting mixture was cooled to 0 °C in a cold trap. A THF solution of borane (160 mL) was added dropwise thereto, and a reaction was conducted at 0 °C for 10 h. The reaction was completed as monitored by TLC. An obtained reaction solution was concentrated under reduced pressure to remove the THF to obtain a white solid, namely the 3-fluoro-4-nitrobenzyl alcohol, with an output of 17.8 g and a yield of 95%.

### Example 19 Synthesis of 4-(bromomethyl)-2-fluoro-1-nitrobenzene

3-Fluoro-4-nitrobenzyl alcohol (17.0 g, 99.4 mmol), phosphorus tribromide (18.5 g, 50 mmol), and DIEA (20 mL) were added into a single-necked flask, and a resulting mixture was stirred at 30 °C for reaction for 10 h. The reaction was completed as monitored by TLC. Water (50 mL) was added thereto, and a resulting system was extracted with ethyl acetate (100 mL), followed by drying, filtering with suction, and concentrating to obtain a yellow solid, namely the 4-(bromomethyl)-2-fluoro-1-nitrobenzene, with an output of 17.4 g and a yield of 92%.

### Example 20 Synthesis of ethyl (S)-1-(3-fluoro-4-nitrobenzyl)piperidine-3-carboxylate

4-(Bromomethyl)-2-fluoro-1-nitrobenzene (2.8 g, 12.9 mmol) and dry acetonitrile (35 mL) were added into a single-necked flask. After the 4-(bromomethyl)-2-fluoro-1-nitrobenzene was dissolved, potassium carbonate (2.13 g, 15.4 mmol), potassium iodide (0.15 g, 1.29 mmol), and ethyl (S)-piperidine-3-carboxylate (2.03 g, 12.9 mmol) were added thereto and the mixture was refluxed for reaction. After 6 h, the reaction was completed as monitored by TLC. The reaction mixture was concentrated under reduced pressure to remove acetonitrile, water (20 mL) was added thereto, and the resulting mixture was extracted with ethyl acetate (30 mL×2). The organic layers were combined, washed with water (20 mL), and washed once with saturated aqueous NaCl solution (25 mL). The organic phase was concentrated under reduced pressure to obtain 5.2 g of a yellow oily substance, which was loaded into a column with 4 times silica gel column after being mixed with 1.2 times silica gel, and subjected to column chromatography with an eluent (EA: PE=1:40) to obtain a yellow oily substance, namely the ethyl (S)-1-(3-fluoro-4-nitrobenzyl)piperidine-3-carboxylate, with an output of 4.4 g and a yield of 82%.

### Example 21 Synthesis of ethyl (S)-1-(4-amino-3-fluorobenzyl)piperidine-3-carboxylate

The ethyl (S)-1-(3-fluoro-4-nitrobenzyl)piperidine-3-carboxylate (3.48 g, 11.6 mmol), 5% Pd-C (0.4 g), and anhydrous ethanol (50 mL) were added into a single-necked flask, the atmosphere therein was replaced with argon three times, and replaced with hydrogen three times, and a resulting mixture was heated to 60°C and stirred for reaction for 12 h. The reaction was completed as monitored by TLC; the resulting reaction solution was cooled to room temperature (25 °C), and filtered. A resulting filtrate was concentrated under reduced pressure to obtain a yellow oily substance, namely the ethyl (S)-1-(4-amino-3-fluorobenzyl)piperidine-3-carboxylate, with an output of 2.76 g and a yield of 86%.

### Example 22 Synthesis of ethyl (S)-1-(4-(3-((1r,3R,5S,7S)-3,5-dimethyladamantan-1-yl)ureido)-3-fluorobenzyl)piperidine-3-car boxylate

BTC (1.06 g, 3.8 mmol) and dry DCM (30 mL) were added into a three-necked flask, and a resulting mixture was cooled to below -78 °C with cold trap. A solution of the ethyl (S)-1-(4-amino-3-fluorobenzyl)piperidine-3-carboxylate (2.10 g, 7.6 mmol) and TEA (1.06 g, 3.980 mmol) in dry DCM (60 mL) was added dropwise thereto, and a resulting mixture after the drop addition was transferred to room temperature and stirred at room temperature for 0.5 h, and the reaction was stopped. An obtained reaction solution was concentrated to dryness under reduced pressure, and the residue was dissolved with dry DCM (10 mL) to obtain an isocyanate solution for later use.

Memantine (1.36 g, 7.6 mmol), TEA (1.54 g, 15.2 mmol), and dry DCM (25 mL) were added into a three-necked flask, and the isocyanate solution was added dropwise thereto. A reaction was conducted at room temperature for 0.5 h, and the reaction was completed as monitored by TLC. A reaction solution was poured into water (40 mL), and extracted with DCM (40 mL×3), followed by washing with 1 mol/L HCl (40 mL×2),washing with water (40 mL×2), and washing with saturated aqueous NaCl solution (40 mL) in sequence, and drying over anhydrous sodium sulfate, and filtering with suction. A filtrate was concentrated under reduced pressure to obtain 3.3 g of a light yellow oily substance, namely, the ethyl (S)-1-(4-(3-((1r,3R,5S,7S)-3,5-dimethyladamantan-1-yl)ureido)-3-fluorobenzyl)piperidine-3-car boxylate, which was then used for next step without purification.

### Example 23 Synthesis of (S)-1-(4-(3-((1r,3R,5S,7S)-3,5-dimethyladamantan-1-yl)ureido)-3-fluorobenzyl)piperidine-3-car boxylic acid

Ethyl (S)-1-(4-(3-((1r,3R,5S,7S)-3,5-dimethyladamantan-1-yl)ureido)-3-fluorobenzyl) piperidine-3-carboxylate (3.3 g, 7.0 mmol) was added into a single-necked flask, and dissolved in THF (20 mL), and sodium hydroxide (0.36 g, 9.00 mmol) and water (10 mL) were added thereto and a resulting mixture was refluxed for reaction. After 12 h, TLC showed that the reaction was completed; a reaction solution was concentrated under reduced pressure to remove THF, and water (300 mL) was added into the residue, and a resulting mixture was placed in a cold trap and the pH value thereof was adjusted to 3 with 6 N hydrochloric acid (10 mL). A light yellow solid was precipitated, followed by filtering with suction. A filter cake was rinsed with water (20 mL), followed by drying (dried in an oven at 60 °C for 24 h) to obtain 2.96 g of a crude product. The crude product was purified by slurrying with petroleum ether and diethyl ether to obtain a light yellow solid, namely the (S)-1-(4-(3-((1r,3R,5S,7S)-3,5-dimethyladamantan-1-yl)ureido)-3-fluorobenzyl)piperidine-3-car boxylic acid, with an output of 2.7 g and a yield of 86%.

### Example 24 Synthesis of ethyl 1-(3-fluoro-4-nitrobenzyl)piperidine-4-formate

This example was operated according to the method in **Example 20,** except that the ethyl (S)-piperidine-3-carboxylate was replaced with ethyl 4-piperidinecarboxylate, and a white solid finally obtained was the ethyl 1-(3-fluoro-4-nitrobenzyl)piperidine-4-formate, with an output of 4.7 g and a yield of 84%.

### Example 25 Synthesis of ethyl 1-(4-amino-3-fluorobenzyl)piperidine-4-formate

This example was operated according to the method in **Example 21,** except that the ethyl (S)-1-(3-fluoro-4-nitrobenzyl)piperidine-3-carboxylate was replaced with ethyl 1-(3-fluoro-4-nitrobenzyl)piperidine-4-formate. A white solid finally obtained was the ethyl 1-(4-amino-3-fluorobenzyl)piperidine-4-formate, with an output of 2.7 g and a yield of 81%.

### Example 26 Synthesis of ethyl 1-(4-(3-((1r,3R,5S,7r)-3,5-dimethyladamantan-1-yl)ureido)-3-fluorobenzyl)piperidine-4-carboxy late

This example was operated according to the method in **Example 22,** except that the ethyl (S)-1-(4-amino-3-fluorobenzyl)piperidine-3-carboxylate was replaced with ethyl 1-(4-amino-3-fluorobenzyl)piperidine-4-formate. A white solid finally obtained was the ethyl 1-(4-(3-((1r,3R,5S,7r)-3,5-dimethyladamantan-1-yl)ureido)-3-fluorobenzyl)piperidine-4-carboxy late, which was a crude product with purification.

### Example 27 Synthesis of 1-(4-(3-((1r,3R,5S,7r)-3,5-dimethyladamantan-1-yl) ureido)-3-fluorobenzyl)piperidine-4-carboxylic acid (CL-5)

This example was operated according to the method in **Example 23,** except that the ethyl (S)-1-(4-(3-((1r,3R,5S,7r)-3,5-dimethyladamantan-1-yl)ureido)-3-fluorobenzyl) piperidine-3-carboxylate was replaced with ethyl 1-(4-(3-((1r,3R,5S,7r)-3,5-dimethyladamantan-1-yl)ureido)-3-fluorobenzyl)piperidine-4-carboxy late. A white solid finally obtained was the 1-(4-(3-((1r,3R,5S,7r)-3,5-dimethyladamantan-1-yl)ureido)-3-fluorobenzyl)piperidine-4-carboxy lic acid, with an output of 2.4 g and a yield of 75%.

### Example 28 Synthesis of (S)-1-(4-(3-((1r,3R,5S,7S)-3,5-dimethyladamantan-1-yl)ureido)-3-fluorobenzyl)-N,N-dimethylpi peridine-3-carboxamide (CL-105)

(S)-1-(4-(3-((1r,3R,5S,7S)-3,5-dimethyladamantan-1-yl)ureido)-3-fluorobenzyl)piperidin e-3-carboxylic acid (0.4 g, 0.85 mmol) and dry THF (10 mL) were added into a single-necked flask. After the (S)-1-(4-(3-((1r,3R,5S,7S)-3,5-dimethyladamantan-1-yl)ureido)-3-fluorobenzyl) piperidine-3-carboxylic acid was dissolved, HATU (0.5 g, 1.28 mmol) was added thereto and stirred for 15 min. DIEA (0.33 g, 2.55 mmol) was added dropwise thereto, followed stirring for 30 min, and a mixed solution of dimethylamine (DMA) ((0.038 g, 0.85 mmol)) in dry THF (5 mL) was added dropwise. After 15 min, TLC indicated that the reaction was completed and the reaction was stopped. The reaction system was concentrated under reduced pressure to remove THF, water (20 mL) was added thereto, and a resulting mixture was extracted with DCM (15 mL×2). The organic layers were combined and washed once with 1 N hydrochloric acid (20 mL), once with saturated sodium carbonate (20 mL), once with water (20 mL), and once with saturated aqueous NaCl solution (20 mL). The organic phase was concentrated under reduced pressure to obtain 0.6 g of a light yellow oily substance, which was loaded into a column with 5 times silica gel, after being mixed with 1.2 times silica gel, and subjected to column chromatography with an eluent (EA: PE=1:1) to obtain a white solid, namely the **CL-105,** with an output of 0.22 g and a yield of 51%, m.p. 111-112°C. ¹H NMR(400MHz,CDCl₃):δ (ppm) 7.93 (t, *J*=8.92Hz, 1H),7.13-7.12 (m, 1H), 6.91-6.85 (m, 2H), 5.53 (s, 1H), 3.39-3.27 (m, 2H), 2.94 (s, 3H), 2.83 (s, 3H), 2.79-2.72 (m, 3H), 2.07-2.02 (m, 2H), 1.89-1.85 (m, 1H), 1.76-1.70 (m, 3H), 1.62-1.54 (m, 6H), 1.42-1.39 (m, 1H), 1.30-1.27 (m, 2H), 1.21-1.18 (m, 2H), 1.10-1.02 (m, 2H), 0.75 (s, 6H).¹³C-NMR (100 MHz, DMSO-*d*₆): δ (ppm) 174.5, 154.5, 153.4, 151.1, 124.9, 120.9, 115.2, 115.1, 65.6, 62.5, 55.6, 53.6, 52.7, 50.7, 48.1, 42.8, 40.7, 39.6, 37.2, 35.6, 32.4, 30.5, 30.2, 30.2, 29.7, 27.3, 24.9, 13.7.

### Example 29 Synthesis of (S)-1-(4-(3-((1r,3R,5S,7S)-3,5-dimethyladamantan-1-yl)ureido)-3-fluorobenzyl)-N,N-diethylpip eridine-3-carboxamide (CL-106)

This example was operated according to the method of **Example 9,** DEA was used as a raw material (the amine in Example 9 was replaced with DEA) to obtain a white solid, namely **CL-106,** with an output of 0.27 g and a yield of 57%, m.p. 159-160 °C. ¹H NMR(400MHz,DMSO-*d₆*):δ (ppm) 8.27-8.22 (m, 2H), 7.42-7.14 (m, 2H), 6.55 (s, 1H), 4.31-4.24 (m, 1H), 3.27-3.17 (m, 6H), 2.91 (s, 3H), 2.09 (s, 1H), 1.75 (s, 5H), 1.57 (s, 4H), 1.41 (s, 2H), 1.40-1.25 (m, 5H), 1.12-1.09 (m, 5H), 1.00-0.98 (m, 3H), 0.83 (s, 6H).¹³C-NMR (100 MHz, DMSO-*d*₆): δ (ppm) 172.5, 153.8, 152.0, 150.4, 129.9, 127.5, 119.8, 117.3, 59.3, 54.0, 52.2, 52.1, 52.1, 52.0, 50.7, 48.0, 42.7, 42.3, 41.8, 32.5, 32.3, 30.5, 30.0, 28.9, 26.8, 23.0, 22.9.

### Example 30 Synthesis of (S)-1-(4-(3-((1r,3R,5S,7S)-3,5-dimethyladamantan-1-yl)ureido)-3-fluorobenzyl)-N,N-bis(2-hydr oxyethyl)piperidine-3-carboxamide (CL-107)

This example was operated according to the method of **Example 9,** DEA was used as a raw material (the amine in Example 9 was replaced with DEA) to obtain a white solid, namely **CL-107,** with an output of 0.30 g and a yield of 64%, m.p. 123-124 °C. ¹H NMR(400MHz,CDCl₃):δ (ppm) 8.08 (t, *J*=8.36Hz, 1H),6.95-6.90 (m, 2H), 5.67 (s, 1H), 3.73-3.62 (m, 2H), 3.45-3.43 (m, 1H), 3.14 (q, *J*=8.00Hz, 2H), 2.86 (s, 2H), 2.45 (s, 1H), 2.14 (s, 2H), 1.98-1.97 (m, 3H), 1.89-1.83 (m, 3H), 1.65 (s, 4H), 1.57 (s, 2H), 1.42-1.33 (m, 5H), 1.30-1.24 (m, 5H), 1.15-1.14 (m, 2H), 0.75 (s, 6H).¹³C-NMR (100 MHz, DMSO-*d*₆): δ (ppm) 174.8, 154.5, 154.4, 153.5, 151.1, 125.3, 120.9, 115.1, 125.3, 120.9, 115.1, 69.7, 62.5, 60.4, 54.3, 54.1, 52.8, 50.6, 48.1, 47.3, 42.7, 40.7, 33.8, 32.4, 30.9, 30.2, 26.7, 22.2, 21.1, 14.2, 8.9.

### Example 31 Synthesis of (S)-N-(1,3-dihydroxypropan-2-yl)-1-(4-(3-((1r,3R,5S,7S)-3,5-dimethyladamantan-1-yl)ureido)-3 -fluorobenzyl)piperidine-3-carboxamide (CL-112)

This example was operated according to the method of **Example 9,** 2-amino-1,3-propanediol was used as a raw material (the amine in Example 9 was replaced with 2-amino-1,3-propanediol) to obtain a white solid, namely **CC-112,** with an output of 0.12 g and a yield of 28%, m.p. 113-114 °C. ¹H NMR(400MHz,CDCl₃):δ (ppm) 7.87-7.86 (m, 1H), 7.45 (s, 1H), 7.04-7.01 (m, 1H), 6.85-6.83 (m, 1H), 5.88 (s, 1H), 3.87 (s, 1H), 3.67-3.30 (m, 7H), 3.01-2.70 (m, 2H), 2.43 (s, 1H), 2.03 (s, 1H), 1.73 (s, 3H), 1.55(s, 7H), 1.28-1.77 (m, 7H), 1.05 (s, 3H), 0.74 (s, 6H).¹³C-NMR (100 MHz, DMSO-*d*₆): δ (ppm) 166.5, 150.0, 148.9, 146.5, 122.8, 120.7, 116.0, 110.9, 57.2, 57.0, 55.7, 49.4, 47.9, 47.6, 45.9, 43.4, 41.9, 37.9, 35.8, 27.6, 27.2, 25.4, 24.9, 24.6, 22.1, 17.9, 17.6, 16.3, 9.4, 9.3.

### Example 31 Synthesis of (S)-1-(4-(3-((1r,3R,5S,7S)-3,5-dimethyladamantan-1-yl)ureido)-3-fluorobenzyl)-N-(2-hydroxypr opyl)piperidine-3-carboxamide (CL-113)

This example was operated according to the method of **Example 9,** 1-amino-2-propanol was used as a raw material (the amine in Example 9 was replaced with 1-amino-2-propanol) to obtain a white solid, namely **CL-113,** with an output of 0.18 g and a yield of 32%, m.p. 91-92 °C. ¹H NMR(400MHz,CDCl₃):δ (ppm) 7.98 (t, *J*=7.36Hz, 1H), 7.01-6.94 (m, 3H), 5.29 (s, 1H), 3.91-3.85 (m, 1H), 3.50-3.26 (m, 4H), 3.10-3.07 (m, 3H), 2.81 (s, 2H), 2.51 (s, 1H), 2.30 (s, 2H), 2.14 (s, 1H), 1.84 (s, 3H), 1.15-1.58 (m, 7H), 1.27-1.22 (m, 4H), 1.16-1.14 (m, 4H), 0.84 (s, 6H).¹³C-NMR (100 MHz, DMSO-*d*₆): δ (ppm) 176.5, 154.3, 153.6, 151.2, 125.3, 121.3, 115.6, 115.4, 67.4, 67.3, 62.3, 54.2, 53.9, 52.9, 50.6, 48.1, 47.3, 47.0, 46.9, 42.7, 40.7, 32.4, 30.2, 30.1, 26.7, 22.5, 20.9, 8.8, 8.6.

### Example 32 Synthesis of (S)-1-(4-(3-((1r,3R,5S,7S)-3,5-dimethyladamantan-1-yl)ureido)-3-fluorobenzyl)-N-(2-hydroxyet hyl)piperidine-3-carboxamide (CL-114)

This example was operated according to the method of **Example 9,** ethanolamine was used as a raw material (the amine in Example 9 was replaced with ethanolamine) to obtain a white solid, namely **CL-114,** with an output of 0.19 g and a yield of 35%, m.p. 94-95 °C. ¹H NMR(400MHz,CDCl₃):δ (ppm) 8.00 (t, *J*=8.33Hz, 1H), 7.08 (s, 1H), 7.00-6.95 (m, 2H), 5.46 (s, 1H), 3.64-3.62 (m, 2H), 3.51-3.45 (m, 1H), 3.37-3.35 (m, 3H), 3.16-3.09 (m, 2H), 2.79 (s, 1H), 2.49 (s, 1H), 2.29 (s, 2H), 2.14 (s, 1H), 1.83 (s, 2H), 1.65-1.62 (m, 6H), 1.42-1.26 (m, 8H), 1.14 (s, 4H), 0.84 (s, 6H).¹³C-NMR (100 MHz, DMSO-*d*₆): δ (ppm) 176.4, 154.3, 151.2, 127.3, 125.3, 121.1, 115.5, 115.3, 62.3, 62.1, 54.1, 52.9, 50.6, 48.1, 47.2, 42.7, 42.2, 40.7, 32.4, 30.2, 30.1, 26.7, 22.6, 22.5, 14.1, 8.9.

### Example 33 Synthesis of (S)-N-(1,3-dihydroxy-2-(hydroxymethyl)propan-2-yl)-1-(4-(3-((1r,3R,5S,7S)-3,5-dimethyladam antan-1-yl)ureido)-3-fluorobenzyl)piperidine-3-carboxamide (CL-115)

This example was operated according to the method of **Example 9,** 2-amino 2-(hydroxymethyl)propane-1,3-diol was used as a raw material (the amine in Example 9 was replaced with 2-amino 2-(hydroxymethyl)propane-1,3-diol) to obtain a white solid, namely **CL-115,** with an output of 0.19 g and a yield of 31%, m.p. 111-112 °C. ¹H NMR(400MHz,CDCl₃):δ (ppm) 7.88 (t, *J*=8.16Hz, 1H), 7.16-7.09 (m, 2H), 6.96-6.94 (m, 1H), 5.50 (s, 1H), 4.63 (s, 2H), 3.67-3.60 (m, 4H), 3.46-3.35 (m, 2H), 3.12 (q, *J*=7.28Hz, 2H), 2.81-2.78 (m, 2H), 2.50 (s, 1H), 2.29 (s, 1H), 2.13 (s, 1H), 1.81 (s, 3H), 1.63 (s, 6H), 1.37-1.26 (m, 7H), 1.17-1.10 (m, 2H), 0.84 (s, 6H).¹³C-NMR (100 MHz, DMSO-*d*₆): δ (ppm) 175.4, 154.1, 152.5, 150.9, 127.7, 125.3, 119.9, 115.6, 62.6, 61.9, 61.1, 60.6, 55.6, 53.3, 52.0, 50.7, 48.1, 46.2, 42.7, 32.4, 32.4, 32.3, 30.6, 30.5, 30.0, 27.7, 23.8.

### Example 34 Synthesis of 1-((1r,3R,5S,7S)-3,5-dimethyladamantan-1-yl)-3-(2-fluoro-4-(((S)-3-(4-(2-hydroxyethyl)piperazi ne-1-carbonyl)piperidin-1-yl)methyl)phenyl)urea (CL-116)

This example was operated according to the method of **Example 9,** 2-(piperazine-1-yl)ethane-1-ol was used as a raw material (the amine in Example 9 was replaced with 2-(piperazine-1-yl)ethane-1-ol) to obtain a white solid, namely **CL-116,** with an output of 0.24 g and a yield of 32%, m.p. 116-117 °C. ¹H NMR(400MHz,CDCl₃):δ (ppm) 7.99 (t, *J*=8.24Hz, 1H), 7.01-6.98 (m, 2H), 6.80 (s, 1H), 5.12 (s, 1H), 3.65-3.62 (m, 3H), 3.49-3.47 (m, 5H), 3.65-3.62 (m, 3H), 3.49-3.47 (m, 5H), 2.89-2.78 (m, 4H), 2.55 (t, *J*=5.32Hz, 2H), 2.50-2.40 (m, 4H), 2.15-2.14 (m, 3H), 1.84-1.73 (m, 4H), 1.66 (s, 4H), 1.36-1.33 (m, 2H), 1.31-1.25 (m, 4H), 1.16-1.15 (m, 2H), 0.84 (s, 6H).¹³C-NMR (100 MHz, DMSO-*d*₆): δ (ppm) 172.6, 154.0, 153.5, 151.1, 125.1, 120.9, 115.4, 115.2, 62.3, 59.2, 57.8, 55.6, 53.7, 53.2, 52.9, 52.6, 50.6, 48.2, 46.8, 45.5, 42.7, 41.6, 40.7, 32.7, 30.2, 30.1, 29.7, 27.3, 24.7, 22.7, 9.2.

### Example 35 Synthesis of (S)-1-(4-(3-((1r,3R,5S,7S)-3,5-dimethyladamantan-1-yl)ureido)-3-fluorobenzyl)piperidine-3-car boxamide (CL-101)

This example was operated according to the method of **Example 9,** ammonia water was used as a raw material (the amine in Example 9 was replaced with ammonia water) to obtain a white solid, namely **CL-101,** with an output of 0.32 g and a yield of 42%, m.p. 87-88 °C. ¹H NMR(400MHz,CDCl₃):δ (ppm) 8.27-8.17 (m, 1H), 7.81-7.56 (m, 1H), 7.04-7.00 (m, 2H), 3.83-3.46 (m, 8H), 2.43-2.35 (m, 2H), 2.12-2.11 (m, 1H), 2.01 (s, 1H), 1.79 (s, 2H), 1.62-1.61 (m, 5H), 1.37-1.33 (m, 2H), 1.29-1.26 (m, 3H), 1.18-1.10 (m, 5H), 0.83 (s, 6H).¹³C-NMR (100 MHz, DMSO-*d*₆): δ (ppm) 173.7, 170.9, 154.1, 153.9, 130.2, 128.1, 122.7, 120.0, 113.4, 52.7, 52.6, 50.8, 50.6, 48.0, 46.9, 46.1, 42.7, 40.6, 40.6, 32.4, 30.2, 30.1, 29.7, 28.3, 26.8, 26.2, 9.6, 9.5.

### Example 36 Synthesis of 1-(4-(3-((1r,7r)-3,5-dimethyladamantan-1-yl)ureido)-3-fluorobenzyl)-N,N-bis(2-hydroxyethyl)pi peridine-4-carboxamide (CL-109)

This example was operated according to the method of **Example 9,** diethanolamine was used as a raw material (the amine in Example 9 was replaced with diethanolamine, and the acid in Example 7 was replaced with 1-(4-(3-((1r,3R,5S,7r)-3,5-dimethyladamantan-1-yl)ureido)-3-fluorobenzyl)piperidine-4-carboxy lic acid) to obtain a white solid, namely **CL-109,** with an output of 0.28 g and a yield of 45%, m.p. 104-105 °C. ¹H NMR(400MHz,CDCl₃):δ (ppm) 7.88 (t, J=8.44Hz, 1H), 7.03-6.94 (m, 2H), 6.61 (s, 1H), 4.92 (s, 1H), 3.83-3.77 (m, 3H), 3.54-3.52 (m, 2H), 3.44 (s, 1H), 2.89-2.81 (m, 3H), 2.57 (s, 1H), 2.15 (s, 1H), 2.03-1.97 (m, 2H), 1.84 (s, 2H), 1.68-1.66 (m, 4H), 1.43 (s, 2H), 1.37-1.21 (m, 8H), 1.20-1.15 (m, 5H), 0.84 (s, 6H). ¹³C-NMR (100 MHz, DMSO-*d₆*): δ (ppm) 177.7, 154.0, 124.9, 121.1, 115.4, 115.2, 61.7, 60.8, 53.0, 52.6, 51.7, 50.6, 50.3, 48.2, 46.6, 42.7, 40.7, 38.8, 32.5, 31.6, 30.2, 30.1, 29.7, 28.7, 26.9, 22.6, 14.1, 10.1.

### Example 37 Synthesis of 1-(4-(3-((1r,7r)-3,5-dimethyladamantan-1-yl)ureido)-3-fluorobenzyl)piperidine-4-carboxamide (CL-118)

This example was operated according to the method of **Example 9,** ammonia water was used as a raw material (the amine in Example 9 was replaced with ammonia water, and the acid in Example 7 was replaced with 1-(4-(3-((1r,3R,5S,7r)-3,5-dimethyladamantan-1-yl)ureido)-3-fluorobenzyl)piperidine-4-carboxy lic acid) to obtain a white solid, namely **CL-118,** with an output of 0.29 g and a yield of 38%, m.p. 117-118 °C. ¹H NMR(400MHz,CDCl₃):δ (ppm) 8.10 (s, 1H), 7.25-7.01 (m, 2H), 6.77 (s, 1H), 6.48 (s, 1H), 3.34 (s, 2H), 3.09 (s, 1H), 2.90 (s, 2H), 2.51 (s, 2H), 2.10 (s, 3H), 1.76-1.58 (m, 8H), 1.40-1.12 (m, 8H), 0.83 (s, 6H).¹³C-NMR (100 MHz, DMSO-*d₆*): δ (ppm) 176.5, 161.4, 154.0, 152.4, 150.8, 125.8, 119.9, 115.9, 52.4, 52.0, 50.2, 48.2, 48.1, 46.2, 43.8, 42.9, 42.7, 42.2, 32.4, 32.3, 30.7, 30.5, 30.0, 28.7, 28.0.

### Example 38 Synthesis of N-(1,3-dihydroxy-2-(hydroxymethyl)propan-2-yl)-1-(4-(3-((1r,7r)-3,5-dimethyladamantan-1-yl) ureido)-3-fluorobenzyl)piperidine-4-carboxamide (CL-117)

This example was operated according to the method of **Example** 9, 2-amino-2-(hydroxymethyl)propane-1,3-diol was used as a raw material (the amine in Example 9 was replaced with 2-amino-2-(hydroxymethyl)propane-1,3-diol, and the acid in Example 7 was replaced with 1-(4-(3-((1r,3R,5S,7r)-3,5-dimethyladamantan-1-yl)ureido)-3-fluorobenzyl) piperidine-4-carboxylic acid) to obtain a white solid, namely **CL-117,** with an output of 0.19 g and a yield of 27%, m.p. 117-118 °C. ¹H NMR(400MHz,DMSO-*d*6):δ (ppm) 8.06-8.01 (m, 2H), 7.08-7.04 (m, 2H), 6.97-6.95 (m, 1H), 6.45 (s, 1H), 4.75 (t, *J*=5.60Hz, 2H), 3.51-3.50 (m, 5H), 3.41-3.33 (m, 4H), 2.87-2.79 (m, 1H), 2.22 (s, 1H), 2.09-2.08 (m, 1H), 1.97-1.84 (m, 2H), 1.77-1.74 (m, 2H), 1.65-1.51 (m, 7H), 1.34-1.24 (m, 6H), 1.15-1.11 (m, 2H), 0.83 (s, 6H).¹³C-NMR (100 MHz, DMSO-*d₆*): δ (ppm) 173.8, 154.1, 152.5, 150.9, 132.1, 125.2, 119.9, 115.4, 72.7, 61.9, 60.7, 60.6, 60.5, 55.7, 53.3, 52.9, 52.0, 50.7, 48.1, 46.2, 42.8, 42.7, 42.6, 32.3, 30.5, 30.0, 29.5, 27.7, 24.2, 22.5.

### Example 39 Synthesis of N-(1,3-dihydroxypropan-2-yl)-1-(4-(3-((1r,7r)-3,5-dimethyladamantan-1-yl)ureido)-3-fluorobenz yl)piperidine-4-carboxamide (CL-119)

This example was operated according to the method of **Example 9,** 2-amino-1,3-propanediol was used as a raw material (the amine in Example 9 was replaced with 2-amino-1,3-propanediol, and the acid in Example 7 was replaced with 1-(4-(3-((1r,3R,5S,7r)-3,5-dimethyladamantan-1-yl)ureido)-3-fluorobenzyl)piperidine-4-carboxy lic acid) to obtain a white solid, namely **CL-119,** with an output of 0.17 g and a yield of 28%, m.p. 150-151 °C. ¹H NMR(400MHz,DMSO-*d*6):δ (ppm) 8.61-8.60 (m, 1H), 8.39 (dd, *J*=8.36Hz, 1.12Hz, 1H), 8.36 (s, 1H), 8.12 (t, *J*=8.44Hz, 1H), 7.67 (d, *J*=7.88Hz, 1H), 7.41-7.38 (m, 2H), 7.15 (d, *J*=8.64Hz, 1H), 6.75 (s, 1H), 4.66 (s, 2H), 3.95 (s, 2H), 3.68-3.64 (m, 1H), 3.39-3.38 (m, 6H), 2.09 (s, 1H), 1.80-1.76 (m, 5H), 1.61-1.54 (m, 4H), 1.36-1.27 (m, 4H), 1.11 (s, 3H), 0.83 (s, 6H). ¹³C-NMR (100 MHz, DMSO-*d₆*): δ (ppm) 173.4, 154.0, 152.0, 150.4, 129.1, 128.0, 119.7, 118.1, 61.4, 60.9, 58.9, 58.2, 54.8, 53.6, 51.0, 50.7, 49.0, 48.2, 42.7, 32.5, 32.2, 30.6, 29.5, 28.8, 26.2, 25.6, 22.8, 22.5.

### Example 40 Synthesis of 1-(4-(3-((1r,7r)-3,5-dimethyladamantan-1-yl)ureido)-3-fluorobenzyl)-N-(2-hydroxypropyl)piperi dine-4-carboxamide (CL-120)

This example was operated according to the method of **Example 9,** 1-amino-2-propanol was used as a raw material (the amine in Example 9 was replaced with 1-amino-2-propanol, and the acid in Example 7 was replaced with 1-(4-(3-((1r,3R,5S,7r)-3,5-dimethyladamantan-1-yl)ureido)-3-fluorobenzyl)piperidine-4-carboxy lic acid) to obtain a white solid, namely **CL-120,** with an output of 0.15 g and a yield of 28%, m.p. 99-100 °C. ¹H NMR(400MHz,CDCl₃):δ (ppm) 7.88 (t, *J*=8.24Hz, 1H),7.02-6.93 (m, 2H), 6.84 (s, 1H), 6.28 (s, 1H), 5.12 (s, 1H), 3.92-3.88 (m, 1H), 3.41 (s, 3H), 3.14-2.96 (m, 3H), 2.88-2.86 (m, 2H), 2.14-1.96 (m, 4H), 1.83-1.72 (m, 5H), 1.68-1.62 (m, 4H), 1.38-1.26 (m, 4H), 1.18-1.15 (m, 4H), 0.86 (s, 6H).¹³C-NMR (100 MHz, DMSO-*d₆*): δ (ppm) 176.5, 154.4, 151.5, 126.5, 124.8, 121.3, 115.3, 115.1, 67.4, 62.0, 52.9, 52.8, 50.6, 48.2, 46.9, 43.0, 42.7, 40.7, 32.4, 30.2, 30.1, 28.7, 20.9, 9.6.

### Example 41 Synthesis of methyl (1r,4r)-4-(3-fluoro-4-nitrobenzamido)cyclohexane-1-carboxylate

This example was operated according to the method in **Example 5,** except that the ethyl (S)-piperidine-3-carboxylate was replaced with methyl (1r,4r)-4-aminocyclohexane-1-carboxylate. A white solid finally obtained was the methyl (1r,4r)-4-(3-fluoro-4-nitrobenzamido)cyclohexane-1-carboxylate, with an output of 2.4 g and a yield of 91%.

### Example 42 Synthesis of methyl (1r,4r)-4-(4-amino-3-fluorobenzamido)cyclohexane-1-carboxylate

This example was operated according to the method in **Example 6,** except that the ethyl (S)-1-(3-fluoro-4-nitrobenzyl)piperidine-3-carboxylate was replaced with methyl (1r,4r)-4-(3-fluoro-4-nitrobenzamido)cyclohexane-1-carboxylate. A white solid finally obtained was the methyl (1r,4r)-4-(4-amino-3-fluorobenzamido)cyclohexane-1-carboxylate, with an output of 2.1 g and a yield of 90%.

### Example 43 Synthesis of methyl (1R,4r)-4-(4-(3-((1r,3R,5S,7r)-3,5-dimethyladamantan-1-yl)ureido)-3-fluorobenzamido)cyclohex ane-1-carboxylate

This example was operated according to the method in **Example** 7, except that the ethyl (S)-1-(4-amino-3-fluorobenzyl)piperidine-3-carboxylate was replaced with methyl (1r,4r)-4-(4-amino-3-fluorobenzamido)cyclohexane-1-carboxylate. A white solid finally obtained was the methyl (1R,4r)-4-(4-(3-((1r,3R,5S,7r)-3,5-dimethyladamantan-1-yl)ureido) -3-fluorobenzamido)cyclohexane-1-carboxylate, with an output of 3.4 g and a yield of 80%.

### Example 44 Synthesis of (1R,4r)-4-(4-(3-((1r,3R,5S,7r)-3,5-dimethyladamantan-1-yl)ureido)-3-fluorobenzamido)cyclohex ane-1-carboxylic acid

This example was operated according to the method in **Example 8,** except that the ethyl (S)-1-(4-(3-((1r,3R,5S,7S)-3,5-dimethyladamantan-1-yl)ureido)-3-fluorobenzyl)piperidine-3-car boxylate was replaced with methyl (1R,4r)-4-(4-(3-((1r,3R,5S,7r)-3,5-dimethyladamantan-1-yl)ureido)-3-fluorobenzamido)cyclohex ane-1-carboxylate. A white solid finally obtained was the (1R,4r)-4-(4-(3-((1r,3R,5S,7r)-3,5-dimethyladamantan-1-yl)ureido)-3-fluorobenzamido)cyclohex ane-1-carboxylic acid, with an output of 2.5 g and a yield of 90%.

### Example 45 Synthesis of methyl (1r,4r)-4-((3-fluoro-4-nitrobenzyl)amino)cyclohexane-1-carboxylate

4-(Bromomethyl)-2-fluoro-1-nitrobenzene (3.0 g, 12.9 mmol) and dry acetonitrile (35 mL) were added into a single-necked flask. After the 4-(bromomethyl)-2-fluoro-1-nitrobenzene was dissolved, potassium carbonate (2.13 g, 15.4 mmol), potassium iodide (0.15 g, 1.29 mmol), and methyl (1r,4r)-4-aminocyclohexane-1-carboxylate (2.24 g, 12.9 mmol) were added thereto and the mixture was refluxed for reaction. After 6 h, the reaction was completed as monitored by TLC. The reaction mixture was concentrated under reduced pressure to remove acetonitrile, water (20 mL) was added thereto, and a resulting mixture was extracted with ethyl acetate (30 mL×2). The organic layers were combined, washed with water (20 mL), and washed once with saturated aqueous NaCl solution (25 mL). The organic phase was concentrated under reduced pressure to obtain 5.2 g of a yellow oily substance, which was loaded into a column with 4 times silica gel, after being mixed with 1.2 times silica gel, and subjected to column chromatography with an eluent (EA: PE=1:40) to obtain a yellow oily substance, namely the methyl (1r,4r)-4-((3-fluoro-4-nitrobenzyl)amino)cyclohexane-1-carboxylate, with an output of 4.2 g and a yield of 82%.

### Example 46 Synthesis of methyl (1r,4r)-4-((4-amino-3-fluorobenzyl)amino)cyclohexane-1-carboxylate

The methyl (1r,4r)-4-((3-fluoro-4-nitrobenzyl)amino)cyclohexane-1-carboxylate (3.60 g, 11.5 mmol), 5% Pd-C (0.4 g), and anhydrous ethanol (50 mL) were added into a single-necked flask, and the atmosphere therein was replaced with argon three times, and replaced with hydrogen three times. The resulting system was heated to 60°C and stirred for reaction for 12 h. The reaction was completed as monitored by TLC; the reaction solution was then cooled to room temperature (25 °C), and filtered. A filtrate was concentrated under reduced pressure to obtain a yellow oily substance, namely the methyl (1r,4r)-4-((4-amino-3-fluorobenzyl)amino)cyclohexane-1-carboxylate, with an output of 2.96g and a yield of 89 %.

### Example 47 Synthesis of methyl (1R,4r)-4-((4-(3-((1r,3R,5S,7r)-3,5-dimethyladamantan-1-yl)ureido)-3-fluorobenzyl)amino)cyclo hexane-1-carboxylate

BTC (1.06 g, 3.8 mmol) and dry DCM (30 mL) were added into a three-necked flask, and a resulting mixture was cooled to below -78 °C with cold trap. A solution of the methyl (1R,4r)-4-((4-(3-((1r,3R,5S,7r)-3,5-dimethyladamantan-1-yl)ureido)-3-fluorobenzyl)amino)cyclo hexane-1-carboxylate (2.23 g, 7.6 mmol) and TEA (1.06 g, 3.980 mmol) in dry DCM (60 mL) was added dropwise thereto. After drop addition, the resulting system was transferred to room temperature and stirred at room temperature for 0.5 h, and the reaction was stopped. An obtained reaction solution was concentrated to dryness under reduced pressure, and the residue was dissolved with dry DCM (10 mL) to obtain an isocyanate solution for later use.

Memantine (1.36 g, 7.6 mmol), TEA (1.54 g, 15.2 mmol), and dry DCM (25 mL) were added into a three-necked flask, and the isocyanate solution was added dropwise thereto. A reaction was conducted at room temperature for 0.5 h, and the reaction was completed as monitored by TLC. A reaction solution was poured into water (40 mL), and a resulting mixture was extracted with DCM (40 mL×3), followed by washing with 1 mol/L HCl (40 mL×2), washing with water (40 mL×2), and washing with saturated aqueous NaCl solution (40 mL) in sequence, and drying over anhydrous sodium sulfate, and filtering with suction. A resulting filtrate was concentrated under reduced pressure to obtain 3.4 g of a light yellow oily substance, namely the methyl (1R,4r)-4-((4-(3-((1r,3R,5S,7r)-3,5-dimethyladamantan-1-yl)ureido)-3-fluorobenzyl)amino)cyclo hexane-1-carboxylate, which was then used for next step without purification.

### Example 48 Synthesis of (1R,4r)-4-((4-(3-((1r,3R,5S,7r)-3,5-dimethyladamantan-1-yl)ureido)-3-fluorobenzyl)amino)cyclo hexane-1-carboxylic acid

Methyl (1R,4r)-4-((4-(3-((1r,3R,5S,7r)-3,5-dimethyladamantan-1-yl) ureido)-3-fluorobenzyl)amino)cyclohexane-1-carboxylate (3.4 g, 6.9 mmol) was added into a single-necked flask, and dissolved in THF (20 mL). Sodium hydroxide (0.36 g, 9.00 mmol) and water (10 mL) were added thereto and a resulting mixture was refluxed for reaction. After 12 h, TLC showed that the reaction was completed. A resulting reaction solution was concentrated under reduced pressure to remove THF, and water (300 mL) was added into the residue. A resulting mixture was placed in a cold trap and the pH value thereof was adjusted to 3 with 6 N hydrochloric acid (10 mL). A light yellow solid was precipitated, followed by filtering with suction. A filter cake was rinsed with water (20 mL) and dried to obtain 2.96 g of a crude product (dried in an oven at 60 °C for 24 h). The crude product was purified by slurrying with petroleum ether and diethyl ether to obtain a light yellow solid, namely the (1R,4r)-4-((4-(3-((1r,3R,5S,7r)-3,5-dimethyladamantan-1-yl)ureido)-3-fluorobenzyl)amino)cyclo hexane-1-carboxylic acid, with an output of 2.9 g and a yield of 89.5%.

### Example 49 Synthesis of 4-(3-((1r,3R,5S,7r)-3,5-dimethyladamantan-1-yl)ureido)-3-fluoro-N-((1r,4R)-4-((2-hydroxyethyl )carbamoyl)cyclohexyl)benzamide (ZT-110)

This example was operated according to the method of **Example 9,** 2-aminoethane-1-ol was used as a raw material (the amine in Example 9 was replaced with 2-aminoethane-1-ol, and the acid was replaced with (1R,4r)-4-(4-(3-((1r,3R,5S,7r)-3,5-dimethyladamantan-1-yl)ureido)-3-fluorobenzamido)cyclohex ane-1-carboxylic acid) to obtain a white solid, namely **ZT-110,** with an output of 0.14 g and a yield of 16%, m.p. 117-118 °C. ¹H NMR (400 MHz, DMSO-*d*₆) δ 8.38 (s, 1H), 8.23 (t, 1H, *J*=8.40 Hz), 8.09 (d, 1H, *J*=7.8 Hz), 7.73 (t, 1H, *J*=5.3 Hz), 7.66-7.59 (m, 2H), 6.64 (s, 1H), 4.64 (s, 1H), 3.71-3.69 (m, 1H), 3.24 (s, 1H), 3.12-3.08 (m, 2H), 3.01-3.00 (m, 1H), 2.09 (s, 2H), 1.87-1.84 (m, 2H), 1.76 (s, 4H), 1.59 (s, 4H), 1.47-1.41 (m, 2H), 1.37-1.32 (m, 4H), 1.28-1.25 (m, 2H), 1.12 (s, 2H), 0.83 (s, 6H). ¹³C NMR (100 MHz, DMSO-*d*₆): δ 175.40, 164.27, 153.71, 152.09, 149.70, 124.07, 118.50, 114.21, 114.01, 60.44, 60.44, 52.16, 52.16, 50.70, 48.36, 47.98, 47.98, 46.01, 43.70, 42.75, 41.80, 32.39, 32.39, 32.00, 30.51, 30.51, 30.04, 28.86, 28.86.

### Example 50 Synthesis of 4-(3-((1r,3R,5S,7r)-3,5-dimethyladamantan-1-yl)ureido)-3-fluoro-N-((1r,4R)-4-((2-hydroxyprop yl)carbamoyl)cyclohexyl)benzamide (ZT-111)

This example was operated according to the method of **Example 9,** 1-aminopropan-2-ol was used as a raw material (the amine in Example 9 was replaced with 1-aminopropan-2-ol, and the acid was replaced with (1R,4r)-4-(4-(3-((1r,3R,5S,7r)-3,5-dimethyladamantan-1-yl)ureido)-3-fluorobenzamido)cyclohex ane-1-carboxylic acid) to obtain a white solid, namely **ZT-111,** with an output of 0.16 g and a yield of 18%, m.p. 123-124 °C. ¹H NMR (400 MHz, DMSO-*d*₆) δ 8.35 (d, 1H, *J*=2.96 Hz), 8.23 (t, 1H, *J*=8.52 Hz), 8.09 (d, 1H, *J*=7.92 Hz), 7.69-7.59 (m, 3H), 6.60 (s, 1H), 4.63 (s, 1H), 3.74-3.67 (m, 1H), 3.64-3.59 (m, 1H), 2.98 (t, 2H, *J*=5.88 Hz), 2.13-2.09 (m, 2H), 1.87-1.84 (m, 2H), 1.76 (s, 3H), 1.59 (s, 3H), 1.48-1.42 (m, 2H), 1.35-1.34 (m, 1H), 1.32 (s, 2H), 1.28 (s, 2H), 1.23 (s, 2H), 1.17 (s, 1H), 1.12 (s, 2H), 1.00 (d, 3H, *J*=6.2 Hz), 0.83 (s, 6H). ¹³C NMR (100 MHz, DMSO-*d*₆):175.40, 164.27, 153.69, 152.10, 149.71, 124.04, 118.50, 114.22, 114.01, 65.75, 65.75, 52.17, 52.17, 50.71, 48.38, 47.99, 47.99, 46.67, 43.68, 42.76, 32.39, 32.39, 32.00, 30.51, 30.51, 30.05, 28.93, 28.84, 21.51.

### Example 51 Synthesis of N-((1r,4R)-4-(bis(2-hydroxyethyl)carbamoyl)cyclohexyl)-4-(3-((1r,3R,5S,7r)-3,5-dimethyladam antan-1-yl)ureido)-3-fluorobenzamide (ZT-112)

This example was operated according to the method of **Example** 9, 2,2'-azadiylbis(ethane-1-ol) was used as a raw material (the amine in Example 9 was replaced with 2,2'-azadiylbis(ethane-1-ol), and the acid was replaced with (1R,4r)-4-(4-(3-((1r,3R,5S,7r)-3,5-dimethyladamantan-1-yl)ureido)-3-fluorobenzamido)cyclohex ane-1-carboxylic acid) to obtain a white solid, namely **ZT-112,** with an output of 0.12 g and a yield of 14%, m.p. 104-105 °C. ¹H NMR (400 MHz, DMSO-*d*₆) δ 8.38 (d, 1H, *J*=2.72 Hz), 8.23 (t, 1H, *J*=8.52 Hz), 8.11 (d, 1H, *J*=7.6 Hz), 7.66-7.59 (m, 2H), 6.63 (s, 1H), 4.88 (s, 1H), 4.67 (s, 1H), 3.71-3.69 (m, 1H), 3.52-3.51 (m, 2H), 3.44-3.43 (m, 4H),3.34-3.32 (m, 1H),2.61-2.55 (m, 1H), 2.10 (s, 1H), 1.88-1.85 (m, 2H), 1.76-1.70 (m, 4H), 1.59 (s, 4H), 1.48-1.44 (m, 2H), 1.41-1.38 (m, 2H), 1.35-1.32 (m, 3H), 1.28-1.25 (m, 2H), 1.12 (s, 2H), 0.83 (s, 6H). ¹³C NMR (100 MHz, DMSO-*d*₆): δ 175.61, 164.37, 153.70, 152.09, 149.69, 124.09, 118.51, 114.24, 114.03, 59.81, 59.40, 52.16, 50.89, 50.70, 48.76, 48.66, 47.98, 47.98, 42.76, 39.12, 32.39, 32.39, 32.39, 31.89, 30.51, 30.51, 30.51, 30.04, 28.91, 28.91.

### Example 52 Synthesis of N-((1r,4R)-4-carbamoylcyclohexyl)-4-(3-((1r,3R,5S,7r)-3,5-dimethyladamantan-1-yl)ureido)-3-f luorobenzamide (ZT-113)

This example was operated according to the method of **Example 9,** ammonia water was used as a raw material (the amine in Example 9 was replaced with ammonia water, and the acid was replaced with (1R,4r)-4-(4-(3-((1r,3R,5S,7r)-3,5-dimethyladamantan-1-yl)ureido)-3-fluorobenzamido)cyclohex ane-1-carboxylic acid) to obtain a white solid, namely **ZT-113,** with an output of 0.18 g and a yield of 20%, m.p. 107-108 °C. ¹¹H NMR (400 MHz, DMSO-*d*₆) δ 8.53 (s, 1H), 8.22 (t, 1H, *J*=8.12 Hz), 8.15 (d, 1H, *J*=7.64 Hz), 7.68-7.60 (m, 2H), 7.26 (s, 1H), 6.88 (s, 1H), 6.70 (s, 1H), 3.69 (s, 1H), 3.05 (q, 4H, *J*=6.84 Hz), 2.69 (s, 1H), 2.09 (s, 1H), 1.77 (s, 3H), 1.59 (s, 3H), 1.45-1.35 (m, 4H), 1.32 (s, 2H), 1.27 (s, 2H), 1.12 (s, 2H), 0.83 (s, 6H). ¹³C NMR (100 MHz, DMSO-*d*₆): δ 177.47, 164.29, 153.82, 152.11, 149.71, 124.07, 118.57, 114.21, 114.02, 52.15, 50.72, 48.41, 47.99, 45.76, 45.76, 43.46, 42.77, 38.71, 32.38, 32.38, 32.00, 30.53, 30.53, 30.03, 28.79, 8.89, 8.89.

### Example 53 Synthesis of 4-(3-((1r,3R,5S,7r)-3,5-dimethyladamantan-1-yl)ureido)-3-fluoro-N-((1r,4R)-4-((2-hydroxyprop yl)carbamoyl)cyclohexyl)benzamide (ZT-114)

This example was operated according to the method of **Example 9,** 2-aminopropane-1,3-diol was used as a raw material (the amine in Example 9 was replaced with 2-aminopropane-1,3-diol, and the acid was replaced with (1R,4r)-4-(4-(3-((1r,3R,5S,7r)-3,5-dimethyladamantan-1-yl)ureido)-3-fluorobenzamido)cyclohex ane-1-carboxylic acid) to obtain a white solid, namely **ZT-114,** with an output of 0.18 g and a yield of 20%, m.p. 110-111 °C. ¹¹H NMR (400 MHz, DMSO-*d*₆) δ 8.38 (s, 1H), 8.22 (t, 1H, *J*=8.5 Hz), 8.09 (d, 1H, *J*=7.9 Hz), 7.65-7.59 (m, 2H), 7.42 (d, 1H, *J*=8.1 Hz), 6.64 (s, 1H), 4.62 (s, 2H), 3.71-3.66 (m, 2H), 3.39-3.38 (m, 4H), 2.10 (s, 1H), 1.86-1.84 (m, 2H), 1.76 (s, 3H), 1.59 (s, 4H), 1.47-1.41 (m, 2H),1.37-1.32 (m, 4H), 1.28 (s, 2H), 1.25 (s, 2H), 1.12 (s, 2H), 0.83 (s, 6H). ¹³C NMR (100 MHz, DMSO-*d*₆): δ 175.33, 164.23, 153.72, 152.10, 149.71, 124.05, 118.52, 114.21, 114.01, 60.67, 60.67, 53.04, 52.16, 52.16, 50.70, 48.37, 47.98, 47.98, 43.66, 42.76, 32.39, 32.39, 32.39, 32.00, 30.52, 30.52, 30.04, 28.92, 28.92.

### Example 54 Synthesis of N-((1r,4R)-4-((1,3-dihydroxy-2-(hydroxymethyl)propan-2-yl)carbamoyl)cyclohexyl)-4-(3-((1r,3 R,5S,7r)-3,5-dimethyladamantan-1-yl)ureido)-3-fluorobenzamide (ZT-115)

This example was operated according to the method of **Example 9,** 2-amino-2-(hydroxymethyl)propane-1,3-diol was used as a raw material (the amine in Example 9 was replaced with 2-amino-2-(hydroxymethyl)propane-1,3-diol, and the acid was replaced with (1R,4r)-4-(4-(3-((1r,3R,5S,7r)-3,5-dimethyladamantan-1-yl)ureido)-3-fluorobenzamido) cyclohexane-1-carboxylic acid) to obtain a white solid, namely **ZT-115,** with an output of 0.20 g and a yield of 24%, m.p. 121-122 °C. ¹H NMR (400 MHz, DMSO-*d*₆) δ 8.53 (s, 1H), 8.22 (t, 1H, *J*=8.4 Hz), 8.14 (d, 1H, *J*=7.8 Hz), 7.67-7.60 (m, 2H), 7.29 (s, 1H), 6.89 (s, 1H), 4.88 (s, 3H), 3.70 (s, 1H), 3.52 (s, 5H), 3.17 (d, 1H, *J*=5.2 Hz), 2.26 (s, 1H), 2.09 (s, 1H), 1.85-1.77 (m, 6H), 1.59 (s, 3H), 1.46-1.40 (m, 2H),1.36-1.32 (m, 4H), 1.27-1.25 (m, 3H), 1.12 (s, 2H), 0.83 (s, 6H). ¹³C NMR (100 MHz, DMSO-*d*₆): δ 177.09, 164.28, 153.84, 152.11, 149.72, 124.08, 118.59, 114.21, 114.00, 62.62, 61.27, 61.27, 52.15, 52.15, 50.73, 49.04, 48.35, 47.99, 47.99, 43.86, 42.77, 38.71, 32.38, 32.38, 32.38, 31.89, 30.53, 30.53, 30.53, 30.03, 28.98.

### Example 55 Synthesis of tert-butyl 4-(2-((1R,4r)-4-(4-(3-((1r,3R,5S,7r)-3,5-dimethyladamantan-1-yl)ureido)-3-fluorobenzamido)cy clohexane-1-carboxamido)ethyl)piperazine-1-carboxylate (ZT-116)

This example was operated according to the method of **Example** 9, 2-amino-2-(hydroxymethyl)propane-1,3-diol was used as a raw material (the amine in Example 9 was replaced with 2-amino-2-(hydroxymethyl)propane-1,3-diol, and the acid was replaced with (1R,4r)-4-(4-(3-((1r,3R,5S,7r)-3,5-dimethyladamantan-1-yl)ureido)-3-fluorobenzamido) cyclohexane-1-carboxylic acid) to obtain a white solid, namely **ZT-116,** with an output of 0.21 g and a yield of 25%, m.p. 131-132 °C. ¹H NMR (400 MHz, DMSO-*d*₆) δ 8.36 (s, 1H), 8.23 (t, 1H, *J*=8.52 Hz), 8.09 (d, 1H, *J*=7.88 Hz), 7.68-7.59 (m, 3H), 6.62 (s, 1H), 3.75-3.66 (m, 1H), 3.29 (s, 3H), 3.16 (s, 2H), 2.33 (s, 5H), 2.10-2.06 (m, 2H), 1.87-1.85 (m, 2H), 1.76 (s, 4H), 1.59 (s, 4H), 1.47-1.45 (m, 1H), 1.39 (s, 10H), 1.34-1.32 (m, 4H), 1.28 (s, 2H), 1.25-1.23 (m, 2H), 1.12 (s, 2H), 0.83 (s, 6H). ¹³C NMR (150 MHz, DMSO-*d₆*): δ 175.21, 164.27, 154.25, 153.69, 152.09, 149.70, 124.04, 118.47, 114.22, 114.01, 79.21, 57.44, 52.91, 52.16, 52.16, 50.70, 48.38, 47.98, 47.98, 46.12, 43.72, 43.72, 42.76, 42.76, 36.45, 32.39, 32.39, 31.98, 31.98, 30.51, 30.51, 30.04, 30.04, 28.83, 28.83, 28.52, 28.52, 28.52.

### Example 56 Synthesis of 4-(3-((1r,3R,5S,7r)-3,5-dimethyladamantan-1-yl)ureido)-3-fluoro-N-((1r,4R)-4-(4-(2-)hydroxyet hyl)piperazine-1-carbonyl)cyclohexyl)benzamide (ZT-117)

This example was operated according to the method of **Example 9,** 2-(piperazine-1-yl)ethane-1-ol was used as a raw material (the amine in Example 9 was replaced with 2-(piperazine-1-yl)ethane-1-ol, and the acid was replaced with (1R,4r)-4-(4-(3-((1r,3R,5S,7r)-3,5-dimethyladamantan-1-yl)ureido)-3-fluorobenzamido)cyclohex ane-1-carboxylic acid) to obtain a white solid, namely **ZT-117,** with an output of 0.21 g and a yield of 25%, m.p. 126-127 °C. ¹H NMR (400 MHz, DMSO-*d*₆) δ 8.35 (s, 1H), 8.23 (t, 1H, *J =* 8.4 Hz), 8.13 (d, 1H, *J* = 7.6 Hz), 7.66-7.60 (m, 2H), 6.61 (s, 1H), 4.44 (s, 1H), 3.71 (s, 1H), 3.53-3.44 (m, 6H), 2.42-2.41 (m, 4H), 2.35 (s, 2H), 2.10 (s, 1H), 1.87 - 1.85 (m, 2H), 1.76-1.69 (m, 4H), 1.59 (s, 4H), 1.46 (s, 1H), 1.43 (s, 1H), 1.40 (s, 1H), 1.35 (s, 1H), 1.32 (s, 2H), 1.28-1.23 (m, 4H), 1.12 (s, 2H), 0.83 (s, 6H). ¹³C NMR (100 MHz, DMSO-*d*₆): δ 173.30, 164.38, 153.69, 152.07, 149.68, 124.15, 118.46, 114.26, 114.05, 60.54, 58.90, 54.26, 53.45, 52.16, 52.16, 50.70, 48.70, 47.98, 47.98, 45.29, 42.75, 42.75, 41.50, 38.71, 32.39, 32.39, 31.83, 31.83, 30.51, 30.51, 30.39, 30.39, 28.68.

### Example 57 Synthesis of ethyl (S)-1-(4-amino-3-fluorobenzoyl)piperidine-3-carboxylate

This example was operated according to the method in **Example 6,** except that the ethyl (S)-1-(3-fluoro-4-nitrobenzyl)piperidine-3-carboxylate was replaced with ethyl (S)-1-(4-amino-3-fluorobenzoyl)piperidine-3-carboxylate. A white solid finally obtained was the ethyl (S)-1-(4-amino-3-fluorobenzoyl)piperidine-3-carboxylate, with an output of 4.1 g and a yield of 90%.

### Example 58 Synthesis of ethyl (S)-1-(4-(3-((1r,3R,5S,7S)-3,5-dimethyladamantan-1-yl)ureido)-3-fluorobenzoyl)piperidine-3-ca rboxylate

This example was operated according to the method in **Example 7,** except that the ethyl (S)-1-(4-amino-3-fluorobenzyl)piperidine-3-carboxylate was replaced with ethyl (S)-1-(4-amino-3-fluorobenzoyl)piperidine-3-carboxylate. A white solid finally obtained was the ethyl (S)-1-(4-(3-((1r,3R,5S,7S)-3,5-dimethyladamantan-1-yl)ureido)-3-fluorobenzoyl) piperidine-3-carboxylate, with an output of 5.4 g and a yield of 80%.

### Example 59 Synthesis of (S)-1-(4-(3-((1r,3R,5S,7S)-3,5-dimethyladamantan-1-yl)ureido)-3-fluorobenzoyl)piperidine-3-ca rboxylic acid

This example was operated according to the method in **Example 8,** except that the ethyl (S)-1-(4-(3-((1r,3R,5S,7S)-3,5-dimethyladamantan-1-yl)ureido)-3-fluorobenzyl)piperidine-3-car boxylate was replaced with ethyl (S)-1-(4-(3-((1r,3R,5S,7S)-3,5-dimethyladamantan-1-yl)ureido)-3-fluorobenzoyl)piperidine-3-carboxylate. A white solid finally obtained was the (S)-1-(4-(3-((1r,3R,5S,7S)-3,5-dimethyladamantan-1-yl)ureido)-3-fluorobenzoyl)piperidine-3-ca rboxylic acid, with an output of 4.8 g and a yield of 90%.

### Example 60 Synthesis of tert-butyl 4-((S)-1-(4-(3-((1r,3R,5S,7S)-3,5-dimethyladamantan-1-yl)ureido)-3-fluorobenzoyl)piperidine-3 -carbonyl)-1,4-diaza-1-carboxylate

(S)-1-(4-(3-((1r,3R,5S,7S)-3,5-dimethyladamantan-1-yl)ureido)-3-fluorobenzoyl)piperidi ne-3-carboxylic acid (0.80 g, 1.45 mmol) and dry THF (20 mL) were added into a single-necked flask. After the (S)-1-(4-(3-((1r,3R,5S,7S)-3,5-dimethyladamantan-1-yl)ureido) -3-fluorobenzoyl)piperidine-3-carboxylic acid was dissolved, HATU (0.83 g, 2.17 mmol) and DIEA (0.58 g, 4.34 mmol) were added thereto, a resulting mixture was stirred for 60 min until the solution turned light yellow, and a solution of N-Boc-homopiperazine (0.29 g, 1.45 mmol) in THF (10 mL) was added dropwise thereto. After 2 h, the reaction was completed as monitored by TLC, and the reaction was stopped. The reaction system was concentrated under reduced pressure to remove THF, water (40 mL) was added thereto, and a resulting mixture was extracted with ethyl acetate (50 mL×2). The organic layers were combined and washed with water (40 mL × 2), once with 1 N hydrochloric acid (40 mL), once with saturated sodium carbonate (40 mL), once with water (40 mL), and once with saturated aqueous NaCl solution (40 mL). The organic phase was concentrated under reduced pressure to obtain 1.2 g of a yellow oily substance, which was loaded on a 3 times silica gel column, mixed with 1.2 times silica gel, and subjected to column chromatography with an eluent (EA: PE=1:1) to obtain a yellow oily substance, with an output of 0.68 g and a yield of 75.5%.

### Example 61 Synthesis of 1-(4-((S)-3-(1,4-diaza-1-carbonyl)piperidine-1-carbonyl)-2-fluorophenyl)-3-((1r,3R,5S,7S)-3,5-d imethyladamantan-1-yl)urea

Tert-butyl 4-((S)-1-(4-(3-((1r,3R,5S,7S)-3,5-dimethyladamantan-1-yl)ureido) -3-fluorobenzoyl)piperidine-3-carbonyl)-1,4-diaza-1-carboxylate (0.6 g, 1.25 mmol) was added into a single-necked flask and dissolved in dry DCM (8 mL), TFA (4 mL) was added dropwise thereto. After 20 min, the reaction was completed as monitored by TLC, and a resulting reaction solution was concentrated under reduced pressure to remove TFA to obtain a product, which was directly used for the next step.

### Example 62 Synthesis of 1-((1r,3R,5S,7S)-3,5-dimethyladamantan-1-yl)-3-(2-fluoro-4-((3S)-3-(4-(2-methylbutanoyl)-1,4-diaza-1-carbonyl)piperidine-1-carbonyl)phenyl)urea (GL-104)

This example was operated according to the method of **Example 9,** 2-methylbutyric acid was used as a raw material (the acid in Example 9 was replaced with 2-methylbutyric acid, and the alkali was replaced with 1-(4-((S)-3-(1,4-diaza-1-carbonyl)piperidine-1-carbonyl)-2-fluorophenyl)-3-((1r,3R,5S,7S)-3,5-d imethyladamantan-1-yl)urea) to obtain a white solid, namely **GL-104,** with an output of 0.25 g and a yield of 36%, m.p. 149-150 °C. ¹H NMR(400MHz,CDCl₃):δ (ppm) 8.26-8.20 (m, 1H), 7.58 (s, 1H), 7.24-7.08 (m, 2H), 5.90 (s, 1H), 4.59 (s, 1H), 3.82-3.37 (m, 10H), 3.05-2.55 (m, 4H), 2.13-2.05(m, 2H), 1.80-1.62 (m, 11H), 1.37-1.24 (m, 6H), 1.14-1.06 (m, 5H), 0.86 (s, 2H), 0.83 (s, 6H).¹³C-NMR (100 MHz, DMSO-*d₆*): δ (ppm) 175.8, 172.6, 168.5, 153.8, 121.7, 150.2, 130.4, 128.8, 123.8, 119.2, 52.1, 50.7, 48.2, 47.9, 47.8, 46.8, 46.7, 46.2, 46.1, 45.1, 44.2, 42.7, 36.7, 36.6, 36.5, 36.4, 32.4, 30.5, 30.0, 28.7, 27.4, 27.3, 27.2, 27.2, 18.0, 17.7.

### Example 63 Synthesis of 1-((1r,3R,5S,7S)-3,5-dimethyladamantan-1-yl)-3-(2-fluoro-4-((S)-3-(4-propionyl-1,4-diaza-1-car bonyl)piperidine-1-carbonyl)phenyl)urea (GL-102)

This example was operated according to the method of **Example 9,** propionic acid was used as a raw material (the acid in Example 7 was replaced with propionic acid, and the alkali was replaced with 1-(4-((S)-3-(1,4-diaza-1-carbonyl)piperidine-1-carbonyl)-2-fluorophenyl)-3-((1r,3R,5S,7S)-3,5-d imethyladamantan-1-yl)urea) to obtain a white solid, namely **GL-102,** with an output of 0.31 g and a yield of 42%, m.p. 96-98 °C. ¹H NMR(400MHz,CDCl₃):δ (ppm) 8.30 (s, 1H),8.16-8.13 (m, 1H), 7.59 (s, 1H), 6.99 (s, 2H), 5.88 (s, 1H), 4.48 (s, 1H), 3.71-3.60 (m, 7H), 3.07-3.04 (m, 3H), 2.69 (s, 2H), 2.25 (s, 3H), 2.05 (s, 1H), 1.74 (s, 6H), 1.56 (s, 4H), 1.22-1.18 (m, 3H), 1.06-1.01 (m, 5H), 0.76 (s, 6H).¹³C-NMR (100 MHz, DMSO-*d₆*): δ (ppm) 173.9, 169.7, 168.4, 154.0, 152.2, 150.6, 130.4, 130.3, 120.1, 124.8, 54.7, 52.8, 50.6, 47.9, 47.4, 47.3, 42.8, 42.7, 40.6, 32.4, 30.2, 30.1, 29.7, 27.7, 26.5, 26.1, 25.9, 18.5, 17.1, 12.2, 9.6, 9.5, 9.4.

### Example 64 Synthesis of 1-(4-((S)-3-(4-acetyl-1,4-diaza-1-carbonyl)piperidine-1-carbonyl)-2-fluorophenyl)-3-((1r,3R,5S, 7S)-3,5-dimethyladamantan-1-yl)urea (GL-101)

This example was operated according to the method of **Example 9,** acetic acid was used as a raw material (the acid in Example 9 was replaced with acetic acid, and the alkali was replaced with 1-(4-((S)-3-(1,4-diaza-1-carbonyl)piperidine-1-carbonyl)-2-fluorophenyl)-3-((1r,3R,5S,7S)-3,5-d imethyladamantan-1-yl)urea) to obtain a white solid, namely **GL-101,** with an output of 0.28 g and a yield of 37%, m.p. 136-137 °C. ¹H NMR(400MHz,CDCl₃):δ (ppm) 8.26-8.20 (m, 1H), 7.60 (s, 1H), 7.08 (s, 2H), 5.92 (s, 1H), 4.58 (s, 1H), 3.74-3.47 (m, 9H), 3.14-2.60 (m, 3H), 2.13-2.05 (m, 4H), 1.87-1.80 (m, 6H), 1.63 (s, 4H), 1.42-1.35 (m, 5H), 1.29-1.24 (m, 3H), 1.18-1.11(m, 2H), 0.83 (s, 6H).¹³C-NMR (100 MHz, DMSO-*d₆*): δ (ppm) 175.8, 172.6, 168.5, 153.8, 151.7, 150.2, 130.3, 128.8, 123.8, 119.2, 52.1, 50.7, 48.2, 47.9, 46.7, 46.6, 46.2, 46.1, 45.1, 44.2, 42.7, 36.6, 36.5, 36.4, 32.4, 30.5, 30.0, 27.4, 27.3, 27.2, 18.1, 12.3, 12.2.

### Example 65 Synthesis of tert-butyl-4-((S)-1-(4-(3-((1r,3R,5S,7S)-3,5-dimethyladamantan-1-yl)ureido)-3-fluorobenzyl)pip eridine-3-carbonyl)-1,4-diaza-1-carboxylate

This example was operated according to the method of **Example 9,** tert-butyl 1,4-diaza-1-carboxylate was used as a raw material (the amine in Example 9 was replaced with tert-butyl 1,4-diaza-1-carboxylate, and the acid was replaced with (S)-1-(4-(3-((1r,3R,5S,7S)-3,5-dimethyladamantan-1-yl)ureido)-3-fluorobenzyl)piperidine-3-car boxylic acid) to obtain a white solid, namely the tert-butyl-4-((S)-1-(4-(3-((1r,3R,5S,7S)-3,5-dimethyladamantan-1-yl)ureido)-3-fluorobenzyl)pip eridine-3-carbonyl)-1,4-diaza-1-carboxylate, with an output of 3.4 g and a yield of 86%.

### Example 66 Synthesis of 1-(4-(((S)-3-(1,4-diaza-1-carbonyl)piperidin-1-yl)methyl)-2-fluorophenyl)-3-((1r,3R,5S,7S)-3,5-dimethyladamantan-1-yl)urea

Tert-butyl 4-((S)-1-(4-(3-((1r,3R,5S,7S)-3,5-dimethyladamantan-1-yl)ureido)-3-fluorobe nzyl)piperidine-3-carbonyl)-1,4-diaza-1-carboxylate (3.0 g, 55.6 mmol) was added into a si ngle-necked flask and dissolved in dry DCM (8 mL), and TFA (4 mL) was added drop wise thereto. After 20 min, a reaction was completed as monitored by TLC, and a result ing reaction solution was concentrated under reduced pressure to remove TFA to obtain a product, which was directly used for the next step.

### Example 67 Synthesis of 1-((1r,3R,5S,7S)-3,5-dimethyladamantan-1-yl)-3-(2-fluoro-4-(((S)-3-(5-propionyl-1,5-)diazoalka ne-1-carbonyl)piperidin-1-yl)methyl)phenyl)urea (CL-102)

This example was operated according to the method of **Example 9,** propionic acid was used as a raw material (the acid in Example 9 was replaced with propionic acid, and the amine in Example 9 was 1-(4-(((S)-3-(1,4-diaza-1-carbonyl)piperidin-1-yl)methyl) -2-fluorophenyl)-3-((1r,3R,5S,7S)-3,5-dimethyladamantan-1-yl)urea) to obtain a white solid, namely **CL-102,** with an output of 0.25 g and a yield of 38%, m.p. 99-100 °C. ¹H-NMR (400 MHz, CDCl₃): δ (ppm) 8.02-7.97 (m, 1H), 7.02-7.69 (m, 2H), 6.85 (br, 1H), 5.20 (m, 1H), 3.84-3.73 (m, 1H), 3.69-3.58 (m, 2H), 3.56-3.49 (m, 4H), 3.47-3.33 (m, 4H), 2.90-2.75 (m, 3H), 2.37-28 (m, 2H), 2.15-2.14 (m, 2H), 1.98-1.92 (m, 1H), 1.84 (s, 4H), 1.74-1.69 (m, 2H), 1.66 (s, 4H), 1.64-1.59 (m, 1H), 1.55-1.46 (m, 1H), 1.40-1.37 (m, 2H), 1.33-1.24 (m, 3H), 1.20-1.13 (m, 3H), 1.12-1.08 (m, 2H), 0.85 (s, 6H).¹³C-NMR (151 MHz, CDCl₃): δ (ppm) 174.19, 173.76, 173.59, 173.16, 154.02, 125.08, 120.93, 115.33, 115.14, 62.39, 56.09, 55.92, 53.42, 52.95, 50.66, 48.56, 47.47, 47.38, 46.92, 46.21, 44.53, 44.29, 42.74, 40.78, 39.47, 32.45, 30.11, 27.84, 27.67, 27.08, 26.54, 25.99, 24.75, 9.57, 9.41.

### Example 68 Synthesis of 1-((1r,3R,5S,7S)-3,5-dimethyladamantan-1-yl)-3-(2-fluoro-4-(((3S)-3-(5-(2-methylbutanoyl)-1,5 -diazoalkane-1-carbonyl)piperidin-1-yl)methyl)phenyl)urea (CL-103)

This example was operated according to the method of **Example 9,** 2-methylbutyric acid was used as a raw material (the acid in Example 9 was replaced with 2-methylbutyric acid, and the amine in Example 9 was replaced with 1-(4-(((S)-3-(1,4-diaza-1-carbonyl)piperidin-1-yl)methyl)-2-fluorophenyl)-3-((1r,3R,5S,7S)-3,5-dimethyladamantan-1-yl)urea) to obtain a white solid, namely **CL-103,** with an output of 0.19 g and a yield of 31%, m.p. 108-110 °C. ¹H-NMR (400 MHz, CDCl₃): δ (ppm) 8.04-7.95 (m, 1H), 7.02-7.95 (m, 2H), 6.83 (br, 1H), 5.18 (m, 1H), 3.87-3.74 (m, 1H), 3.66-3.55 (m, 4H), 3.53-3.46 (m, 4H), 3.40-3.38 (m, 2H), 2.84-2.73 (m, 3H), 2.55-2.51 (m, 1H), 2.15 (s, 2H), 2.00-1.84 (m, 6H), 1.71-1.62 (m, 7H), 1.58-1.53 (m, 2H), 1.43-1.37 (m, 3H), 1.31 (s, 1H), 1.26-1.24 (m, 1H), 1.19-1.15 (m, 2H), 1.12-1.05 (m, 2H), 0.88-0.83 (m, 9H).¹³C-NMR (151 MHz, CDCl₃): δ (ppm) 176.63, 176.48, 174.21, 174.12, 154.05, 125.02, 120.92, 115.30, 115.11, 62.45, 56.20, 56.10, 55.99, 53.51, 52.95, 50.66, 48.22, 42.75, 40.78, 37.34, 32.46, 30.23, 30.11, 28.04, 27.88, 27.68, 27.42, 27.35, 27.26, 27.13, 27.01, 24.81, 17.90, 17.46, 10.04.

### Example 69 Synthesis of 1-(4-(((S)-3-(4-acryloyl-1,4-diaza-1-carbonyl)piperidin-1-yl)methyl)-2-fluorophenyl)-3-((1r,3R,5 S,7S)-3,5-dimethyladamantan-1-yl)urea (CL-201)

This example was operated according to the method of **Example 9,** acrylic acid was used as a raw material (the acid in Example 9 was replaced with acrylic acid, and the amine in Example 7 was replaced with 1-(4-(((S)-3-(1,4-diaza-1-carbonyl)piperidin-1-yl)methyl)-2-fluorophenyl)-3-((1r,3R,5S,7S)-3,5-dimethyladamantan-1-yl)urea) to obtain a white solid, namely **CL-201,** with an output of 0.15 g and a yield of 28%, m.p. 62-65 °C. ¹H NMR(400MHz,DMSO-*d₆*):δ (ppm) 8.93-8.85 (m, 2H), 8.17 (s, 2H), 7.20-7.09 (m, 2H), 6.52 (s, 1H), 6.51-5.59 (m, 1H), 3.65-3.59 (m, 8H), 3.53-3.51 (m, 6H), 2.09 (s, 1H), 1.75-1.60 (m, 6H), 1.58 (s, 5H), 1.34-1.32 (m, 5H), 1.28-1.23 (m, 3H), 0.86 (s, 6H).¹³C-NMR (100 MHz, DMSO-*d₆*): δ (ppm) 168.5, 165.7, 165.3, 153.9, 150.5, 128.9, 128.3, 128.0, 119.8, 52.9, 52.7, 52.6, 52.2, 52.1, 50.7, 48.2, 44.8, 44.3, 42.7, 32.4, 30.5, 30.0, 27.7, 27.3, 25.6, 25.2, 24.8.

### Example 70 Synthesis of 1-(4-(((S)-3-(4-(cyclopropanecarbonyl)-1,4-diaza-1-carbonyl)piperidin-1-yl)methyl)-2-fluorophe nyl)-3-((1r,3R,5S,7S)-3,5-dimethyladamantan-1-yl)urea (CL-204)

This example was operated according to the method of **Example 9,** cyclopropanecarboxylic acid was used as a raw material (the acid in Example 9 was replaced with cyclopropanecarboxylic acid, and the amine in Example 9 was replaced with 1-(4-(((S)-3-(1,4-diaza-1-carbonyl)piperidin-1-yl)methyl)-2-fluorophenyl)-3-((1r,3R,5S,7S)-3,5-dimethyladamantan-1-yl)urea) to obtain a white solid, namely **CL-204,** with an output of 0.19 g and a yield of 29%, m.p. 106-108 °C. ¹H NMR (400 MHz, DMSO-*d₆*): δ (ppm) 8.00-7.96 (m, 1H), 7.05-6.98 (m, 2H), 6.74 (s, 1H), 5.04 (s, 1H), 3.76-3.48 (m, 12H), 2.87-2.78 (m, 3H), 2.16-2.14 (m, 1H), 1.93-1.90 (m, 1H), 1.83-1.73 (m, 4H), 1.72-1.66 (m, 8H), 1.40-1.37 (m, 2H), 1.28-1.26 (m, 2H), 1.20-1.12 (m, 2H), 0.96-0.91 (m, 2H), 0.85 (s, 6H), 0.77-0.75 (m, 2H).¹³C-NMR (100 MHz, DMSO-*d₆*): δ (ppm) 169.6, 168.8, 168.4, 167.7, 149.5, 149.1, 120.5, 116.4, 110.7, 49.3, 48.7, 48.1, 45.9, 43.6, 43.1, 42.7, 42.5, 40.3, 39.7, 37.9, 37.4, 36.1, 28.0, 27.7, 25.6, 25.1, 23.7, 23.7, 23.0, 22.4, 21.9, 6.4, 4.4, 3.0, 2.8.

### Example 71 Synthesis of 1-((1r,3R,5S,7S)-3,5-dimethyladamantan-1-yl)-3-(2-fluoro-4-((S)-3-(4-methacryloyl-1,4-)diaza-1-carbonyl)piperidine (CL-205)

This example was operated according to the method of **Example 9,** methacrylic acid was used as a raw material (the acid in Example 9 was replaced with methacrylic acid, and the amine in Example 9 was replaced with 1-(4-(((S)-3-(1,4-diaza-1-carbonyl)piperidin-1-yl)methyl)-2-fluorophenyl)-3-((1r,3R,5S,7S)-3,5-dimethyladamantan-1-yl)urea) to obtain a white solid, namely **CL-205,** with an output of 0.23 g and a yield of 32%, m.p. 79-81 °C. ¹H NMR (400 MHz, DMSO-*d₆*): δ (ppm) 7.99 (s, 1H), 7.19-7.02 (m, 2H), 5.15 (s, 1H), 4.96-4.93 (s, 1H), 3.60-3.49 (m, 7H), 3.13-3.11 (m, 4H), 2.15-2.08 (m, 1H), 1.93-1.90 (m, 2H), 1.84 (m, 2H), 1.77-1.76 (m, 2H), 1.66 (m, 3H), 1.31-1.25 (m, 14H), 1.17-1.15 (m, 2H), 0.85 (s, 6H), 0.80-0.77 (m, 2H).¹³C-NMR (100 MHz, DMSO-d6): δ (ppm) 171.9, 171.4, 153.9, 152.1, 150.5, 141.0, 119.8, 115.1, 114.5, 60.1, 52.1, 50.7, 49.4, 48.5, 48.0, 46.7, 46.4, 45.6, 44.9, 43.9, 43.3, 42.7, 32.5, 32.4, 32.2, 30.5, 30.0, 28.2, 28.0, 23.0, 20.7, 20.6, 19.1.

### Example 72 Synthesis of tert-butyl 4-(3-fluoro-4-nitrobenzoyl)-1,4-diaza-1-carboxylate

3-Fluoro-4-nitrobenzoic acid (3.0 g, 16.2 mmol) and dry THF (40 mL) were added into a single-necked flask. After the 3-fluoro-4-nitrobenzoic acid was dissolved, HATU (7.39 g, 19.5 mmol) and DIEA (5.03 g, 38.90 mmol) were added thereto, and the resulting mixture was stirred for 60 min until the solution turned light yellow, and N-Boc homopiperazine (3.25 g, 16.2 mmol) was then added dropwise thereto. After 15 min, the reaction was completed as monitored by TLC, and the reaction was stopped. The reaction system was concentrated under reduced pressure to remove THF, water (20 mL) was added thereto, and a resulting mixture was extracted with DCM (15 mL×2). The organic layers were combined and washed once with 1 N hydrochloric acid (20 mL), once with saturated sodium carbonate (20 mL), once with water (20 mL), and once with saturated aqueous NaCl solution (20 mL). The organic phase was concentrated under reduced pressure to obtain 6.7 g of a light yellow oily substance, which was loaded into a column with 5 times silica gel, after being mixed with 1.2 times silica gel, and subjected to column chromatography with an eluent (EA: PE=1:1) to obtain a white solid, namely the tert-butyl 4-(3-fluoro-4-nitrobenzoyl)-1,4-diaza-1-carboxylate, with an output of 4.8 g and a yield of 82%.

### Example 73 Synthesis of tert-butyl 4-(4-amino-3-fluorobenzoyl)-1,4-diaza-1-carboxylate

This example was operated according to the method in **Example 6,** except that the ethyl (S)-1-(3-fluoro-4-nitrobenzyl)piperidine-3-carboxylate was replaced with tert-butyl 4-(3-fluoro-4-nitrobenzoyl)-1,4-diaza-1-carboxylate. A white solid finally obtained was the tert-butyl 4-(4-amino-3-fluorobenzoyl)-1,4-diaza-1-carboxylate, with an output of 4.1 g and a yield of 90%.

### Example 74 Synthesis of tert-butyl 4-(4-(3-((1r,3R,5S,7r)-3,5-dimethyladamantan-1-yl)ureido)-3-fluorobenzoyl)-1,4-diaza-1-carbox ylate

This example was operated according to the method in **Example** 7, except that the ethyl (S)-1-(4-amino-3-fluorobenzyl)piperidine-3-carboxylate was replaced with tert-butyl 4-(4-amino-3-fluorobenzoyl)-1,4-diaza-1-carboxylate. A white solid finally obtained was the tert-butyl 4-(4-amino-3-fluorobenzoyl)-1,4-diaza-1-carboxylate, with an output of 5.3 g and a yield of 78%.

### Example 75 Synthesis of 1-(4-(1,4-diaza-1-carbonyl)-2-fluorophenyl)-3-((1r,3R,5S,7r)-3,5-dimethyladamantan-1-yl)urea

Tert-butyl 4-(4-(3-((1r,3R,5S,7r)-3,5-dimethyladamantan-1-yl)ureido)-3-fluorobenzoyl)-1,4-diaza-1-carboxylate (2.0 g, 35.6 mmol) was added into a single-necked flask and dissolved in dry DCM (8 mL), and TFA (4 mL) was added dropwise thereto. After 20 min, the reaction was completed as monitored by TLC, and a resulting reaction solution was concentrated under reduced pressure to remove TFA to obtain a product, which was directly used for the next step.

### Example 76 Synthesis of tert-butyl 4-(2-(4-(4-(3-((1r,3R,5S,7r)-3,5-dimethyladamantan-1-yl)ureido)-3-fluorobenzoyl)-1,4-diaza-1-y l)-2-oxoethyl)piperidine-1-carboxylate

This example was operated according to the method of **Example 9,** 2-(1-(tert-butoxycarbonyl)piperidin-4-yl)acetic acid was used as a raw material (the acid in Example 9 was replaced with 2-(1-(tert-butoxycarbonyl)piperidin-4-yl)acetic acid, and the alkali in Example 7 was replaced with 1-(4-(1,4-diaza-1-carbonyl)-2-fluorophenyl)-3-((1r,3R,5S,7r)-3,5-dimethyladamantan-1-yl)urea) to obtain a white solid, namely the tert-butyl 4-(2-(4-(4-(3-((1r,3R,5S,7r)-3,5-dimethyladamantan-1-yl)ureido)-3-fluorobenzoyl)-1,4-diaza-1-y l)-2-oxoethyl)piperidine-1-carboxylate, with an output of 1.9 g and a yield of 85%.

### Example 77 Synthesis of 1-(4-(1,4-diaza-1-carbonyl)-2-fluorophenyl)-3-((1r,3R,5S,7r)-3,5-dimethyladamantan-1-yl)urea

Tert-butyl 4-(2-(4-(4-(3-((1r,3R,5S,7r)-3,5-dimethyladamantan-1-yl)ureido)-3-fluorobenzoyl)-1,4-diaza-1-yl)-2-oxoethyl)piperidine-1-carboxylate (5.0 g, 65.6 mmol) was added into a single-necked flask and dissolved in dry DCM (8 mL), and TFA (4 mL) was added dropwise thereto. After 20 min, the reaction was completed as monitored by TLC, and a resulting reaction solution was concentrated under reduced pressure to remove TFA to obtain a product, which was directly used for the next step.

### Example 78 Synthesis of 1-((1r,3R,5S,7r)-3,5-dimethyladamantan-1-yl)-3-(2-fluoro-4-(4-(2-(1-(2-methylbutyryl)piperidin -4-yl)acetyl)-1,4-diaza-1-carbonyl)phenyl)urea (CC-103)

This example was operated according to the method of **Example 9,** 2-methylbutyric acid was used as a raw material (the acid in Example 9 was replaced with 2-methylbutyric acid, and the alkali was replaced with 1-(4-(1,4-diaza-1-carbonyl)-2-fluorophenyl)-3-((1r,3R,5S,7r)-3,5-dimethyladamantan-1-yl)urea) to obtain a white solid, namely **CC-103,** with an output of 0.24 g and a yield of 35%, m.p. 106-107 °C. ¹H NMR(400MHz,CDCl₃):δ (ppm) 8.26-8.21 (m, 1H), 7.57-7.42 (m, 1H), 7.05-7.00 (m, 2H), 4.63 (s, 1H), 3.73-3.46 (m, 9H), 3.02 (s, 1H), 2.66-2.59 (m, 2H), 2.30-2.27 (m, 2H), 2.14 (s, 2H), 1.99 (s, 1H), 1.82 (s, 4H), 1.71-1.64 (m, 5H), 1.40-1.36 (m, 3H), 1.30-1.19 (m, 3H), 1.19-1.10 (m, 3H), 1.10-1.07 (m, 4H), 0.90-0.87 (m, 3H), 0.83 (s, 6H).¹³C-NMR (100 MHz, DMSO-*d₆*): δ (ppm) 175.1, 171.2, 171.0, 170.8, 153.9, 130.2, 128.3, 122.8, 120.1, 133.6, 60.4, 52.8, 50.7, 48.1, 45.7, 42.7, 42.11, 40.7, 39.6, 39.1, 36.9, 33.3, 33.2, 32.4, 30.2, 30.1, 27.1, 21.1, 17.3, 14.2, 10.7.

### Example 79 Synthesis of 1-((1r,3R,5S,7r)-3,5-dimethyladamantan-1-yl)-3-(2-fluoro-4-(4-(2-(1-propionylpiperidin-4-yl)ac etyl)-1,4-diaza-1-carbonyl)phenyl)urea (CC-105)

This example was operated according to the method of **Example 9,** propionic acid was used as a raw material (the acid in Example 9 was replaced with propionic acid, and the alkali was replaced with 1-(4-(1,4-diaza-1-carbonyl)-2-fluorophenyl)-3-((1r,3R,5S,7r)-3,5-dimethyladamantan-1-yl)urea) to obtain a white solid, namely **CC-105,** with an output of 0.25 g and a yield of 37%, m.p. 95-96 °C. ¹H NMR(400MHz,CDCl₃):δ (ppm) 8.27-8.22 (m, 1H), 7.68-7.56 (m, 1H), 7.04-7.02 (m, 2H), 4.60 (s, 1H), 3.72-3.46 (m, 9H), 3.62 (s, 1H), 2.58 (s, 1H), 2.37-2.25 (m, 4H), 2.13-1.99 (m, 4H), 1.81 (s, 4H),1.63 (s, 5H), 1.38-1.24 (m, 4H), 1.14-1.11 (m, 7H), 0.84 (s, 6H).¹³C-NMR (100 MHz, DMSO-*d₆*): δ (ppm) 171.2, 171.0, 170.9, 154.1, 130.4, 130.3, 128.1, 122.7, 120.1, 113.5, 60.4, 52.7, 50.6, 48.1, 46.4, 45.7, 43.9, 42.7, 41.9, 40.6, 39.6, 39.1, 33.2, 33.1, 32.4, 32.0, 30.2, 30.1, 29.7, 29.3, 26.6, 21.1, 14.2, 9.7, 9.7.

### Example 80 Synthesis of 1-(4-(4-(2-(1-acetylpiperidin-4-yl)acetyl)-1,4-diaza-1-carbonyl)-2-fluorophenyl)-3-((1r,3R,5S,7r) -3,5-dimethyladamantan-1-yl)urea (CC-106)

This example was operated according to the method of **Example 9,** acetic acid was used as a raw material (the acid in Example 9 was replaced with acetic acid, and the alkali was replaced with 1-(4-(1,4-diaza-1-carbonyl)-2-fluorophenyl)-3-((1r,3R,5S,7r)-3,5-dimethyladamantan-1-yl)urea) to obtain a white solid, namely **CC-106,** with an output of 0.31 g and a yield of 42%, m.p. 101-102 °C. ¹H NMR(400MHz,CDCl₃):δ (ppm) 8.27-8.22 (m, 1H), 7.68-7.55 (m, 1H), 7.04-7.02 (m, 2H), 4.60 (s, 1H), 3.72-3.46 (m, 9H), 3.02 (s, 1H), 2.58 (s, 1H), 2.37-2.25 (m, 4H), 2.13-1.99 (m, 4H), 1.88 (s, 4H), 1.63 (s, 5H), 1.38-1.26 (m, 4H), 1.14-1.11 (m, 7H), 0.84 (s, 6H).¹³C-NMR (100 MHz, DMSO-*d₆*): δ (ppm) 169.9, 169.8, 168.1, 153.0, 129.4, 129.3, 127.1, 121.7, 119.1, 112.5, 59.4, 52.8, 51.8, 49.6, 47.1, 45.6, 41.7, 41.0, 40.8, 39.6, 32.0, 31.9, 31.7, 31.7, 31.4, 30.9, 29.2, 29.1, 20.4, 20.0, 17.6, 16.4, 13.2, 10.9.

### Example 81 Synthesis of 1-((1r,3R,5S,7r)-3,5-dimethyladamantan-1-yl)-3-(4-(4-(2-(1-(1-(dimethylamino)propan-2-yl)piperidin-4-yl)acetyl)-1,4-diaza-1-carbonyl)-2-fluoropheny l)urea (CC-124)

1-(4-(1,4-diaza-1-carbonyl)-2-fluorophenyl)-3-((1r,3R,5S,7r)-3,5-dimethyladamantan-1-yl)urea (0.5 g, 1.4 mmol) and dry acetonitrile (35 mL) were added into a single-necked flask. After the 4-(bromomethyl)-2-fluoro-1-nitrobenzene was added thereto and dissolved, potassium carbonate (0.3 g, 1.6 mmol), potassium iodide (0.02 g, 0.2 mmol), and 2-chloro-N,N-dimethylpropane-1-amine (0.2 g, 1.4 mmol) were added thereto and refluxed. After 6 h, the reaction was completed as monitored by TLC. The reaction mixture was concentrated under reduced pressure to remove acetonitrile, water (20 mL) was added thereto, and a resulting mixture was extracted with ethyl acetate (30 mL×2). The organic layers were combined, washed with water (20 mL), and washed once with saturated aqueous NaCl solution (25 mL). The organic phase was concentrated under reduced pressure to obtain 0.8 g of a light yellow oily substance, which was loaded into a column with 4 times silica gel, after being mixed with 1.2 times silica gel, and subjected to column chromatography with an eluent (EA: PE=1:40) to obtain a white solid, namely the **CC-124,** with an output of 0.34 g and a yield of 35%. ¹H NMR(400MHz,CDCl₃):δ (ppm) 8.28-8.20 (m, 1H), 7.76-7.57 (m, 1H), 7.05-7.02 (m, 2H), 5.97-5.84 (m, 1H), 3.81-3.40 (m, 9H), 2.88-2.80 (m, 3H), 2.47-2.43 (m, 1H), 2.31-2.25 (m, 7H), 2.25-2.23 (m, 2H), 2.13-2.12 (m, 2H), 2.04-1.91 (m, 3H), 1.80 (s, 2H), 1.67-1.62 (m, 6H), 1.37-1.26 (m, 7H), 1.81-1.11 (m, 2H), 1.01-0.99 (m, 4H), 0.84 (s, 6H).¹³C-NMR (100 MHz, DMSO-*d₆*): δ (ppm) 174.3, 168.3, 153.9, 151.8, 150.2, 130.3, 123.8, 119.4, 114.2, 73.6, 61.5, 60.6, 58.2, 53.2, 52.1, 50.7, 47.9, 46.6, 45.9, 42.7, 42.2, 32.4, 32.3, 30.5, 30.0, 29.4, 29.1, 19.2.

### Example 82 Synthesis of tert-butyl 4-(3-fluoro-4-nitrobenzyl)-1,4-diaza-1-carboxylate

This example was operated according to the method in **Example 5,** except that the ethyl (S)-piperidine-3-carboxylate was replaced with N-Boc homopiperazine. A white solid finally obtained was the tert-butyl 4-(3-fluoro-4-nitrobenzyl)-1,4-diaza-1-carboxylate, with an output of 4.1 g and a yield of 94%.

### Example 83 Synthesis of tert-butyl 4-(4-amino-3-fluorobenzyl)-1,4-diaza-1-carboxylate

This example was operated according to the method in **Example 6,** except that the ethyl (S)-1-(3-fluoro-4-nitrobenzyl)piperidine-3-carboxylate was replaced with tert-butyl 4-(3-fluoro-4-nitrobenzyl)-1,4-diaza-1-carboxylate. A white solid finally obtained was the tert-butyl 4-(4-amino-3-fluorobenzyl)-1,4-diaza-1-carboxylate, with an output of 3.5 g and a yield of 90%.

### Example 84 Synthesis of tert-butyl 4-(4-(3-((1r,3R,5S,7r)-3,5-dimethyladamantan-1-yl)ureido)-3-fluorobenzyl)-1,4-diaza-1-carboxy late

This example was operated according to the method in **Example 7,** except that the ethyl (S)-1-(4-amino-3-fluorobenzyl)piperidine-3-carboxylate was replaced with tert-butyl 4-(4-amino-3-fluorobenzyl)-1,4-diaza-1-carboxylate. A white solid finally obtained was the tert-butyl 4-(4-(3-((1r,3R,5S,7r)-3,5-dimethyladamantan-1-yl)ureido)-3-fluorobenzyl)-1,4-diaza-1-carboxylate, with an output of 5.4 g and a yield of 86%.

### Example 85 Synthesis of 1-(4-((1,4-diaza-1-yl)methyl)-2-fluorophenyl)-3-((1r,3R,5S,7r)-3,5-dimethyladamantan-1-yl)urea

Tert-butyl 4-(4-(3-((1r,3R,5S,7r)-3,5-dimethyladamantan-1-yl)ureido)-3-fluorobenzoyl)-1,4-diaza-1-carboxylate (2.0 g, 35.6 mmol) was added into a single-necked flask and dissolved in dry DCM (8 mL), and TFA (4 mL) was added dropwise thereto. After 20 min, the reaction was completed as monitored by TLC, and a resulting reaction solution was concentrated under reduced pressure to remove TFA to obtain a product with an output of 1.7 g and yield of 89%, which was directly used for the next step.

### Example 86 Synthesis of tert-butyl 4-(2-(4-(4-(3-((1r,3R,5S,7r)-3,5-dimethyladamantan-1-yl)ureido)-3-fluorobenzyl)-1,4-diaza-1-yl) -2-oxoethyl)piperidine-1-carboxylate

1-(4-((1,4-diaza-1-yl)methyl)-2-fluorophenyl)-3-((1r,3R,5S,7r)-3,5-dimethyladamantan-1-yl)urea (0.80 g, 1.35 mmol) and dry THF (20 mL) were added into a one-necked flask. After the 1-(4-((1,4-diaza-1-yl)methyl)-2-fluorophenyl)-3-((1r,3R,5S,7r)-3,5-dimethyladamantan-1-yl)urea was dissolved, HATU (0.83 g, 2.17 mmol) and DIEA (0.58 g, 4.34 mmol) were added thereto, a resulting mixture was stirred for 60 min until the solution turned light yellow, and a solution (10 mL) of 2-(1-(tert-butoxycarbonyl)piperidin-4-yl)acetic acid (0.29 g, 1.35 mmol) in THF was added dropwise thereto. After 2 h, the reaction was completed as monitored by TLC, and the reaction was stopped. The reaction system was concentrated under reduced pressure to remove THF, water (40 mL) was added thereto, and a resulting mixture was extracted with ethyl acetate (50 mL×2). The organic layers were combined and washed with water (40 mL × 2), once with 1 N hydrochloric acid (40 mL), once with saturated sodium carbonate (40 mL), once with water (40 mL), and once with saturated aqueous NaCl solution (40 mL). The organic phase was concentrated under reduced pressure to obtain 1.2 g of a yellow oily substance, which was loaded on a 3 times silica gel column, mixed with 1.2 times silica gel, and subjected to column chromatography with an eluent (EA: PE=1:1) to obtain a yellow oily substance, with an output of 0.7 g and a yield of 75.5%.

### Example 87 Synthesis of 1-(4-(((S)-3-(1,4-diaza-1-carbonyl)piperidin-1-yl)methyl)-2-fluorophenyl)-3-((1r,3R,5S,7S)-3,5-dimethyladamantan-1-yl)urea

Tert-butyl 4-(2-(4-(4-(3-((1r,3R,5S,7r)-3,5-dimethyladamantan-1-yl)ureido)-3-fluorobenzyl)-1,4-diaza-1-yl)-2-oxoethyl)piperidine-1-carboxylate (0.7 g, 3.6 mmol) was added into a single-necked flask and dissolved in dry DCM (8 mL), and TFA (4 mL) was added dropwise thereto. After 20 min, the reaction was completed as monitored by TLC, and a resulting reaction solution was concentrated under reduced pressure to remove TFA to obtain a product with an output of 0.6 g and a yield of 89%, which was directly used for the next step.

### Example 88 Synthesis of 1-((1r,3R,5S,7r)-3,5-dimethyladamantan-1-yl)-3-(2-fluoro-4-((4-(2-(1-propionylpiperidin-4-yl)acetyl)-1,4-diaza-1-yl)methyl)phenyl)urea (GL-1)

This example was operated according to the method of **Example 9,** propionic acid was used as a raw material (the acid in Example 9 was replaced with propionic acid, and the alkali was replaced with 1-(4-(((S)-3-(1,4-diaza-1-carbonyl)piperidin-1-yl)methyl) -2-fluorophenyl)-3-((1r,3R,5S,7S)-3,5-dimethyladamantan-1-yl)urea) to obtain a white solid, namely GL-1, with an output of 0.13 g and a yield of 29%, m.p. 86-89 °C. ¹H NMR(400MHz,CDCl₃):δ (ppm) 8.04-7.99 (m, 1H), 7.10-7.09 (s, 1H), 7.01-6.93 (m, 3H), 5.45-5.41 (m, 1H), 4.65-4.58 (m, 1H), 3.85-3.82 (d, 1H, *J=* 12.56 Hz), 3.71-3.43(m, 6H), 3.07-3.01 (m, 1H), 2.66 (s, 1H), 2.60-2.56 (m, 4H), 2.35 (q, 2H, *J*= 7.48 Hz), 2.26-2.14 (m, 4H), 1.85-1.84 (m, 4H), 1.67 (s, 5H), 1.39-1.36 (m, 2H), 1.30-1.27 (m, 3H), 1.16-1.12 (m, 6H), 0.85 (s, 6H). ¹³C NMR (100 MHz, CDCl₃): δ 172.36, 172.32, 171.17, 171.10, 154.26, 154.23, 124.57, 120.86, 120.77, 61.93, 61.59, 55.55, 55.41, 55.07, 52.83, 52.81, 50.67, 48.18, 45.81, 45.76, 42.75, 40.75, 39.56, 39.54, 33.24, 33.21, 32.89, 32.43, 32.05, 30.22, 30.14, 26.65, 26.64, 9.72, 9.68.

### Example 89 Synthesis of 1-((1r,3R,5S,7r)-3,5-dimethyladamantan-1-yl)-3-(2-fluoro-4-((4-(2-(1-(2-methylbutyryl)piperidi n-4-yl)acetyl)-1,4-diaza-1-yl)methyl)phenyl)urea (GL-2)

This example was operated according to the method of **Example 9,** 2-methylbutyric acid was used as a raw material (the acid in Example 9 was replaced with 2-methylbutyric acid, and the alkali was replaced with 1-(4-(((S)-3-(1,4-diaza-1-carbonyl) piperidin-1-yl)methyl)-2-fluorophenyl)-3-((1r,3R,5S,7S)-3,5-dimethyladamantan-1-yl)urea) to obtain a white solid, namely **GL-2,** with an output of 0.18 g and a yield of 31%, m.p. 96-100 °C. ¹H NMR(400MHz,CDCl₃):δ (ppm) 7.99 (t, 1H, *J=* 8.36 Hz), 7.17 (d, 1H, *J*= 7.48 Hz), 7.06 (s, 1H), 6.99 (s, 1H), 6.96-6.93 (m, 1H), 5.50-5.48 (m, 1H), 4.68-4.61 (m, 1H), 3.97-3.83 (d, 1H, *J*=12.76 Hz), 3.75-3.44 (m, 6H), 3.08-3.02 (m, 1H), 2.65-2.55 (m, 6H), 2.27-2.14 (m, 4H), 1.84 (s, 5H), 1.66 (s, 5H), 1.39-1.36 (m, 3H), 1.30-1.26 (m, 3H), 1.16-1.14 (m, 2H), 1.11-1.08 (m, 4H), 0.89-0.87 (m, 3H), 0.84 (s, 6H). ¹³C NMR (100 MHz, CDCl₃): δ 175.04, 171.22, 171.14, 154.31, 154.31, 124.56, 121.00, 120.87, 114.94, 114.75, 61.91, 61.59, 55.53, 55.43, 52.81, 52.78, 50.68, 48.20, 47.17, 45.81, 45.35, 44.49, 42.76, 42.18, 40.74, 39.56, 36.95, 33.37, 33.27, 30.22, 30.13, 28.46, 27.51, 27.06, 17.39, 17.20, 12.01, 11.91.

### Example 90 Synthesis of 1-(4-((4-(2-(1-acetylpiperidin-4-yl)acetyl) -1,4-diaza-1-yl)methyl)-2-fluorophenyl)-3-((1r,3R,5S,7r)-3,5-dimethyladamantan-1-yl)urea (GL-3)

This example was operated according to the method of **Example 9,** acetic acid was used as a raw material (the acid in Example 9 was replaced with acetic acid, and the alkali was replaced with 1-(4-(((S)-3-(1,4-diaza-1-carbonyl)piperidin-1-yl)methyl)-2-fluorophenyl)-3-((1r,3R,5S,7S)-3,5-dimethyladamantan-1-yl)urea) to obtain a white solid, namely **GL-3,** with an output of 0.12 g and a yield of 23%, m.p. 92-95 °C. ¹H NMR(400MHz,CDCl₃):δ (ppm) 8.05-8.00 (m, 1H), 7.11 (s, 1H), 7.03-6.99 (m, 2H), 6.95-6.93 (m, 1H), 5.47 (s, 1H), 4.63-4.57 (m, 1H), 3.81-3.78 (m, 1H), 3.72-3.65(m, 2H), 3.56-3.49 (m, 3H), 3.46-3.43 (m, 1H), 3.11-3.05 (m, 1H), 2.67 (s, 1H), 2.59-2.57 (m, 4H), 2.26-2.23 (m, 1H), 2.18 (s, 1H), 2.15-2.14 (m, 1H), 2.09 (s, 3H), 1.84 (s, 4H), 1.67 (s, 5H), 1.39-1.36 (m, 2H), 1.31-1.26 (m, 3H), 1.19-1.11 (m, 4H), 0.85 (s, 6H). ¹³C NMR (100 MHz, CDCl₃): δ 171.12, 171.04, 169.01, 168.98, 154.25, 154.22, 151.15, 124.58, 120.72, 61.94, 61.58, 55.54, 55.34, 55.14, 53.68, 52.83, 52.81, 50.66, 48.19, 46.74, 46.70, 44.49, 42.75, 41.91, 41.86, 40.75, 39.51, 33.10, 32.80, 32.43, 31.98, 30.21, 30.14, 21.50.

### Example 91 Synthesis of 1-(4-((4-(2-(1-acetylpiperidin-4-yl)acetyl)-1,4-diaza-1-yl)methyl)-2-fluorophenyl)-3-((1r,3R,5S, 7r)-3,5-dimethyladamantan-1-yl)urea (GL-4)

This example was operated according to the method of **Example 9,** methanol was used as a raw material (the ethyl (S)-1-(4-amino-3-fluorobenzyl)piperidine-3-carboxylate in Example 9 was replaced with 1-((1r,3R,5S,7r)-3,5-dimethyladamantan-1-yl)-3-(2-fluoro-4-((4-(2-(piperidin-4-yl)acetyl)-1,4-diaza-1-yl)methyl)phenyl)urea, and the memantine was replaced with methanol) to obtain a white solid, namely **GL-4,** with an output of 0.11 g and a yield of 19%, m.p. 121-122°C. ¹H NMR(400MHz, CDCl₃):δ (ppm) 8.05-8.00 (m, 1H), 7.24-7.20 (m, 1H), 7.00-6.93 (m, 2H), 5.61-5.60 (m, 1H), 4.13-4.12 (m, 2H), 3.68 (s, 3H), 3.64-3.59 (m, 2H), 3.53-3.51 (m, 3H), 3.46 (s, 1H), 2.78 (s, 2H), 2.64-2.60 (m, 2H), 2.56-2.55 (m, 2H), 2.25-2.19 (m, 2H), 2.13-2.12 (m, 1H),1.83-1.78 (m, 4H), 1.74-1.72 (m, 2H), 1.65 (s, 4H), 1.38-1.35 (m, 2H), 1.29-1.26 (m, 3H), 1.15-1.14 (m, 3H), 0.85 (s, 6H). ¹³C NMR (150 MHz, DMSO-*d₆*): 171.32, 171.26, 156.04, 154.13, 124.56, 120.86, 114.84, 61.85, 61.85, 55.63, 55.58, 55.02, 53.77, 52.88, 52.56, 50.64, 48.19, 48.19, 47.15, 44.50, 44.11, 42.73, 40.76, 39.70, 39.66, 33.04, 32.15, 32.15, 30.20, 30.12, 30.12, 27.48.

### Example 92 Synthesis of 1-((1r,3R,5S,7r)-3,5-dimethyladamantan-1-yl)-3-(2-fluoro-4-((4-(2-(1-(2-hydroxyethyl)piperidin-4-yl)acetyl)-1,4-diaza-1-yl)methyl)phenyl)urea (GL-21)

1-((1r,3R,5S,7r)-3,5-dimethyladamantan-1-yl)-3-(2-fluoro-4-((4-(2-(piperidin-4-yl)acetyl )-1,4-diaza-1-yl)methyl)phenyl)urea (0.5 g, 2.31 mmol) and dry acetonitrile (10 mL) were added into a single-necked flask. After the 1-((1r,3R,5S,7r)-3,5-dimethyladamantan-1-yl)-3-(2-fluoro-4-((4-(2-(piperidin-4-yl)acetyl)-1,4-diaza-1-yl)methyl)phenyl)urea was dissolved, potassium carbonate (0.67 g, 6.70 mmol), potassium iodide (0.15 g, 1.29 mmol), and 2-chloroethan-1-ol (0.15 g, 2.31 mmol) were added thereto and a resulting mixture was refluxed. After 6 h, the reaction was completed as monitored by TLC. The resulting reaction mixture was concentrated under reduced pressure to remove acetonitrile, water (10 mL) was added thereto, and a resulting mixture was extracted with ethyl acetate (10 mL×2). The organic layers were combined, washed with water (10 mL), and washed once with saturated aqueous NaCl solution (15 mL). The organic phase was concentrated under reduced pressure to obtain 0.41 g of a white solid, which was loaded into a column with 4 times silica gel, after being mixed with 1.2 times silica gel, and subjected to column chromatography with an eluent (EA: PE=1:5) to obtain a white solid, namely the 1-((1r,3R,5S,7r)-3,5-dimethyladamantan-1-yl)-3-(2-fluoro-4-((4-(2-(1-(2-hydroxyethyl)piperidin-4-yl)acetyl)-1,4-diaza-1-yl)methyl)phenyl)urea, with an output of 0.21 g and a yield of 34%, m.p. 101-102 °C. ¹H NMR (400 MHz, DMSO-*d₆*): δ (ppm) 8.14 (s, 1H), 8.04-8.00 (m, 1H), 7.08-7.04 (m, 1H Hz), 6.96-6.94 (m, 1H),6.57 (s, 1H), 5.34 (s, 1H), 4.48 (s, 1H), 3.52-3.46 (m, 10 H), 2.90-2.88 (m, 2H), 2.59 (s, 1H), 2.44 (s, 3H), 2.21-2.17 (m, 2H), 2.08-2.05 (m, 3H), 1.75 (s, 4H), 1.65-1.62 (m, 4H), 1.57 (s, 4H), 1.34-1.31 (m, 4H), 1.11 (s, 2H), 0.82 (s, 6H). ¹³C NMR (100 MHz, DMSO-*d₆*): δ 170.97, 154.17, 153.00, 150.61, 124.67, 120.04, 115.09, 114.90, 63.05, 60.72, 60.62, 58.61, 56.03, 55.22, 54.52, 54.01, 53.79, 52.48, 51.99, 50.75, 48.09, 47.75, 46.73, 44.99, 44.39, 42.80, 32.66, 32.37, 31.90, 30.55, 30.05, 28.50, 27.42, 7.66.

### Example 93 Synthesis of 1-(4-((4-(2-(1-(2,3-dihydroxypropyl)piperidin-4-yl)acetyl)-1,4-diaza-1-yl)methyl)-2-fluorophenyl)-3-((1r,3R,5S,7r)-3,5-dimethyladamantan-1-yl)urea (GL-22)

This example was operated according to the method of **Example 93,** 3-chloropropane-1,2-diol was used as a raw material (the chloride in Example 93 was replaced with 3-chloropropane-1,2-diol) to obtain a white solid, namely **GL-22,** with an output of 0.24 g and a yield of 27%, m.p. 104-105 °C. ¹H NMR (400 MHz, DMSO-*d₆*): δ (ppm) 8.14 (s, 1H), 8.03-7.99 (m, 1H), 7.09-7.04 (m, 1H), 6.98-6.94 (m, 1H),6.57 (s, 1H), 5.33 (s, 4H), 5.01 (s, 1H), 3.67 (s, 1H), 3.52-3.43 (m, 10H), 2.99 (s, 3H), 2.60-2.59 (m, 2H), 2.21-2.19 (m, 4H), 2.09 (s, 1H), 1.75 (s, 5H), 1.68 (s, 5H), 1.57 (s, 3H), 1.34 (s, 1H), 1.31 (s, 1H), 1.11 (s, 2H), 0.82 (s, 6H). ¹³C NMR (100 MHz, DMSO-*d₆*): δ 170.90, 154.18, 127.62, 124.68, 120.08, 65.23, 63.07, 56.03, 55.20, 54.51, 53.83, 52.50, 52.50, 50.76, 48.10, 48.10, 47.74, 46.71, 44.41, 42.80, 42.80, 32.38, 32.38, 30.55, 30.55, 30.05, 28.48, 27.39, 7.67, 7.67.

### Example 94 Synthesis of 1-((1r,3R,5S,7r)-3,5-dimethyladamantan-1-yl)-3-(4-((4-(2-(1-(2-(dimethylamino)ethyl)piperidin-4-yl)acetyl)-1,4-diaza-1-yl)methyl)-2-fluorophenyl) urea (GL-23)

This example was operated according to the method of **Example 93,** 2-chloro-N,N-dimethyl-1-amine was used as a raw material (the chloride in Example 93 was replaced with 2-chloro-N,N-dimethyl-1-amine) to obtain a white solid, namely **GL-23,** with an output of 0.21 g and a yield of 26%, m.p. 108-109 °C. ¹H NMR(400MHz,CDCl₃):δ (ppm) 8.08-8.01 (m, 1H), 7.01-6.96 (m, 1H), 6.94-6.91 (m, 1H), 5.62 (s, 2H), 3.62-3.61 (m, 5H), 3.54 (s, 1H), 3.51-3.48 (m, 2H), 3.44-3.42 (m, 1H), 3.08-3.06 (m, 2H), 2.72 (s, 1H), 2.66 (s, 1H), 2.61 (s, 3H), 2.57-2.55 (m, 1H), 2.43 (s, 2H), 2.37 (s, 2H), 2.32 (s, 1H), 2.25-2.23 (m, 2H), 2.20-2.18 (m, 1H), 2.13 (s, 1H), 1.95 (s, 1H), 1.88 (s, 2H), 1.80-1.77 (m, 4H), 1.70 (s, 3H), 1.39 (s, 1H), 1.36 (s, 1H), 1.25 (s, 4H), 1.16 (s, 1H), 1.13 (s, 1H), 0.84 (s, 6H). ¹³C NMR (100 MHz, CDCl₃): δ 171.33, 154.79, 154.73, 124.48, 120.86, 114.79, 114.61, 63.43, 62.07, 61.59, 53.95, 53.01, 53.01, 52.75, 50.74, 48.07, 47.26, 45.30, 45.15, 44.34, 42.83, 40.70, 39.37, 32.51, 32.51, 32.40, 31.62, 31.53, 30.20, 29.69, 28.69, 27.92, 8.13.

### Example 95 Synthesis of (S)-1-(4-(3-((1r,3R,5S,7S)-3,5-dimethyladamantan-1-yl)ureido)-3-fluorobenzyl)-N,N-bis(2-hydroxyethyl))piperidine-3-carboxamide (GL-24)

This example was operated according to the method of **Example 93,** diethanolamine was used as a raw material (the chloride in Example 93 was replaced with diethanolamine) to obtain a white solid, namely **GL-24,** with an output of 0.22 g and a yield of 27%, m.p. 110-111 °C.¹H NMR(400MHz,CDCl₃):δ (ppm) 8.06-7.98 (m, 1H), 6.94-6.89 (m, 1H), 6.83-6.77 (m, 1H), 5.57 (s, 2H), 4.55 (s, 3H), 3.57-3.55 (m, 5H), 3.46-3.43(m, 3H), 3.35 (s, 1H), 3.10-3.07 (m, 1H), 3.01-2.98 (m, 2H), 2.95-2.93 (m, 2H), 2.86-2.84 (m, 2H), 2.80-2.78 (m, 2H), 2.63 (s, 1H), 2.55 (s, 2H), 2.51 (s, 1H), 2.23-2.15 (m, 2H), 2.05 (s, 1H), 1.91 (s, 1H), 1.82 (s, 1H), 1.74 (s, 3H), 1.65 (s, 2H), 1.32-1.29 (m, 2H), 1.24 (s, 1H), 1.22-1.21 (m, 3H), 1.18-1.15 (m, 5H), 1.09 (s, 1H), 1.05 (s, 1H), 0.77 (s, 6H). ¹³C NMR (100 MHz, CDCl₃): δ 171.29,171.10, 154.99, 154.90, 124.63, 63.47, 61.60, 55.76, 55.44, 55.13, 54.78, 53.82, 53.04, 53.04, 52.69, 50.78, 48.31, 48.02, 48.02, 47.09, 44.09, 42.87, 40.68, 39.19, 32.38, 32.38, 32.07, 31.46, 31.19, 30.23,30.23, 29.68, 8.15.

### Test Example 1

### 1. Determination of inhibitory activity

Detection principle: a specific substrate, (3-phenyl-oxy)-acetic acid cyano-(6-methoxy-naphthalen-2-yl) methyl ester, namely PHOME, is non-fluorescent but can be hydrolyzed under the action of sEH enzyme to generate a product 6-methoxy-2-naphthaldehyde. The 6-methoxy-2-naphthaldehyde can emit fluorescence with a wavelength of 465 nm under 330 nm light wave excitation, and an intensity of the detected fluorescence signal is inversely proportional to an inhibitory effect on the sEH enzyme. Based on the above principle, the inhibition rates of samples with different concentrations were calculated compared with the positive control group. The IC₅₀ value of the compound was calculated based on the inhibition rate and concentration using SPSS 20 software.

### 2. Preparation of reagents and drugs

25 mM Tris-HCl buffer (pH=7.4, containing 0.1 mg/mL BSA): 5 mg BAS was added to 12.5 mL of 1 M Tris-HCl buffer, followed by diluting with purified water, and a resulting mixture was adjusted to a pH value of 7.4 with hydrochloric acid, and diluted to 500 mL.

PHOME solution: 0.79 mg PHOME was dissolved in 106 µL DMSO to obtain a 20 mM PHOME solution, which was diluted to 1/3 mM with Tris-HCl buffer before use.

sEH solution: sEH (5 mg/mL) stock solution was stored in a refrigerator at -80 °C and diluted to 4 µg/mL with 25 mM Tris-HCl buffer before use.

A sample powder to be tested was dissolved in DMSO to a 20 mM solution, stored in a refrigerator at -20 °C for later use, and diluted with Tris-HCl buffer to corresponding concentrations when used.

### 3. Experimental group

Experimental design: solvent group, 100% activity group (A), inhibitor group (B), positive control group (C), as shown in Table 1.

**Table 1 Experimental Grouping**

| Well | Buffer | DMSO | Inhibitor | sEH | Substrates |
|---|---|---|---|---|---|
| Solvent group | 168 µL | 2 µL | - | - | 30 µL |
| 100% activity group (A) | 148 µL | 2 µL | - | 20 µL | 30 µL |
| Inhibitor group (B) | 148 µL | - | 2 µL | 20 µL | 30 µL |
| Positive control group (C) | 148 µL | - | 2 µL | 20 µL | 30 µL |

### 4. Experimental steps

(a) 148 µL/well Tris-HCl buffer was added into a black-bottom 96-well microplate;
(b) 2 µL of a sample solution to be tested was added; for the solvent group and 100% activity group, an equal volume of DMSO was added instead; and for the positive control group, the lead compound t-TUCB was added instead, with a structural formula of ;
(c) for the inhibitor group, there were a total of 5 concentrations, with final concentrations of 10 nM, 5 nM, 2.5 nM, 1.25 nM, and 0.625 nM, respectively;
(d) 20 µL of s-EH solution (final concentration of 400 ng/mL) was added, for the solvent group, an equal volume of Tris-HCl buffer was added instead;
(e) 30 µL of PHOME substrate was added to start the reaction (final concentration of 50 µM) and incubated in a 37 °C incubator for 10 min;
(f) fluorescence signal data were measured by microplate reader, with excitation wavelength of 330 nm and emission wavelength of 465 nm.

### 5. Data analysis

Three replicate wells were set for each sample, and the average value of the three replicate wells was the fluorescence value (F) of the compound to be tested. The inhibition rate % = [(AF-BF)/AF]×100, where AF was the fluorescence value of the 100% activity group and BF was the fluorescence value of the inhibitor group. The IC₅₀ value of the compound was calculated based on the inhibition rate and concentration using SPSS 20 software.

The inhibitory activities of CL, CC, GL and ZT compounds against HsEH and MsEH are shown in Table 1.

**Table 1 Inhibitory activities of CL, CC, GL and ZT compounds against HsEH and MsEH**

| No. | HsEH IC₅₀ (nM) | MsEH IC₅₀ (nM) | No. | HsEH IC₅₀ (nM) | MsEH IC₅₀ (nM) |
|---|---|---|---|---|---|
| | Inhibitory activity against HsEH | Inhibitory activity against MsEH | | Inhibitory activity against HsEH | Inhibitory activity against MsEH |
| CL-101 | 0.14 | 0.087 | CC-113 | 0.28 | 0.23 |
| CL-102 | 0.05 | 0.14 | CC-114 | 3.43 | 0.26 |
| CL-103 | 0.25 | 0.62 | CC-115 | 4.5 | 0.18 |
| CL-104 | 2.7 | 4.14 | CC-116 | 7.1 | 0.55 |
| CL-105 | 0.7 | 0.12 | CC-120 | 0.35 | 0.15 |
| CL-106 | 0.35 | 0.50 | CC-121 | 0.84 | 0.15 |
| CL-107 | 0.28 | 0.29 | CC-124 | 19.6 | 0.8 |
| CL-109 | 6.72 | 0.55 | GL-1 | 0.33 | 0.07 |
| CL-112 | 0.28 | 0.35 | GL-2 | 0.77 | 0.16 |
| CL-113 | 0.28 | 0.58 | GL-3 | 0.49 | 0.11 |
| CL-114 | 0.63 | 0.18 | GL-4 | 1.1 | 0.41 |
| CL-115 | 0.14 | 0.32 | GL-21 | 4.69 | 0.61 |
| CL-116 | 0.98 | 0.55 | GL-22 | 58.1 | 1.25 |
| CL-117 | 6.51 | 0.12 | GL-23 | 2.59 | 0.67 |
| CL-118 | 1.54 | 0.058 | GL-24 | 2.1 | 1.37 |
| CL-119 | 31.6 | 2.25 | GL-101 | 0.6 | 0.26 |
| CL-120 | 6.0 | 0.47 | GL-102 | 0.56 | 0.58 |
| CL-201 | 0.42 | 1.1 | GL-104 | 1.4 | 0.9 |
| CL-204 | 0.42 | 0.8 | ZT-110 | 0.8 | 0.18 |
| CL-205 | 0.9 | 1.3 | ZT-111 | 1.54 | 0.44 |
| CL-5 | 2.3 | 0.61 | ZT-112 | 2.94 | 0.23 |
| CC-103 | 1.0 | 0.35 | ZT-113 | 0.84 | 0.35 |
| CC-105 | 0.9 | 0.39 | ZT-114 | 0.91 | 0.55 |
| CC-106 | 1.0 | 0.13 | ZT-115 | 0.35 | 0.12 |
| CC-110 | 0.35 | 0.38 | ZT-116 | 183 | 2.0 |
| CC-111 | 2.2 | 0.53 | ZT-117 | 16 | 0.53 |
| CC-112 | 12.5 | 0.32 | t-TUCB | 0.28 | 2.8 |

As shown in Table 1, the HsEH IC₅₀ values of the compounds **CL, CC, GL** and **ZT** are in a range of 0.05 nM to 183 nM, showing desirable inhibitory effects. The MsEH IC₅₀ values of the compounds **CL, CC, GL** and **ZT** are in a range of 0.058 nM to 4.14 nM, also showing desirable inhibitory effects. The results show that the inhibitory activity of **CL-101, CL-102, CL-103,** and **CL-115** against HsEH is better than that of the lead compound t-TUCB, among which compound **CL-102** has the strongest inhibitory activity with an IC₅₀ of 0.05 nM. The results show that all compounds have better inhibitory activity against MsEH than the lead compound t-TUCB, except for compound **CL-104.** Among them, compound **CL-118** has the strongest inhibitory activity with an IC₅₀ of 0.058 nM. Finally, it was found that compounds **CL-101, CL-102, CL-103, CL-107, CL-112, CL-113, CL-115,** and **CC-113** had better HsEH IC₅₀ and MsEH IC₅₀ values than that of the lead compound t-TUCB, among which compound **CL-102** had the strongest inhibitory activity with an HsEH IC₅₀ of 0.05 nM and an MsEH IC₅₀ of 0.14 nM, showing extremely desirable development prospects.

The above are merely preferred embodiments of the present application. It should be noted that a person of ordinary skill in the art may further make several improvements and modifications without departing from the principle of the present application, but such improvements and modifications should be deemed as falling within the scope of the present application.

## Claims

1. A memantyl urea derivative, having a structure selected from the group consisting of Formula A and Formula B: 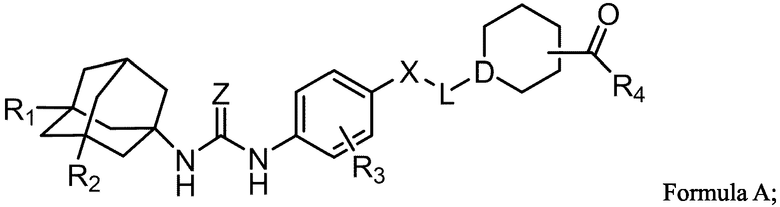 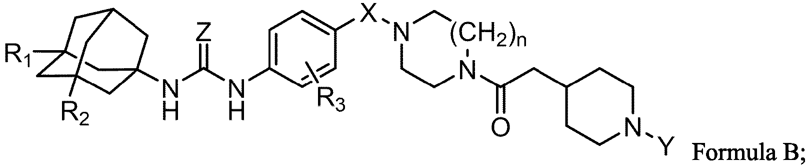
wherein in Formula A and Formula B,
R₁ and R₂ each are independently selected from the group consisting of -H, -OH, -NH₂, -SH, -CN, a halogen group, alkyl, alkoxy, aryl, and heteroaryl;
R₃ is selected from the group consisting of -H, -OH, -NH₂, -SH, -CN, a halogen group, alkyl, and alkoxy;
R₄ is selected from the group consisting of -OH, -NH₂, hydroxylamino, alkyl-substituted amino, alkoxy-substituted amino, alcohol group-substituted amino, phenyl-substituted amino, naphthyl-substituted amino, and 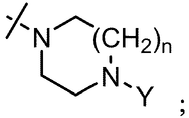
Y is selected from the group consisting of -H, 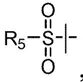 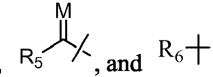 , wherein
R₅ is selected from the group consisting of alkyl, alkoxy, and a heterocyclic group, the alkyl being selected from the group consisting of chain alkyl and cycloalkyl; and
R₆ is selected from the group consisting of alkyl-substituted amino and alcohol group-substituted alkyl;
X is selected from the group consisting of -CH₂- and 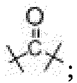
L is absent or -NH-;
D is selected from the group consisting of 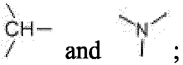
n is 1 or 2; and
Z and M each are independently selected from the group consisting of =O and =S.

2. The memantyl urea derivative as claimed in claim 1, wherein R₁ and R₂ each are independently selected from the group consisting of -H, -OH, -NH₂, -SH, -CN, -F, -Cl, -Br, methyl, ethyl, propyl, butyl, pentyl, isobutyl, isopropyl, isopentyl, tert-butyl, methoxy, ethoxy, propoxy, isopropoxy, butoxy, cyclopentyloxy, cyclohexyloxy, phenoxy, and benzyloxy.

3. The memantyl urea derivative as claimed in claim 2, wherein R₁ and R₂ both are methyl.

4. The memantyl urea derivative as claimed in claim 1, wherein R₃ is selected from the group consisting of -H, -OH, -NH₂, -SH, -CN, -F, -Cl, -Br, unsubstituted or substituted C1-C6 alkyl, and unsubstituted or substituted C1-C6 alkoxy; and a substituent on the substituted C1-C6 alkyl and the substituted C1-C6 alkoxy is independently selected from the group consisting of -F, -Cl, -Br, -OH, -NH₂, -NHCH₃, -N(CH₃)₂, and C1-C6 alkyl.

5. The memantyl urea derivative as claimed in claim 4, wherein R₃ is -F.

6. The memantyl urea derivative as claimed in claim 1, wherein the alkyl-substituted amino for R₄ is unsubstituted or substituted C1-C6 alkyl-substituted amino; the alkoxy-substituted amino for R₄ is unsubstituted or substituted C1-C6 alkoxy-substituted amino; the phenyl-substituted amino for R₄ is unsubstituted or substituted phenyl-substituted amino; the naphthyl-substituted amino for R₄ is unsubstituted or substituted naphthyl-substituted amino; the alcohol group-substituted amino for R₄ is unsubstituted or substituted C1-C6 alcohol group-substituted amino; and a substituent on substituted C1-C6 alkoxy, substituted phenyl, substituted naphthyl, and substituted C1-C6 alcohol group is independently selected from the group consisting of -F, -Cl, -Br, -OH, -NH₂, -NHCH₃, -N(CH₃)₂, and C1-C6 alkyl.

7. The memantyl urea derivative as claimed in claim 6, wherein R₄ is selected from the group consisting of -OH, -NH₂, -N(CH₃)₂, -N(CH₂CH₃)₂, -N(CH₂CH₂OH)₂, -NHCH(CH₂OH)₂, -NHC(CH₂OH)₃, -NHCH₂CH₂OH, -NHCH₂CHCH₃OH, -N(CH₃)₂, 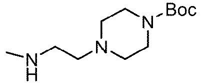 , and

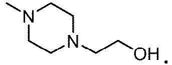

8. The memantyl urea derivative as claimed in claim 1, wherein the chain alkyl for R₅ is unsubstituted or substituted C1-C6 saturated or unsaturated chain alkyl, and a substituent on the substituted C1-C6 saturated or unsaturated chain alkyl is selected from the group consisting of -OH, -NH₂, and C1-C6 alkyl;
the cycloalkyl for R₅ is unsubstituted or substituted C3-C6 cycloalkyl, and a substituent on the substituted C3-C6 cycloalkyl is selected from the group consisting of -F, -Cl, -Br, -OH, -NH₂, -NHCH₃, -N(CH₃)₂, and C1-C6 alkyl; and
the heterocyclic group for R₅ is an unsubstituted or substituted C3-C6 saturated or unsaturated heterocyclic group, and a substituent on the substituted C3-C6 saturated or unsaturated heterocyclic group is selected from the group consisting of -F, -Cl, -Br, -OH, -NH₂, -NHCH₃, -N(CH₃)₂, and C1-C6 alkyl.

9. The memantyl urea derivative as claimed in claim 8, wherein R₅ is selected from the group consisting of -CH₃, -CH₂CH₃, -CHCH₃CH₂CH₃, -OCH₃,

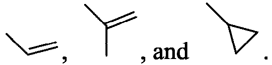

10. The memantyl urea derivative as claimed in claim 1, wherein for R₆, the alkyl-substituted amino is unsubstituted or substituted C1-C6 alkyl-substituted amino, and the alcohol group-substituted alkyl is unsubstituted or substituted C1-C6 alcohol group-substituted alkyl.

11. The memantyl urea derivative as claimed in claim 10, wherein R₆ is selected from the group consisting of -CHCH₃CH₂N(CH₃)₂, -CH₂CH₂N(CH₂CH₃)₂, -CH₂CH₂N(CH₃)₂, -CH₂CH₂OH, and -CH₂CHOHCH₂OH.

12. The memantyl urea derivative as claimed in claim 1, wherein n is 2.

13. The memantyl urea derivative as claimed in claim 1, comprising: (S)-1-(4-(3-((1r, 3R,5S,7S)-3,5-dimethyladamantan-1-yl)ureido)-3-fluorobenzyl)piperidine-3-carboxamide, 1-((1 r,3R,5S,7S)-3,5-dimethyladamantan-1-yl)-3-(2-fluoro-4-(((S)-3-(4-propionyl-1,4-)diaza-1-carbo nyl)piperidin-1-yl)methyl)phenyl)urea, 1-((1r,3R,5S,7S)-3,5-dimethyladamantan-1-yl)-3-(2-fluo ro-4-(((3S)-3-(4-(2-methylbutanoyl)-1,4-diaza-1-carbonyl)piperidin-1-yl)methyl)phenyl)urea, (S) -1-(4-(3-((1r,3R,5S,7S)-3,5-dimethyladamantan-1-yl)ureido)-3-fluorobenzyl)-N,N-dimethylpiper idine-3-carboxamide, (S)-1-(4-(3-((1r,3R,5S,7S)-3,5-dimethyladamantan-1-yl)ureido)-3-fluorob enzyl)-N,N-diethylpiperidine-3-carboxamide, (S)-1-(4-(3-((1r,3R,5S,7S)-3,5-dimethyladamanta n-1-yl)ureido)-3-fluorobenzyl)-N,N-bis(2-hydroxyethyl)piperidine-3-carboxamide, 1-(4-(3-((1r, 7r)-3,5-dimethyladamantan-1-yl)ureido)-3-fluorobenzyl)-N,N-bis(2-hydroxyethyl)piperidine-4-c arboxamide, (S)-N-(1,3-dihydroxypropan-2-yl)-1-(4-(3-((1r,3R,5S,7S)-3,5-dimethyladamantan-1-yl)ureido)-3-fluorobenzyl)piperidine-3-carboxamide, (3S)-1-(4-(3-((1r,3R,5S,7S)-3,5-dimethy ladamantan-1-yl)ureido)-3-fluorobenzyl)-N-(2-hydroxypropyl)piperidine-3-carboxamide, (S)-1-(4-(3-((1r,3R,5S,7S)-3,5-dimethyladamantan-1-yl)ureido)-3-fluorobenzyl)-N-(2-hydroxyethyl)pi peridine-3-carboxamide, (S)-N-(1,3-dihydroxy-2-(hydroxymethyl)propan-2-yl)-1-(4-(3-((1r,3R, 5S,7S)-3,5-dimethyladamantan-1-yl)ureido)-3-fluorobenzyl)piperidine-3-carboxamide, 1-((1r,3 R,5S,7S)-3,5-dimethyladamantan-1-yl)-3-(2-fluoro-4-(((S)-3-(4-(2-hydroxyethyl)piperazine-1-ca rbonyl)piperidin-1-yl)methyl)phenyl)urea, N-(1,3-dihydroxy-2-(hydroxymethyl)propan-2-yl)-1-(4-(3-((1r,7r)-3,5-dimethyladamantan-1-yl)ureido)-3-fluorobenzyl)piperidine-4-carboxamide, 1-(4-(3-((1r,7r)-3,5-dimethyladamantan-1-yl)ureido)-3-fluorobenzyl)piperidine-4-carboxamide, N-(1,3-dihydroxypropan-2-yl)-1-(4-(3-((1r,7r)-3,5-dimethyladamantan-1-yl)ureido)-3-fluorobenzyl) piperidine-4-carboxamide, 1-(4-(3-((1r,7r)-3,5-dimethyladamantan-1-yl)ureido)-3-fluorobenzyl) -N-(2-hydroxypropyl)piperidine-4-carboxamide, 1-(4-(((S)-3-(4-acryloyl-1,4-diaza-1-carbonyl) piperidin-1-yl)methyl)-2-fluorophenyl)-3-((1r,3R,5S,7S)-3,5-dimethyladamantan-1-yl)urea, 1-(4 -((S)-3-(4-(cyclopropanecarbonyl)-1,4-diaza-1-carbonyl)piperidin-1-yl)methyl)-2-fluorophenyl)-3-((1r,3R,5S,7S)-3,5-dimethyladamantan-1-yl)urea, 1-((1r,3R,5S,7S)-3,5-dimethyladamantan-1-yl)-3-(2-fluoro-4-(((3S)-3-(1-(2-methylbutyryl)azacyclohexane-4-carbonyl)piperidin-1-yl)methyl) phenyl)urea, (S)-1-(4-(3-((1r,3R,5S,7S)-3,5-dimethyladamantan-1-yl)ureido)-3-fluorobenzyl)pip eridine-3-carboxylic acid, 1-((1r,3R,5S,7r)-3,5-dimethyladamantan-1-yl)-3-(2-fluoro-4-(4-(2-(1 -(2-methylbutyryl)piperidin-4-yl)acetyl)-1,4-diaza-1-carbonyl)phenyl)urea, 1-((Ir,3R,5S,7r)-3,5 -dimethyladamantan-1-yl)-3-(2-fluoro-4-(4-(2-(1-propionylpiperidin-4-yl)acetyl)-1,4-diaza-1-car bonyl)phenyl)urea, 1-(4-(4-(2-(1-acetylpiperidin-4-yl)acetyl)-1,4-diaza-1-carbonyl)-2-fluorophe nyl)-3-((1r,3R,5S,7r)-3,5-dimethyladamantan-1-yl)urea, (S)-1-(4-(3-((1r,3R,5S,7S)-3,5-dimethyl adamantan-1-yl)ureido)-3-fluorobenzoyl)-N,N-diethylpiperidine-3-carboxamide, 1-(4-(3-((1r,3R, 5S,7r)-3,5-dimethyladamantan-1-yl)ureido)-3-fluorobenzoyl)-N,N-diethylpiperidine-4-carboxam ide, 1-((1r,3R,5S,7r)-3,5-dimethyladamantan-1-yl)-3-(2-fluoro-4-(4-(4-(2-hydroxyethyl)piperazi ne-1-carbonyl)piperidine-1-carbonyl)phenyl)urea, (3S)-1-(4-(3-((1r,3R,5S,7S)-3,5-dimethylada mantan-1-yl)ureido)-3-fluorobenzoyl)-N-(2-hydroxypropyl)piperidine-3-carboxamide, 1-(4-(3-((1r,3R,5S,7r)-3,5-dimethyladamantan-1-yl)ureido)-3-fluorobenzoyl)-N-(2-hydroxyethyl)piperid ine-4-carboxamide, 1-(4-(3-((1r,3R,5S,7r)-3,5-dimethyladamantan-1-yl)ureido)-3-fluorobenzoyl) -N-(2-hydroxypropyl)piperidine-4-carboxamide, N-(1,3-dihydroxypropan-2-yl)-1-(4-(3-((1r,3R, 5S,7r)-3,5-dimethyladamantan-1-yl)ureido)-3-fluorobenzoyl)piperidine-4-carboxamide, (S)-1-(4 -(3-((1r,3R,5S,7S)-3,5-dimethyladamantan-1-yl)ureido)-3-fluorobenzoyl)-N-(2-hydroxyethyl)pip eridine-3-carboxamide, 1-((1r,3R,5S,7S)-3,5-dimethyladamantan-1-yl)-3-(2-fluoro-4-((S)-3-(4-(2-hydroxyethyl)piperazine-1-carbonyl)piperidine-1-carbonyl)phenyl)urea, 1-((1r,3R,5S,7r)-3,5-dimethyladamantan-1-yl)-3-(4-(4-(2-(1-(1-(dimethylamino)propan-2-yl)piperidin-4-yl)acetyl)-1, 4-diaza-1-carbonyl)-2-fluorophenyl)urea, 1-((1r,3R,5S,7r)-3,5-dimethyladamantan-1-yl)-3-(2-fl uoro-4-((4-(2-(1-propionylpiperidin-4-yl)acetyl)-1,4-diaza-1-yl)methyl)phenyl)urea, 1-((1r,3R,5 S,7r)-3,5-dimethyladamantan-1-yl)-3-(2-fluoro-4-((4-(2-(1-(2-methylbutyryl)piperidin-4-yl)acet yl)-1,4-diaza-1-yl)methyl)phenyl)urea, 1-(4-((4-(2-(1-acetylpiperidin-4-yl)acetyl)-1,4-diaza-1-yl) methyl)-2-fluorophenyl)-3-((1r,3R,5S,7r)-3,5-dimethyladamantan-1-yl)urea, methyl 4-(2-(4-(4-(3-((1r,3R,5S,7r)-3,5-dimethyladamantan-1-yl)ureido)-3-fluorobenzyl)-1,4-diaza-1-yl)-2-oxoeth yl)piperidine-1-carboxylate, 1-((1r,3R,5S,7r)-3,5-dimethyladamantan-1-yl)-3-(2-fluoro-4-((4-(2-(1-(2-hydroxyethyl)piperidin-4-yl)acetyl)-1,4-diaza-1-yl)methyl)phenyl)urea, 1-(4-((4-(2-(1-(2,3 -dihydroxypropyl)piperidin-4-yl)acetyl)-1,4-diaza-1-yl)methyl)-2-fluorophenyl)-3-((1r,3R,5S, 7r) -3,5-dimethyladamantan-1-yl)urea, 1-((1r,3R,5S,7r)-3,5-dimethyladamantan-1-yl)-3-(4-((4-(2-(1 -(2-(dimethylamino)ethyl)piperidin-4-yl)acetyl)-1,4-diaza-1-yl)methyl)-2-fluorophenyl)urea, 1-(4-((4-(2-(1-(2-(diethylamino)ethyl)piperidin-4-yl)acetyl)-1,4-diaza-1-yl)methyl)-2-fluorophenyl) -3-((1r,3R,5S,7r)-3,5-dimethyladamantan-1-yl)urea, 1-(4-((S)-3-(4-acetyl-1,4-diaza-1-carbonyl) piperidine-1-carbonyl)-2-fluorophenyl)-3-((1r,3R,5S,7S)-3,5-dimethyladamantan-1-yl)urea, 1-((1r,3R,5S,7S)-3,5-dimethyladamantan-1-yl)-3-(2-fluoro-4-((S)-3-(4-propionyl-1,4-diaza-1-carb onyl)piperidine-1-carbonyl)phenyl)urea, 1-((1r,3R,5S,7S)-3,5-dimethyladamantan-1-yl)-3-(2-flu oro-4-((3S)-3-(4-(2-methylbutanoyl)-1,4-diaza-1-carbonyl)piperidine-1-carbonyl)phenyl)urea, 4 -(3-((1r,3R,5S,7r)-3,5-dimethyladamantan-1-yl)ureido)-3-fluoro-N-((1r,4R)-4-((2-hydroxyethyl) carbamoyl)cyclohexyl)benzamide, 4-(3-((1r,3R,5S,7r)-3,5-dimethyladamantan-1-yl)ureido)-3-fl uoro-N-((1r,4R)-4-((2-hydroxypropyl)carbamoyl)cyclohexyl)benzamide, N-((1r,4R)-4-(bis(2-hy droxyethyl)carbamoyl)cyclohexyl)-4-(3-((1r,3R,5S,7r)-3,5-dimethyladamantan-1-yl)ureido)-3-fl uorobenzamide, N-((1r,4R)-4-carbamoylcyclohexyl)-4-(3-((1r,3R,5S,7r)-3,5-dimethyladamanta n-1-yl)ureido)-3-fluorobenzamide, N-((1r,4R)-4-((1,3-dihydroxypropan-2-yl)carbamoyl)cyclohe xyl)-4-(3-((1r,3R,5S,7r)-3,5-dimethyladamantan-1-yl)ureido)-3-fluorobenzamide, N-((Ir,4R)-4-((1,3-dihydroxy-2-(hydroxymethyl)propan-2-yl)carbamoyl)cyclohexyl)-4-(3-((1r,3R,5S,7r)-3,5-dimethyladamantan-1-yl)ureido)-3-fluorobenzamide, tert-butyl 4-(2-((IR, 4r)-4-(4-(3-((Ir,3R,5 S,7r)-3,5-dimethyladamantan-1-yl)ureido)-3-fluorobenzamido)cyclohexane-1-carboxamido)ethy l)piperazine-1-carboxylate, or 4-(3-((1r,3R,5S,7r)-3,5-dimethyladamantan-1-yl)ureido)-3-fluoro -N-((1r,4R)-4-((2-hydroxyethyl)carbamoyl)cyclohexyl)benzamide.

14. A method for preparing the memantyl urea derivative as claimed in any one of claims 1 to 13, wherein
(1) for preparation of the memantyl urea derivative having the structure shown in Formula A:
(1-1) under the condition that the memantyl urea derivative has the structure shown in Formula A where X is 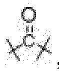 , L is absent, D is 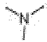 , and R₄ is not 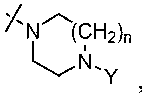 , the method comprises:
subjecting a compound 1 and a compound a to a first acylation to obtain a compound b;
subjecting the compound b to a first reduction to obtain a compound c;
subjecting a compound 2 and a compound 4 to a second acylation to obtain an intermediate compound 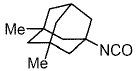 or 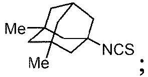
subjecting the intermediate compound and the compound c to a first nucleophilic substitution to obtain a compound d;
subjecting the compound d to a first hydrolysis to obtain the memantyl urea derivative having the structure shown in Formula A where R₄ is -OH; and
mixing the memantyl urea derivative having the structure shown in Formula A where R₄ is -OH with a compound 3, and subjecting a resulting mixture to a third acylation in the presence of 2-(7-azabenzotriazol-1-yl)-*N*,*N*,*N*',*N*'-tetramethyluronium hexafluorophosphate (HATU) and N,N-diisopropylethylamine (DIEA) to obtain the memantyl urea derivative having the structure shown in Formula A; wherein
structural formulas of the compound 1 and the compound 2 are 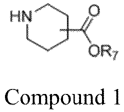 and 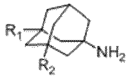 Compound 2, respectively;
the compound 3 is selected from the group consisting of NH₃, hydroxylamine, alkylamine, alkoxyamine, alcoholamine, phenylamine, and naphthylamine;
the compound 4 is selected from the group consisting of bis(trichloromethyl)carbonate (BTC) and thiophosgene;
structural formulas of the compound a, the compound b, the compound c, the compound d, and the compound e are as follows: 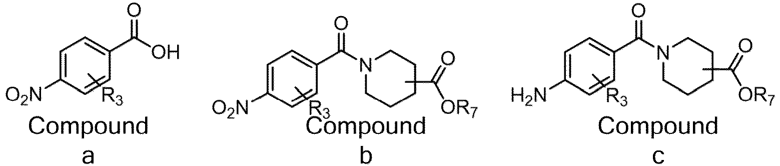 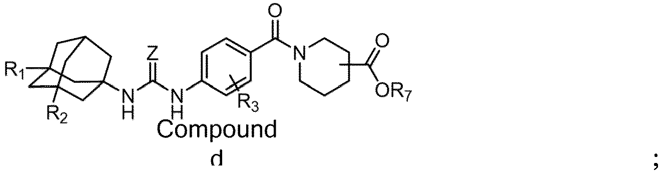
in the structural formulas of the compound 1, the compound b, the compound c, and the compound d, R₇ is C₁-C₆ alkyl;
(1-2) under the condition that the memantyl urea derivative has the structure shown in Formula A where X is -CH₂-, L is absent, D is 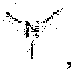 and R₄ is not 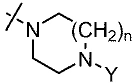 , the method comprises:
subjecting the compound a to a second reduction to obtain a compound e;
subjecting the compound e and a bromination reagent to a second nucleophilic substitution to obtain a compound f;
subjecting the compound f and the compound 1 to a third nucleophilic substitution to obtain a compound g;
subjecting the compound g to a third reduction to obtain a compound h;
subjecting the intermediate compound and the compound h to a fourth nucleophilic substitution to obtain a compound i;
subjecting the compound i to a second hydrolysis to obtain the memantyl urea derivative having the structure shown in Formula A where R₄ is -OH; and
mixing the memantyl urea derivative having the structure shown in Formula A where R₄ is -OH with the compound 3, and subjecting an obtained mixture to a fourth acylation in the presence of HATU and DIEA to obtain the memantyl urea derivative having the structure shown in Formula A; wherein
structural formulas of the compound e, the compound f, the compound g, the compound h, and the compound i are as follows: 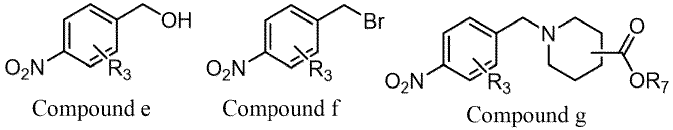 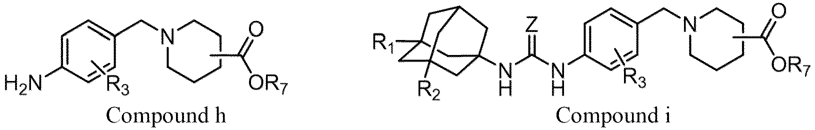 ; and
in the structural formulas of the compound g, the compound h, and the compound i, R₇ is C₁-C₆ alkyl;
(1-3) under the condition that the memantyl urea derivative has the structure shown in Formula A where X is 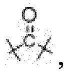 L is -NH-, D is 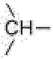 , and R₄ is not 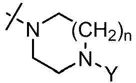 , the method comprises:
subjecting a compound z and the compound a to a fifth acylation to obtain a compound j;
subjecting the intermediate compound and the compound j to a fifth nucleophilic substitution to obtain a compound y;
subjecting the compound y to a third hydrolysis to obtain the memantyl urea derivative having the structure shown in Formula A where R₄ is -OH; and
mixing the memantyl urea derivative having the structure shown in Formula A where R₄ is -OH with the compound 3, and subjecting a mixture thereof to a sixth acylation in the presence of HATU and DIEA to obtain the memantyl urea derivative having the structure shown in Formula A; wherein
the compound z has a structural formula shown as 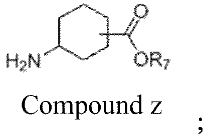
the compound j has a structural formula shown as 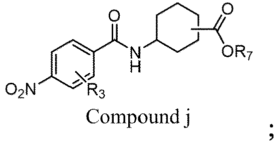
the compound y has a structural formula shown as 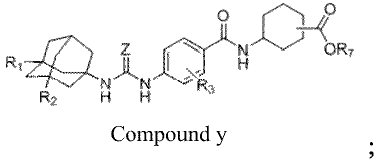 and
in structural formulas of the compound z, the compound j, and the compound y, R₇ is C₁-C₆ alkyl;
(1-4) under the condition that the memantyl urea derivative has the structure shown in Formula A where X is -CH₂-, L is -NH-, D is 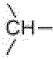 , and R₄ is not 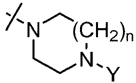 , the method comprises:
subjecting the compound f and the compound z to a sixth nucleophilic substitution to obtain a compound k;
subjecting the compound k to a fourth reduction to obtain a compound 5;
subjecting the intermediate compound and the compound 5 to a seventh nucleophilic substitution to obtain a compound 6;
subjecting the compound 6 to a fourth hydrolysis to obtain the memantyl urea derivative having the structure shown in Formula A where R₄ is -OH; and
mixing the memantyl urea derivative having the structure shown in Formula A where R₄ is -OH with the compound 3, and subjecting an acquired mixture to a seventh acylation in the presence of HATU and DIEA to obtain the memantyl urea derivative having the structure shown in Formula A; wherein
structural formulas of the compound k, the compound 5, and the compound 6 are as follows: 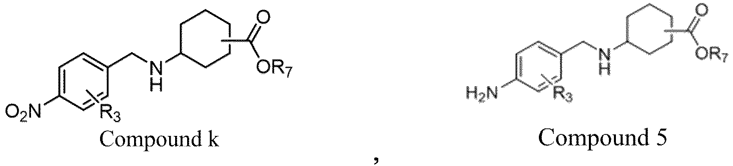 , and 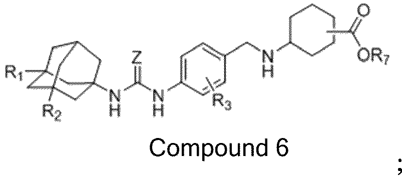 and
in the structural formulas of the compound k, the compound 5, and the compound 6, R₇ is C₁-C₆ alkyl;
(1-5) under the condition that the memantyl urea derivative has the structure shown in Formula A where X is 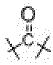 , L is absent, D is 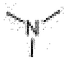 , and R₄ is 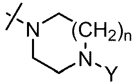 , the method comprises:
subjecting a compound 7 and a compound L to an eighth acylation to obtain a compound m;
subjecting the compound m to a first deprotection in an acidic environment to obtain a compound n, the compound n being the memantyl urea derivative having the structure shown in Formula A where Y is -H;
mixing the compound n with a compound 8, and subjecting a resulted mixture to a ninth acylation in the presence of HATU and DIEA to obtain the memantyl urea derivative having the structure shown in Formula A where Y is 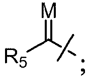
subejcting the compound n and a compound 9 to a tenth acylation to obtain the memantyl urea derivative having the structure shown in Formula A where Y is 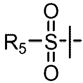 ; and
subjecting the compound n and a compound 10 to an eighth nucleophilic substitution to obtain the memantyl urea derivative having the structure shown in Formula A where Y is 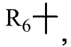 wherein
the compound 7 has a structural formula shown as 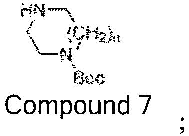
the compound 8 has a structural formula shown as 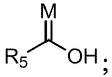
the compound 9 has a structural formula shown as 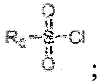
the compound 10 has a structural formula shown as R₆-Cl; and
structural formulas of the compound L, the compound m, and the compound n are as follows: 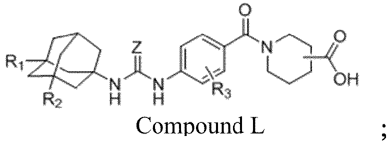 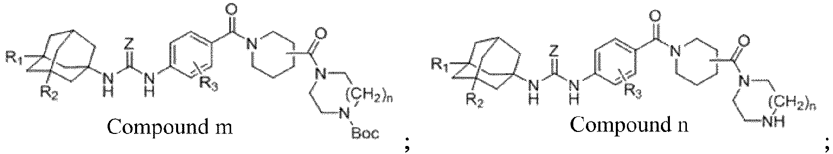
(1-6) under the condition that the memantyl urea derivative has the structure shown in Formula A where X is -CH₂-, L is absent, D is 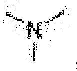 , and R₄ is 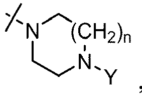 , the method comprises:
subjecting a compound 7 and a compound o to an eleventh acylation to obtain a compound 11;
subjecting the compound 11 to a second deprotection in an acidic environment to obtain a compound 12, the compound 12 being the memantyl urea derivative having the structure shown in Formula A where Y is -H;
mixing the compound 12 with the compound 8 and conducting a twelfth acylation in the presence of HATU and DIEA to obtain the memantyl urea derivative having the structure shown in Formula A where Y is 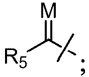
subjecting the compound 12 and the compound 9 to a thirteenth acylation to obtain the memantyl urea derivative having the structure shown in Formula A where Y is 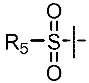 ; and
subjecting the compound 12 and the compound 10 to a ninth nucleophilic substitution to obtain the memantyl urea derivative having the structure shown in Formula A where Y is R₆, wherein
the compound o is 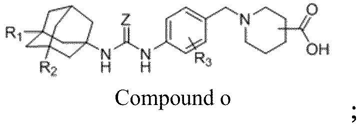
the compound 11 has a structural formula shown as 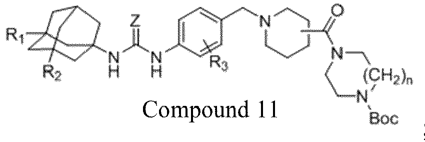 ; and
the compound 12 has a structural formula shown as 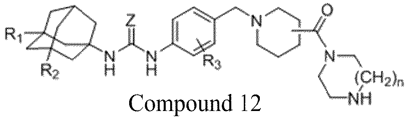 ; and
(2) for preparation of the memantyl urea derivative having the structure shown in Formula B:
(2-1) under the condition that the memantyl urea derivative has the structure shown in Formula B where X is 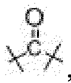 , the method comprises:
subjecting the compound 7 and the compound a to a fourteenth acylation to obtain a compound p;
subjecting the compound p to a fifth reduction to obtain a compound q;
subjecting the intermediate compound and the compound q to a tenth nucleophilic substitution to obtain a compound r;
subjecting the compound r to a third deprotection in an acidic environment to obtain a compound s;
subjecting the compound 13 and the compound s to a fifteenth acylation to obtain a compound t;
subjecting the compound t to a fourth deprotection in an acidic environment to obtain a compound u, the compound u being the memantyl urea derivative having the structure shown in Formula B where Y is -H;
mixing the compound u with the compound 8 and conducting a sixteenth acylation in the presence of HATU and DIEA to obtain the memantyl urea derivative having the structure shown in Formula B where Y is 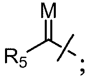
subjecting the compound u and the compound 9 to a seventeenth acylation to obtain the memantyl urea derivative having the structure shown in Formula B where Y is 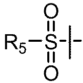 ; and
subjecting the compound u and the compound 10 to an eleventh nucleophilic substitution to obtain the memantyl urea derivative having the structure shown in Formula B where Y is 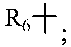 wherein
the compound 13 has a structural formula shown as 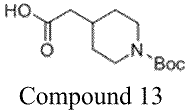 ; and
structural formulas of the compound p, the compound q, the compound r, the compound s, the compound t, and the compound u are as follows: 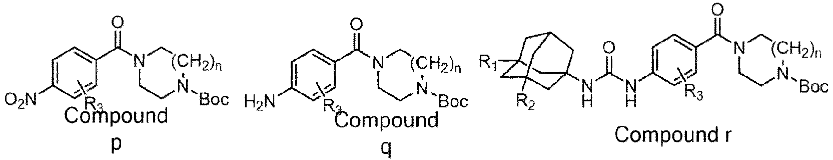 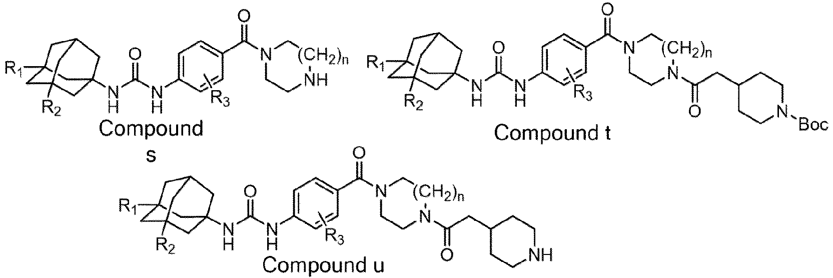 ;
(2-2) under the condition that the memantyl urea derivative has the structure shown in Formula B where X is -CH₂-, the method comprises:
subjecting the compound g and the compound 7 to a twelfth nucleophilic substitution to obtain a compound v;
subjecting the compound v to a sixth reduction to obtain a compound w;
subjecting the intermediate compound and the compound w to a thirteenth nucleophilic substitution to obtain a compound x, the compound x being the memantyl urea derivative having the structure shown in Formula B where Y is -H;
mixing the compound x with the compound 8 and conducting an eighteenth acylation in the presence of HATU and DIEA to obtain the memantyl urea derivative having the structure shown in Formula B where Y is 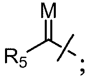
subjecting the compound x and the compound 9 to a nineteenth acylation to obtain the memantyl urea derivative having the structure shown in Formula B where Y is 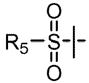 ; and
subjecting the compound x and the compound 10 to a fourteenth nucleophilic substitution to obtain the memantyl urea derivative having the structure shown in Formula B where Y is R₆; wherein
structural formulas of the compound v, the compound w, and the compound x are as follows:

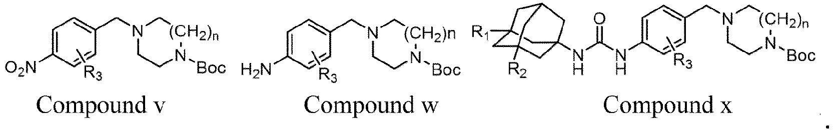

15. Use of the memantyl urea derivative as claimed in any one of claims 1 to 13 or the memantyl urea derivative prepared by the method as claimed in claim 14 in preparation of a drug for treating a soluble epoxide hydrolase (sEH)-mediated disease.

16. The use as claimed in claim 15, wherein the sEH-mediated disease comprises one selected from the group consisting of an inflammatory disease, a pain, a cardiovascular disease, a neurodegenerative disease, diabetes, a diabetic complication, chronic nephritis, renal failure, a chronic obstructive pulmonary disease, and pulmonary hypertension.
